# EUROPEAN PATENT APPLICATION

(11) **EP 4 506 454 A1**
(43) Date of publication of application: **12.02.2025**
(21) Application number: 23784100.2
(22) Date of filing: 24.02.2023
(51) Int. Cl.: C12N 9/78, C07K 19/00, C12N 15/62, C12N 15/55, A61K 38/50, A61P 3/06

(54) **ADENOSINE DEAMINASE, BASE EDITOR, AND USE THEREOF**

(30) Priority: 07.04.2022 CN 202210363757
(71) Applicant: Yoltech Therapeutics Co., Ltd, Shanghai 201109 (CN)
(72) Inventor: ZHANG, Hongling, Shanghai 201109 (CN); LAI, Chongping, Shanghai 201109 (CN)
(74) Representative: IP TRUST SERVICES
(86) International application number: PCT/CN2023/078133
(87) International publication number: WO 2023/193536

(57) **Abstract**

The present invention belongs to the field of biotechnology, and provides a deaminase and an adenine base editor, and also provides a mutated deaminase and a corresponding adenine base editor. The mutated deaminase undergoes mutation of a plurality of amino acids compared with a parent deaminase, thereby improving a base editing efficiency and obtaining a good application prospect.

## Description

### TECHNICAL FIELD

The present invention belongs to the field of biotechnology, and more specifically, the present invention relates to an adenosine deaminase, a base editor fusion protein, a base editor system, and a use thereof.

### BACKGROUND OF THE INVENTION

How to modify a genome precisely and efficiently is an important goal of research in the field of life sciences, and clustered regularly interspaced short palindromic repeats (CRISPR)/Cas9-mediated gene editing technology has become the most powerful tool to achieve this goal. In the CRISPR/Cas9 technology in the related art, intracellular homologous recombination (HR) and non-homologous end joining (NHEJ) repair pathways are induced by generating DNA double-strand breaks (DSB) at a target site, thereby achieving modifications such as site-specific knockout, replacement, and insertion of genomic DNA. However, it is difficult to achieve efficient and stable single-base mutations in DNA repair induced by DSB.

Single nucleotide mutations cause about 2/3 of human genetic diseases and are also a genetic basis for important trait mutations in many plants and animals, and thus it is particularly important to develop a technology that can accurately and efficiently achieve single-base replacement. Base editors developed by David R. Liu's laboratory were born for this purpose. David R. Liu's laboratory has developed three different base editors, that is, a cytosine base editor (CBE), an adenine base editor (ABE), and a prime editor, and these base editors do not rely on generation of DSBs and do not require participation of donor DNA when working.

Adenine base editing technology based on adenosine deaminase mainly uses a fusion protein constituted by nickase Cas9n (D10A) or dCas9 combined with adenosine deaminase, deaminates, under guidance of sgRNA, a target base adenine A located in a base editing active window to form hypoxanthine I, which is then gradually replaced with G after DNA repair 25 and replication, eventually forming a directional replacement of A to G (A to G).

Base editors can also achieve the treatment of some diseases by editing gene targets, such as hypercholesterolemia, transthyretin amyloidosis, and β-hemoglobinopathy, and thus optimizing existing base editors also has better prospects for clinical application.

However, current base editors have differences in site-dependent editing efficiency, and a wide editing window leads to unnecessary editing, so the modification of existing base editors is very necessary.

### SUMMARY OF THE INVENTION

The present invention discloses a deaminase, a base editor containing the deaminase, and a use thereof. Through experiments and exploration, a site-directed mutation is performed on a basic sequence of the deaminase to obtain an adenine base editor with improved editing efficiency, thereby improving an editing efficiency in eukaryotic cells. A large number of experiments are also performed on a structure of a base editor fusion protein, a position suitable for an adenosine deaminase to be embedded in a nuclease domain is found, so as to obtain a base editor fusion protein structure with improved editing efficiency, thereby obtaining a better application prospect.

### Definitions

**Specifically, the present invention includes the following contents.**

### <Adenosine Deaminase>

In some embodiments of a first aspect of the present invention, there is provided an adenosine deaminase including: one or more of the following sequences:
an amino acid sequence that has at least 80%, 82%, 85%, 87%, 90%, 92%, 95%, 96%, 97%, 98%, or 99% identity to an amino acid sequence set forth in SEQ ID NO: 1 or SEQ ID NO: 170, and which retains a deamination activity of the amino acid sequence set forth in SEQ ID NO: 1 or SEQ ID NO: 170;
an amino acid sequence in which one or more amino acid residues are added, substituted, deleted, or inserted into the amino acid sequence set forth in SEQ ID NO: 1 or SEQ ID NO: 170, and which retains the deamination activity of the amino acid sequence set forth in SEQ ID NO: 1 or SEQ ID NO: 170; or
an amino acid sequence encoded by a nucleotide sequence, the nucleotide sequence hybridizing, under a stringency condition, with a polynucleotide sequence encoding the amino acid sequence set forth in SEQ ID NO: 1 or SEQ ID NO: 170, the amino acid sequence retaining the deamination activity of the amino acid sequence set forth in SEQ ID NO: 1 or SEQ ID NO: 170, and the stringency condition being a medium stringency condition, a medium-high stringency condition, a high stringency condition, or a very high stringency condition.

The present invention also relates to an adenosine deaminase variant, and the adenosine deaminase variant has a change in any of the following amino acid positions relative to the amino acid sequence set forth in SEQ ID NO: 1: 33, 35, 36, 46, 47, 48, 49, 104, 105, 107, 148, 149, 150, 151, 152, 153, 154, and 155.

Further, the deaminase variant has a change in at least one selected from the following amino acid positions relative to the amino acid sequence set forth in SEQ ID NO: 1: V33I, D35G, D35R, D36N, D36G, A46C, I47Y, I47R, I47V, T48G, T48R, L49T, L49H, L49K, V104A, V104M, S105C, S105G, S107R, S107K, S107A, Q148P, Q148C, Q148A, Q148G, Q149M, Q149G, Q149L, P150R, P150L, P150C, R151K, E152R, E152T, E152G, E152W, V153P, V153F, V153I, V153T, F154H, F154K, F154L, N155T, N155R, and N155H. A specific position change may be performed alone or may be combined in multiple ways.

Specifically, the adenosine deaminase variant has a change in any one of the following groups relative to the amino acid sequence set forth in SEQ ID NO: 1:
(1) Q148G + Q149M + P150R, (2) S107A, (3) E152R + V153P + F154H + N155T, (4) S107K, (5) Q148P + Q149G + P150L, (6) A46C + I47Y + T48G + L49H, (7) E152T + V153F + N155T, (8) S107R, (9) V104M, (10) E152G + V153I + F154K + N155R, (11) S105C, (12) Q148C + Q149L + P150R + R151K, (13) Q148A + Q149G + P150C, (14) E152W + V153T + F154L + N155H, (15) S105G, (16) I47R + T48G + L49T, (17) D35G + D36N, (18) V33I + D35R + D36G, (19) V104A, and (20) I47V + T48R + L49K.

The present invention provides an adenosine deaminase variant, and the adenosine deaminase variant has a change in any one of amino acid positions relative to the amino acid sequence set forth in SEQ ID NO: 170. In some embodiments, the substitution is a substitution that occurs at one or more of the following sites of the amino acid sequence set forth in SEQ ID NO: 170:
S15, D16, H17, E18, F19, N20, D21, E22, Y23, W24, M25, R26, H27, A28, L29, T30, K33, R34, A35, R36, V41, V43, L47, L49, N51, N59, A61, I62, L64, A69, E72, G80, L81, V82, L83, Q84, N85, Y86, I89, D90, A91, T92, V95, F97, A106, R111, I112, S113, R114, L115, F117, V119, R120, N121, S122, K123, R124, N132, V133, L134, N135, P137, G138, M139, N140, H141, R142, E144, D160, V168, F169, and N170.

Further, in some embodiments, the substitution is a substitution that occurs at one or more of the following sites of the amino acid sequence set forth in SEQ ID NO: 170:
S15T, S15G, D16E, D16N, H17C, H17K, E18D, E18K, F19C, F19Y, F19S, N20Q, N20L, N20S, D21Q, E22D, Y23F, W24F, M25L, M25V, R26K, R26T, H27R, A28C, L29I, T30E, K33R, K33S, R34K, A35S, R36Q, L49F, N51G, L64M, L64Q, L64P, G80A, L81N, V82A, V82T, L83I, L83Q, Q84N, N85S, Y86W, I89E, I89L, D90G, A91C, A91T, T92D, A106C, I112L, S113K, R114K, V119L, S122N, S122P, R124H, R124T, N132K, V133I, L134F, N135H, N135S, P137F, G138A, M139L, N140K, H141A, R142L, R142S, E144H, D160E, V168A, F169C, F169V, and N170D.

In some specific embodiments, the substitution is a substitution that occurs at a combination of the following sites of the amino acid sequence set forth in SEQ ID NO: 170:
(1) S15T + D16E + H17K + F19Y + N20Q (adenosine deaminase 004V2),
(2) E22D + Y23F + W24F + R26K + H27R + L29I (adenosine deaminase 004V3),
(3) K33R + R34K + A35S (adenosine deaminase 004V4),
(4) G80A + L81N + V82A + L83I + Q84N + N85S + Y86W (adenosine deaminase 004V7),
(5) I89L + D90G + A91T + T92D (adenosine deaminase 004V8),
(6) I112L + S113K + R114K (adenosine deaminase 004V10),
(7) N132K + V133I + L134F + N135H (adenosine deaminase 004V12),
(8) P137F + G138A + M139L (adenosine deaminase 004V13),
(9) E18K + F19S + N20L (adenosine deaminase 004V14),
(10) M25L (adenosine deaminase 004V15),
(11) S15G + D16N + H17C (adenosine deaminase 004V16),
(12) L64Q + S122P + V168A (adenosine deaminase 004V17),
(13) R36Q + L64Q + S122P + F169V (adenosine deaminase 004V18),
(14) R36Q + V119L + V168A (adenosine deaminase 004V19),
(15) R36Q + V119L + N170D (adenosine deaminase 004V20),
(16) L64Q + V119L + V168A (adenosine deaminase 004V21),
(17) F169C (adenosine deaminase 004V22),
(18) L64Q + V119L + V168A (adenosine deaminase 004V23),
(19) L64P + R124T + F169V (adenosine deaminase 004V24),
(20) I89E + A91C (adenosine deaminase 004V25),
(21) L64Q + V119L + F169V (adenosine deaminase 004V26),
(22) L64M (adenosine deaminase 004V27),
(23) L64Q + S122P + N170D (adenosine deaminase 004V28),
(24) E18D + F19C + N20S (adenosine deaminase 004V29),
(25) L64P + V119L + N170D (adenosine deaminase 004V30),
(26) S122N + R124H + N135S + D160E (adenosine deaminase 004V31),
(27) R26T + H27R + A28C (adenosine deaminase 004V32),
(28) V82T + L83Q (adenosine deaminase 004V33),
(29) M25V (adenosine deaminase 004V34),
(30) D21Q + T30E + K33 S (adenosine deaminase 004V35),
(31) N140K + H141A + R142S (adenosine deaminase 004V36),
(32) L49F + N51G (adenosine deaminase 004V37),
(33) A106C (adenosine deaminase 004V38),
(34) R142L + E144H (adenosine deaminase 004V39),
(35) E22R + Y23F + W24P + M25H (adenosine deaminase 004V40), and/or
(36) L64Q + R124T + V168A (adenosine deaminase 004V41).

In the present invention, the expression "retaining the deamination activity", for example, "retaining the deamination activity of the amino acid sequence set forth in SEQ ID NO: 1", or "retaining the deamination activity of the amino acid sequence set forth in SEQ ID NO: 170", may mean completely retaining a deamination activity of an adenosine deaminase in an original sequence, or partially retaining the deamination activity of the adenosine deaminase in the original sequence, for example, retaining 30%, 40%, 50%, 60%, 70%, 80%, 90%, 95%, or 99% of the deamination activity. In other embodiments, an adenosine deaminase having a modified sequence, such as an adenosine deaminase having an amino acid substituted sequence, may have a deamination activity higher than that of the adenosine deaminase of the original sequence.

The adenosine deaminase provided by the present invention can act on any polynucleotide, including DNA, RNA, and DNA-RNA hybrids. In certain embodiments, the adenosine deaminase can deaminate a target adenine (A) of a polynucleotide containing DNA. In certain embodiments, the adenosine deaminase can deaminate a target adenine (A) of a polynucleotide containing RNA.

In some embodiments, the substitution is a conservative substitution.

In the present invention, the term "medium stringency condition", "medium-high stringency condition", "high stringency condition", or "very high stringency condition" indicates a condition for nucleic acid hybridization and washing. For guidance on performing a hybridization reaction, see Current Protocols in Molecular Biology, John Wiley&Sons, N.Y. (1989), 6.3.1-6.3.6, which is incorporated herein by reference. Aqueous and non-aqueous methods are described in this document and either may be used. For example, specific hybridization conditions are as follows: (1) low stringency hybridization condition: hybridization is performed in 6 × sodium chloride/sodium citrate (SSC) at about 45°C, then washing is performed twice in 0.2 × SSC, 0.1% SDS at at least 50°C (wash temperature can be increased to 55°C in the low stringency condition), (2) medium stringency hybridization condition; hybridization is performed in 6 × SSC at about 45°C, then washing is performed once or more in 0.2 × SSC, 0.1% SDS at 60°C, (3) high stringency hybridization condition: hybridization is performed in 6 × SSC at about 45°C, and then washing is performed once or more in 0.2 × SSC, 0.1% SDS at 65°C, and the condition is preferred, (4) very high stringency hybridization condition: hybridization is performed in 0.5 M sodium phosphate, 7% SDS, at 65°C, then washing is performed once or more in 0.2 × SSC, 1% SDS at 65°C.

An amino acid sequence of an adenosine deaminase 005V1 is as follows:

MSELNDAYWMKQALALAQKAREQGEVPVGAILVLDDEVIGQGWNRAITLHDPT
AHAEIMALQQGGQIVQNYRLLNATLYVTFEPCVMCAGAMVHSRIKRLVYGVSNSKRG
AAGSLLNVLNYPGMNHQIEITAGVMANECSEMLCQFYQQPREVFNAEREARRLNQPD
RAD (SEQ ID NO. 1).

An amino acid sequence of an adenosine deaminase 004V1 is as follows:

MYNAPRFFCRSAAVSDHEFNDEYWMRHALTLAKRAREEGEVPVGAVLVLNNQV
IGEGWNRAIGLHDPTAHAEIMALRQGGLVLQNYRLIDATLYVTFEPCVMCAGAMVHS
RISRLVFGVRNSKRGAAGSLINVLNYPGMNHRVEITEGILAESCSAMLCDFYRWPREV
FNALKKARQEEG (SEQ ID NO: 170).

A nucleotide sequence of the adenosine deaminase 005V1 is as follows:

ATGAGTGAGCTGAATGATGCTTACTGGATGAAACAGGCACTCGCTTTAGCTCA
GAAGGCCCGGGAACAGGGAGAAGTTCCAGTGGGCGCTATTCTGGTGCTGGATGAT
GAAGTGATAGGACAGGGATGGAATAGAGCCATCACCCTGCACGACCCCACCGCCC
ACGCCGAGATCATGGCCCTGCAGCAGGGCGGCCAGATCGTGCAGAACTACCGGCT
GCTGAACGCCACCCTGTACGTGACCTTCGAGCCCTGCGTGATGTGCGCCGGCGCCA
TGGTGCACAGCAGGATCAAGAGACTGGTCTACGGCGTGAGCAACTCAAAAAGAGG
CGCCGCCGGCAGCCTGCTGAACGTGCTGAACTACCCCGGCATGAACCACCAGATC
GAGATCACCGCCGGCGTGATGGCCAACGAGTGCAGCGAGATGCTGTGCCAGTTCT
ATCAGCAGCCTAGGGAAGTGTTCAATGCTGAGCGTGAGGCTAGGCGGCTGAACCA
ACCTGATAGAGCTGAC (SEQ ID NO. 2).

A nucleotide sequence of the adenosine deaminase 004V1 is as follows:

atgtacaatgcccctagattcttttgtagatctgctgccgtgtctgatcatgagtttaacgatgagtactggatgagacacgccctga
cactggccaagagggctcgggaggaaggagaggtgccagtgggagctgtgctggtgctgaataatcaggtcattggagaaggatgg
aatagagccatcggcctgcacgaccccaccgcccacgccgagatcatggccctgagacagggcggcctggtgctgcagaactaccg
gctgatcgacgccaccctgtacgtgaccttcgagccctgcgtgatgtgcgccggcgccatggtgcatagcagaatcagcagactggtg
ttcggcgtgagaaactcaaagagaggcgccgccggcagcctgatcaacgtgctgaactaccccggcatgaaccacagagtggagat
caccgagggcatcctggccgagagctgcagcgccatgctgtgcgacttctataggtggcctagggaggtgttcaatgccctgaagaag
gccagacaggaggagggc (SEQ ID NO. 156).

### <Base Editor Fusion Protein>

A second aspect of the present invention provides a base editor fusion protein containing the adenosine deaminase according to the first aspect of the present invention, and a nucleic acid programmable nucleotide binding domain.

In the present invention, the nucleic acid programmable nucleotide binding domain, when in conjunction with a guide polynucleotide (for example, gRNA), can specifically bind to a target polynucleotide sequence (that is, through a complementary base pairing sequence between a base of the guide nucleic acid to be bound and a base of the target polynucleotide), thereby positioning the base editor to a target nucleic acid sequence that needs to be edited. In some embodiments, the target polynucleotide sequence includes single-stranded DNA or double-stranded DNA. In some embodiments, the target polynucleotide sequence includes RNA. In some embodiments, the target polynucleotide sequence includes a DNA-RNA hybrid. It should be understood that the nucleic acid programmable nucleotide binding domain may include a nucleic acid programmable protein that binds RNA.

In some embodiments of the present invention, the nucleic acid programmable nucleotide binding domain in the base editor is a Cas protein or an AGO protein. The Cas protein or the AGO protein includes a naturally occurring Cas protein or AGO protein, as well as orthologs thereof or modified or engineered versions thereof. For example, in some embodiments, the Cas protein or the AGO protein may be a protein including an amino acid sequence having at least 60%, at least 65%, at least 70%, at least 75%, at least 80%, at least 85%, at least 90%, at least 95%, at least 96%, at least 97%, at least 98%, at least 99%, or at least 99.5% identity to an amino acid sequence of the naturally occurring Cas protein or AGO protein. In other embodiments, the Cas protein further includes a protein in its nickase or nuclease-inactive form.

In some embodiments, non-limiting examples of the Cas protein that can be used as the nucleic acid programmable nucleotide binding domain include Cas1, Cas1B, Cas2, Cas3, Cas4, Cas5, Cas5d, Cas5t, Cas5h, Cas5a, Cas6, Cas7, Cas8, Cas8a, Cas8b, Cas8c, Cas9 (also known as Csn1 or Csx12), Cas10, Cas10d, Cas12a/Cpfl, Cas12b/C2cl, Cas12c/C2c3, Cas12d/CasY, Cas12e/CasX, Cas12f (C2c10/Cas14), Cas12g, Cas12h, Cas12i, Cas12j, Cas12k/C2c5, Cas12l, Cas12m, Cas12n, Cas13a (C2c2), cas13b, Cas13c, Cas13d, Csy1, Csy2, Csy3, Csy4, Css1, Css2, Cse5e, Csc2, Csa5, Csn1, Csn2, Csm1, Csm2, Csm3, Csm4, Csm5, Csm6, Cmr1, Cmr2, Cmr3, Cmr4, Cmr5, Cmr6, Csb1, Csb2, Csb3, Csx17, Csx14, Csx10, Csx16, Csx1, Csx1S, Csx11, Csf1, Csf2, CsO, Csf4, Csd1, Csd2, Cst1, Cst2, Csh1, Csh2, Csa1, Csa2, Csa3, Csa4, Csa5, class II Cas effector proteins, type V Cas effector proteins, class VI Cas proteins, CARF, DinG, orthologs thereof, or modified or engineered versions thereof. Although not specifically listed in this disclosure, other nucleic acid programmable nucleotide binding domains are also within the scope of this disclosure.

In some specific embodiments of the present invention, the Cas protein is selected from a Cas9 family, a Cas12 family, and a Cas13 family, such as, but not limited to, Cas9, Cas12a (Cpf1), Cas12b (C2c1), Cas12c (C2c3), Cas12d (CasY), Cas12e (CasX), Cas12f (C2c10/Cas14), Cas12g, Cas12h, Cas12i, Cas12j, Cas12k (C2c5), Cas12l, Cas12m, Cas12n, Cas13a (C2c2), Cas13b, Cas13c, Cas13d, orthologs thereof, or modified or engineered versions thereof. **In** some specific embodiments of the present invention, the Cas protein includes a nuclease-inactive form of the above Cas protein, such as dCas9, dCas12a, dCas12b, dCas12c, dCas12d, dCas12e, dCas12f, dCas12g, dCas12h, dCas12i, dCas12j, dCas12k, dCas12l, dCas12m, dCas12n, dCas13a, dcas13b, dCas13c, and dCas13d. **In** some specific embodiments of the present invention, the Cas protein further includes a nickase form of the above protein, such as nCas9, but not limited to nCas9.

**In** some preferred embodiments of the present invention, the nucleic acid programmable nucleotide binding domain is Cas9. **In** some specific embodiments of the present invention, the Cas9 is S. pyogenes (SpCas9), S. aureus (SaCas9), or S. thermophilus 1 (St1Cas9). In some preferred embodiments of the present invention, Cas9 is S. pyogenes (SpCas9).

In some more preferred embodiments of the present invention, the Cas9 may be a nucleaseactive Cas9, a Cas9 nickase (nCas9), or a nuclease-inactive Cas9 (dCas9).

In some further preferred embodiments of the present invention, the nucleic acid programmable nucleotide binding domain is a Cas9 nickase (nCas9). In other further preferred embodiments of the present invention, the nucleic acid programmable nucleotide binding domain includes an amino acid sequence having at least 80%, at least 85%, at least 90%, at least 95%, at least 96%, at least 97%, at least 98%, at least 99%, or at least 99.5% identity to an amino acid sequence of the Cas9 nickase (nCas9) provided herein.

In some embodiments of the present invention, the adenosine deaminase and the nucleic acid programmable nucleotide binding domain are directly fused/linked to form a fusion protein, or are fused/linked through a linker to form a fusion protein. There is no particular limitation on the order of fusion/linking of the adenosine deaminase and the nucleic acid programmable nucleotide binding domain, for example, the adenosine deaminase can be at an N-terminal of the base editor, or the nucleic acid programmable nucleotide binding domain may be located at the N-terminal of the base editor.

In embodiments where the adenosine deaminase is directly fused with the nucleic acid programmable nucleotide binding domain, an exemplary base editor fusion protein has the following structure:
NH₂-[adenosine deaminase]-[napDNAbp]-COOH,
NH₂-[napDNAbp]-[adenosine deaminase]-COOH, or
NH₂-[N-terminal fragment of a napDNAbp]-[adenosine deaminase]-[C-terminal fragment of a napDNAbp]-COOH.

In embodiments where the adenosine deaminase is fused with the nucleic acid programmable nucleotide binding domain by a linker, an exemplary base editor fusion protein has the following structure:
NH₂-[adenosine deaminase]-[optional linker]-[napDNAbp]-COOH,
NH₂-[napDNAbp]-[optional linker]-[adenosine deaminase]-COOH, or
NH₂-[N-terminal fragment of a napDNAbp]-[optional linker]-[adenosine deaminase]-[optional linker]-[C-terminal fragment of a napDNAbp]-COOH.

In some embodiments, a domain of the nuclear base editor is fused by a linker including the following amino acid sequence:

| Sequence name | Amino acid sequence |
|---|---|
| Linker sequence 1 | GGGGS (SEQ ID NO: 171) |
| Linker sequence 2 | SGGS (SEQ ID NO: 172) |
| Linker sequence 3 | SGGSSGSETPGTSESATPESSGGS (SEQ ID NO: 173) |
| Linker sequence 4 | SGGSSGGSSGSETPGTSESATPESSGGSSGGS (SEQ ID NO: 174) |
| Linker sequence 5 | GGSGGSPGSPAGSPTSTEEGTSESATPESGPGTSTEPSEGSAPGSPA |
| | GSPTSTEEGTSTEPSEGSAPGTSTEPSEGSAPGTSESATPESGPGSE PATSGGSGGS (SEQ ID NO: 175) |
| Linker sequence 6 | SGSETPGTSESATPES (also known as XTEN short peptide or XTEN linker) (SEQ ID NO: 176) |
| Linker sequence 7 | SGGSSGGSSGSETPGTSESATPES (SEQ ID NO: 177) |
| Linker sequence 8 | SGGSSGGSSGSETPGTSESATPESSGGSSGGSSGGSSGGS (SEQ ID NO: 178) |
| Linker sequence 9 | SGGSSGGSSGSETPGTSESATPESSGGSSGGSSGGSSGGSSGSETP GTSESATPESSGGSSGGS (SEQ ID NO: 179) |
| Linker sequence 10 | PGSPAGSPTSTEEGTSESATPESGPGTSTEPSEGSAPGSPAGSPTSTE EGTSTEPSEGSAPGTSTEPSEGSAPGTSESATPESGPGSEPATS (SEQ ID NO: 180) |

In some embodiments of the present invention, the base editor further includes at least one nuclear localization signal sequence (NLS sequence), and the NLS sequence may be selected from amino acid sequences in the following table:

| Sequence name | Amino acid sequence |
|---|---|
| NLS sequence 1 | KRTADGSEFESPKKKRKV (SEQ ID NO: 181) |
| NLS sequence 2 | KRPAATKKAGQAKKKK (SEQ ID NO: 182) |
| NLS sequence 3 | KKTELQTTNAENKTKKL (SEQ ID NO: 183) |
| NLS sequence 4 | KRGINDRNFWRGENGRKTR (SEQ ID NO: 184) |
| NLS sequence 5 | RKSGKIAAIVVKRPRK (SEQ ID NO: 185) |
| NLS sequence 6 | PKKKRKV (SEQ ID NO: 186) |
| NLS sequence 7 | MDSLLMNRRKFLYQFKNVRWAKGRRETY (SEQ ID NO: 187) |
| NLS sequence 8 | MKRTADGSEFESPKKKRKV (SEQ ID NO: 188) |
| NLS sequence 9 | SGGSKRTADGSEFEPKKKRKV (SEQ ID NO: 189) |

In some embodiments of the present invention, the nuclear localization signal sequence can be located at an N-terminal, a C-terminal, or both terminals of the base editor, or located between the adenosine deaminase and the nucleic acid programmable nucleotide binding domain. In some embodiments of the present invention, the nuclear localization signal sequence can be fused directly in the base editor, or fused in the base editor by a linker.

An exemplary structure of the base editor including the nuclear localization signal sequence is as follows:
NH₂-[NLS]-[adenosine deaminase]-[napDNAbp]-COOH,
NH₂-[adenosine deaminase]-[NLS]-[napDNAbp]-COOH,
NH₂-[adenosine deaminase]-[napDNAbp]-[NLS]-COOH,
NH₂-[NLS]-[napDNAbp]-[adenosine deaminase]-COOH,
NH₂-[napDNAbp]-[NLS]-[adenosine deaminase]-COOH,
NH₂-[napDNAbp]-[adenosine deaminase]-[NLS]-COOH,
NH₂-[NLS]-[adenosine deaminase]-[optional linker]-[napDNAbp]-COOH,
NH₂-[adenosine deaminase]-[optional linker]-[NLS]-[optional linker]-[napDNAbp]-COOH,
NH₂-[adenosine deaminase]-[optional linker]-[napDNAbp]-[NLS]-COOH,
NH₂-[NLS]-[napDNAbp]-[optional linker]-[adenosine deaminase]-COOH,
NH₂-[napDNAbp]-[optional linker]-[NLS]-[optional linker]-[adenosine deaminase]-COOH,
NH₂-[napDNAbp]-[optional linker]-[adenosine deaminase]-[NLS]-COOH, or
NH₂-[NLS]-[adenosine deaminase]-[optional linker]-[napDNAbp]-[NLS]-COOH.

In some preferred embodiments of the present invention, the base editor has the following structure:
NH₂-[NLS]-[adenosine deaminase]-[optional linker]-[napDNAbp]-[NLS]-COOH, or
NH₂-[NLS]-[N-terminal fragment of a napDNAbp]-[optional linker]-[adenosine deaminase]-[optional linker]-[C-terminal fragment of a napDNAbp]-[NLS]-COOH.

In some further preferred embodiments of the present invention, the base editor includes one or more of the following sequences:
(i) an amino acid sequence set forth in SEQ ID NO: 190 or SEQ ID NO: 3,
(ii) an amino acid sequence that has at least 80%, 82%, 85%, 87%, 90%, 92%, 95%, 96%, 97%, 98%, or 99% identity to the amino acid sequence set forth in SEQ ID NO: 190 or SEQ ID NO: 3, and which retains a polynucleotide binding and base editing activity of the amino acid sequence set forth in SEQ ID NO: 190 or SEQ ID NO: 3,
(iii) an amino acid sequence in which one or more amino acid residues are added, substituted, deleted, or inserted into the amino acid sequence set forth in SEQ ID NO: 190 or SEQ ID NO: 3, and which retains the polynucleotide binding and base editing activity of the amino acid sequence set forth in SEQ ID NO: 190 or SEQ ID NO: 3, or
(iv) an amino acid sequence encoded by a nucleotide sequence, the nucleotide sequence hybridizing, under a stringency condition, with a polynucleotide sequence encoding the amino acid sequence set forth in SEQ ID NO: 190 or SEQ ID NO: 3, the amino acid sequence retaining the polynucleotide binding and base editing activity of the amino acid sequence set forth in SEQ ID NO: 190 or SEQ ID NO: 3, and the stringency condition being a medium stringency condition, a medium-high stringency condition, a high stringency condition, or a very high stringency condition.

Sequences of base editors constituted by the adenosine deaminase 004V1 and nCas9 and the adenosine deaminase 005V1 and nCas9 are as follows.

Amino acid sequence of 004V1-nCas9:
MKRTADGSEFESPKKKRKVMYNAPRFFCRSAAVSDHEFNDEYWMRHALTLAKR AREEGEVPVGAVLVLNNQVIGEGWNRAIGLHDPTAHAEIMALRQGGLVLQNYRLIDA TLYVTFEPCVMCAGAMVHSRISRLVFGVRNSKRGAAGSLINVLNYPGMNHRVEITEGI LAESCSAMLCDFYRWPREVFNALKKARQEEG*SGGSSGGSSGSETPGTSESATPESSGGSS GGS***DKKYSIGLAIGTNSVGWAVITDEYKVPSKKFKVLGNTDRHSIKKNLIGALLFD SGETAEATRLKRTARRRYTRRKNRICYLQEIFSNEMAKVDDSFFHRLEESFLVEED KKHERHPIFGNIVDEVAYHEKYPTIYHLRKKLVDSTDKADLRLIYLALAHMIKFR GHFLIEGDLNPDNSDVDKLFIQLVQTYNQLFEENPINASGVDAKAILSARLSKSRR LENLIAQLPGEKKNGLFGNLIALSLGLTPNFKSNFDLAEDAKLQLSKDTYDDDLD NLLAQIGDQYADLFLAAKNLSDAILLSDILRVNTEITKAPLSASMIKRYDEHHQDL TLLKALVRQQLPEKYKEIFFDQSKNGYAGYIDGGASQEEFYKFIKPILEKMDGTE ELLVKLNREDLLRKQRTFDNGSIPHQIHLGELHAILRRQEDFYPFLKDNREKIEKI LTFRIPYYVGPLARGNSRFAWMTRKSEETITPWNFEEVVDKGASAQSFIERMTNF DKNLPNEKVLPKHSLLYEYFTVYNELTKVKYVTEGMRKPAFLSGEQKKAIVDLL FKTNRKVTVKQLKEDYFKKIECFDSVEISGVEDRFNASLGTYHDLLKIIKDKDFL DNEENEDILEDIVLTLTLFEDREMIEERLKTYAHLFDDKVMKQLKRRRYTGWGR LSRKLINGIRDKQSGKTILDFLKSDGFANRNFMQLIHDDSLTFKEDIQKAQVSGQG DSLHEHIANLAGSPAIKKGILQTVKVVDELVKVMGRHKPENIVIEMARENQTTQK GQKNSRERMKRIEEGIKELGSQILKEHPVENTQLQNEKLYLYYLQNGRDMYVDQ ELDINRLSDYDVDHIVPQSFLKDDSIDNKVLTRSDKNRGKSDNVPSEEVVKKMKN YWRQLLNAKLITQRKFDNLTKAERGGLSELDKAGFIKRQLVETRQITKHVAQILD SRMNTKYDENDKLIREVKVITLKSKLVSDFRKDFQFYKVREINNYHHAHDAYLN AVVGTALIKKYPKLESEFVYGDYKVYDVRKMIAKSEQEIGKATAKYFFYSNIMNF FKTEITLANGEIRKRPLIETNGETGEIVWDKGRDFATVRKVLSMPQVNIVKKTEV QTGGFSKESILPKRNSDKLIARKKDWDPKKYGGFDSPTVAYSVLVVAKVEKGKSK KLKSVKELLGITIMERSSFEKNPIDFLEAKGYKEVKKDLIIKLPKYSLFELENGRK RMLASAGELQKGNELALPSKYVNFLYLASHYEKLKGSPEDNEQKQLFVEQHKH YLDEIIEQISEFSKRVILADANLDKVLSAYNKHRDKPIREQAENIIHLFTLTNLGAP AAFKYFDTTIDRKRYTSTKEVLDATLIHQSITGLYETRIDLSQLGGD**SGGSKRTADG SEFEPKKKRKV* (SEQ ID NO: 190), wherein, the bold sequence indicates a sequence derived from nCas9, the italic indicates a linker sequence, the double underlined sequence indicates a nuclear localization sequence, the single underlined sequence is a sequence of a deaminase 004V1, and the asterisk at a C-terminal indicates a stop codon position.

Amino acid sequence of 005V1-nCas9:
MKRTADGSEFESPKKKRKVMSELNDAYWMKQALALAQKAREQGEVPVGAILVL DDEVIGQGWNRAITLHDPTAHAEIMALQQGGQIVQNYRLLNATLYVTFEPCVMCAGA MVHSRIKRLVYGVSNSKRGAAGSLLNVLNYPGMNHOIEITAGVMANECSEMLCOFY OOPREVFNAEREARRLNOPDRAD*SGGSSGGSSGSETPGTSESATPESSGGSSGGS***DKKYS IGLAIGTNSVGWAVITDEYKVPSKKFKVLGNTDRHSIKKNLIGALLFDSGETAEAT RLKRTARRRYTRRKNRICYLQEIFSNEMAKVDDSFFHRLEESFLVEEDKKHERHP IFGNIVDEVAYHEKYPTIYHLRKKLVDSTDKADLRLIYLALAHMIKFRGHFLIEGD LNPDNSDVDKLFIQLVQTYNQLFEENPINASGVDAKAILSARLSKSRRLENLIAQL PGEKKNGLFGNLIALSLGLTPNFKSNFDLAEDAKLQLSKDTYDDDLDNLLAQIGD QYADLFLAAKNLSDAILLSDILRVNTEITKAPLSASMIKRYDEHHQDLTLLKALVR QQLPEKYKEIFFDQSKNGYAGYIDGGASQEEFYKFIKPILEKMDGTEELLVKLNR EDLLRKQRTFDNGSIPHQIHLGELHAILRRQEDFYPFLKDNREKIEKILTFRIPYYV GPLARGNSRFAWMTRKSEETITPWNFEEVVDKGASAQSFIERMTNFDKNLPNEK VLPKHSLLYEYFTVYNELTKVKYVTEGMRKPAFLSGEQKKAIVDLLFKTNRKVT VKQLKEDYFKKIECFDSVEISGVEDRFNASLGTYHDLLKIIKDKDFLDNEENEDIL EDIVLTLTLFEDREMIEERLKTYAHLFDDKVMKQLKRRRYTGWGRLSRKLINGIR DKQSGKTILDFLKSDGFANRNFMQLIHDDSLTFKEDIQKAQVSGQGDSLHEHIAN LAGSPAIKKGILQTVKVVDELVKVMGRHKPENIVIEMARENQTTQKGQKNSRER MKRIEEGIKELGSQILKEHPVENTQLQNEKLYLYYLQNGRDMYVDQELDINRLS DYDVDHIVPQSFLKDDSIDNKVLTRSDKNRGKSDNVPSEEVVKKMKNYWRQLLN AKLITQRKFDNLTKAERGGLSELDKAGFIKRQLVETRQITKHVAQILDSRMNTKY DENDKLIREVKVITLKSKLVSDFRKDFQFYKVREINNYHHAHDAYLNAVVGTALI KKYPKLESEFVYGDYKVYDVRKMIAKSEQEIGKATAKYFFYSNIMNFFKTEITLA NGEIRKRPLIETNGETGEIVWDKGRDFATVRKVLSMPQVNIVKKTEVQTGGFSKE SILPKRNSDKLIARKKDWDPKKYGGFDSPTVAYSVLVVAKVEKGKSKKLKSVKEL LGITIMERSSFEKNPIDFLEAKGYKEVKKDLIIKLPKYSLFELENGRKRMLASAGE LQKGNELALPSKYVNFLYLASHYEKLKGSPEDNEQKQLFVEQHKHYLDEIIEQIS EFSKRVILADANLDKVLSAYNKHRDKPIREQAENIIHLFTLTNLGAPAAFKYFDTTI DRKRYTSTKEVLDATLIHQSITGLYETRIDLSQLGGD**SGGSKRTADGSEFEPKKKRK V* (SEQ ID NO. 3), wherein, the bold sequence indicates a sequence derived from nCas9, the italic sequence indicates a linker sequence, a double underlined sequence indicates a nuclear localization sequence, the single underlined sequence is a sequence of an adenosine deaminase 005V1, and the asterisk indicates a stop codon position.

A nucleotide sequence of 005V1-nCas9 is shown below:

atgaaacggacagccgacggaagcgagttcgagtcaccaaagaagaagcggaaagtcATGAGTGAGCTGAAT
GATGCTTACTGGATGAAACAGGCACTCGCTTTAGCTCAGAAGGCCcggGAACAGGG
AGAAGTTCCAGTGGGCGCTATTCTGGTGctgGATGATGAAGTGATAGGACAGGGATG
GAATAGAgccATCACCctgCACGACCCCACCGCCCACGCCGAGATCATGGCCCTGCAG
CAGGGCGGCCAGATCGTGCAGAACTACCGGCTGCTGAACGCCACCCTGTACGTGA
CCttcGAGCCCTGCGTGATGTGCGCCGGCGCCATGGTGCACAGCAGGATCAAGAGAC
TGGTCTACGGCgtgAGCaactcaAAAagaGGCGCCGCCGGCAGCCTGCTGaacGTGCTGaac
tacCCCGGCATGAACCACCAGATCGAGATCACCGCCGGCGTGATGGCCAACGAGTGC
AGCGAGATGCTGtgcCAGTTCtatCAGCAGcctAGGGAAgtgttcaatGCTGAGCGTGAGGCT
AGGCGGCTGAACCAACCTGATAGAGCTGACtccggaggatctagcggaggctcctctggctctgagacac

ctggcacaagcgagagcgcaacacctgaaagcagcgggggcagcagcggggggtcagacaagaagtacagcatcggcctggcca
tcggcaccaactctgtgggctgggccgtgatcaccgacgagtacaaggtgcccagcaagaaattcaaggtgctgggcaacaccgacc
ggcacagcatcaagaagaacctgatcggagccctgctgttcgacagcggcgaaacagccgaggccacccggctgaagagaaccgc
cagaagaagatacaccagacggaagaaccggatctgctatctgcaagagatcttcagcaacgagatggccaaggtggacgacagctt
cttccacagactggaagagtccttcctggtggaagaggataagaagcacgagcggcaccccatcttcggcaacatcgtggacgaggt
ggcctaccacgagaagtaccccaccatctaccacctgagaaagaaactggtggacagcaccgacaaggccgacctgcggctgatcta
tctggccctggcccacatgatcaagttccggggccacttcctgatcgagggcgacctgaaccccgacaacagcgacgtggacaagct
gttcatccagctggtgcagacctacaaccagctgttcgaggaaaaccccatcaacgccagcggcgtggacgccaaggccatcctgtct
gccagactgagcaagagcagacggctggaaaatctgatcgcccagctgcccggcgagaagaagaatggcctgttcggaaacctgatt
gccctgagcctgggcctgacccccaacttcaagagcaacttcgacctggccgaggatgccaaactgcagctgagcaaggacacctac
gacgacgacctggacaacctgctggcccagatcggcgaccagtacgccgacctgtttctggccgccaagaacctgtccgacgccatc
ctgctgagcgacatcctgagagtgaacaccgagatcaccaaggcccccctgagcgcctctatgatcaagagatacgacgagcaccac
caggacctgaccctgctgaaagctctcgtgcggcagcagctgcctgagaagtacaaagagattttcttcgaccagagcaagaacggct
acgccggctacattgacggcggagccagccaggaagagttctacaagttcatcaagcccatcctggaaaagatggacggcaccgag
gaactgctcgtgaagctgaacagagaggacctgctgcggaagcagcggaccttcgacaacggcagcatcccccaccagatccacct
gggagagctgcacgccattctgcggcggcaggaagatttttacccattcctgaaggacaaccgggaaaagatcgagaagatcctgacc
ttccgcatcccctactacgtgggccctctggccaggggaaacagcagattcgcctggatgaccagaaagagcgaggaaaccatcacc
ccctggaacttcgaggaagtggtggacaagggcgcttccgcccagagcttcatcgagcggatgaccaacttcgataagaacctgccca
acgagaaggtgctgcccaagcacagcctgctgtacgagtacttcaccgtgtataacgagctgaccaaagtgaaatacgtgaccgaggg
aatgagaaagcccgccttcctgagcggcgagcagaaaaaggccatcgtggacctgctgttcaagaccaaccggaaagtgaccgtgaa
gcagctgaaagaggactacttcaagaaaatcgagtgcttcgactccgtggaaatctccggcgtggaagatcggttcaacgcctccctgg
gcacataccacgatctgctgaaaattatcaaggacaaggacttcctggacaatgaggaaaacgaggacattctggaagatatcgtgctg
accctgacactgtttgaggacagagagatgatcgaggaacggctgaaaacctatgcccacctgttcgacgacaaagtgatgaagcagc
tgaagcggcggagatacaccggctggggcaggctgagccggaagctgatcaacggcatccgggacaagcagtccggcaagacaat

cctggatttcctgaagtccgacggcttcgccaacagaaacttcatgcagctgatccacgacgacagcctgacctttaaagaggacatcca

gaaagcccaggtgtccggccagggcgatagcctgcacgagcacattgccaatctggccggcagccccgccattaagaagggcatcc

tgcagacagtgaaggtggtggacgagctcgtgaaagtgatgggccggcacaagcccgagaacatcgtgatcgaaatggccagagag

aaccagaccacccagaagggacagaagaacagccgcgagagaatgaagcggatcgaagagggcatcaaagagctgggcagcca

gatcctgaaagaacaccccgtggaaaacacccagctgcagaacgagaagctgtacctgtactacctgcagaatgggcgggatatgtac

gtggaccaggaactggacatcaaccggctgtccgactacgatgtggaccatatcgtgcctcagagctttctgaaggacgactccatcga

caacaaggtgctgaccagaagcgacaagaaccggggcaagagcgacaacgtgccctccgaagaggtcgtgaagaagatgaagaa

ctactggcggcagctgctgaacgccaagctgattacccagagaaagttcgacaatctgaccaaggccgagagaggcggcctgagcg

aactggataaggccggcttcatcaagagacagctggtggaaacccggcagatcacaaagcacgtggcacagatcctggactcccgga

tgaacactaagtacgacgagaatgacaagctgatccgggaagtgaaagtgatcaccctgaagtccaagctggtgtccgatttccggaa

ggatttccagttttacaaagtgcgcgagatcaacaactaccaccacgcccacgacgcctacctgaacgccgtcgtgggaaccgccctg

atcaaaaagtaccctaagctggaaagcgagttcgtgtacggcgactacaaggtgtacgacgtgcggaagatgatcgccaagagcgag

caggaaatcggcaaggctaccgccaagtacttcttctacagcaacatcatgaactttttcaagaccgagattaccctggccaacggcgag

atccggaagcggcctctgatcgagacaaacggcgaaaccggggagatcgtgtgggataagggccgggattttgccaccgtgcggaa

agtgctgagcatgccccaagtgaatatcgtgaaaaagaccgaggtgcagacaggcggcttcagcaaagagtctatcctgcccaagag

gaacagcgataagctgatcgccagaaagaaggactgggaccctaagaagtacggcggcttcgacagccccaccgtggcctattctgt

gctggtggtggccaaagtggaaaagggcaagtccaagaaactgaagagtgtgaaagagctgctggggatcaccatcatggaaagaa

gcagcttcgagaagaatcccatcgactttctggaagccaagggctacaaagaagtgaaaaaggacctgatcatcaagctgcctaagtac

tccctgttcgagctggaaaacggccggaagagaatgctggcctctgccggcgaactgcagaagggaaacgaactggccctgccctcc

aaatatgtgaacttcctgtacctggccagccactatgagaagctgaagggctcccccgaggataatgagcagaaacagctgtttgtgga

acagcacaagcactacctggacgagatcatcgagcagatcagcgagttctccaagagagtgatcctggccgacgctaatctggacaaa

gtgctgtccgcctacaacaagcaccgggataagcccatcagagagcaggccgagaatatcatccacctgtttaccctgaccaatctggg

agcccctgccgccttcaagtactttgacaccaccatcgaccggaagaggtacaccagcaccaaagaggtgctggacgccaccctgatc

caccagagcatcaccggcctgtacgagacacggatcgacctgtctcagctgggaggtgactctggcggctcaaaaagaaccgccgac

ggcagcgaattcgagcccaagaagaagaggaaagtc (SEQ ID NO. 4).

Further, the present invention also provides a mutant obtained after amino acid substitution based on the adenosine deaminase 004V1, including: adenosine deaminase 004V2, adenosine deaminase 004V3, adenosine deaminase 004V4, adenosine deaminase 004V7, adenosine deaminase 004V8, adenosine deaminase 004V10, adenosine deaminase 004V12, adenosine deaminases 004V13 to 004V41, as follow.

| Mutant name | Substitution based on adenosine deaminase 004V1 (SEQ ID NO: 170) |
|---|---|
| Adenosine deaminase 004V2 | S15T + D16E + H17K + F19Y + N20Q |
| Adenosine deaminase 004V3 | E22D + Y23F + W24F + R26K + H27R + L29I |
| Adenosine deaminase 004V4 | K33R + R34K + A35S |
| Adenosine deaminase 004V7 | G80A + L81N + V82A + L83I + Q84N + N85S + Y86W |
| Adenosine deaminase 004V8 | I89L + D90G + A91T + T92D |
| Adenosine deaminase 004V10 | I112L + S113K + R114K |
| Adenosine deaminase 004V12 | N132K + V133I + L134F + N135H |
| Adenosine deaminase 004V13 | P137F + G138A + M139L |
| Adenosine deaminase 004V14 | E18K + F19S + N20L |
| Adenosine deaminase 004V15 | M25L |
| Adenosine deaminase 004V16 | S15G + D16N + H17C |
| Adenosine deaminase 004V17 | L64Q + S122P + V168A |
| Adenosine deaminase 004V18 | R36Q + L64Q + S122P + F169V |
| Adenosine deaminase 004V19 | R36Q + V119L + V168A |
| Adenosine deaminase 004V20 | R36Q + V119L + N170D |
| Adenosine deaminase 004V21 | L64Q + V119L + V168A |
| Adenosine deaminase 004V22 | F169C |
| Adenosine deaminase 004V23 | L64Q + V119L + V168A |
| Adenosine deaminase 004V24 | L64P + R124T + F169V |
| Adenosine deaminase 004V25 | I89E + A91C |
| Adenosine deaminase 004V26 | L64Q + V119L + F169V |
| Adenosine deaminase 004V27 | L64M |
| Adenosine deaminase 004V28 | L64Q + S122P + N170D |
| Adenosine deaminase 004V29 | E18D + F19C + N20S |
| Adenosine deaminase 004V30 | L64P + V119L + N170D |
| Adenosine deaminase 004V31 | S122N + R124H + N135S + D160E |
| Adenosine deaminase 004V32 | R26T + H27R + A28C |
| Adenosine deaminase 004V33 | V82T + L83Q |
| Adenosine deaminase 004V34 | M25V |
| Adenosine deaminase 004V35 | D21Q + T30E + K33S |
| Adenosine deaminase 004V36 | N140K + H141A + R142S |
| Adenosine deaminase 004V37 | L49F + N51G |
| Adenosine deaminase 004V38 | A106C |
| Adenosine deaminase 004V39 | R142L + E144H |
| Adenosine deaminase 004V40 | E22R + Y23F + W24P + M25H |
| Adenosine deaminase 004V41 | L64Q + R124T + V168A |

An exemplary sequence of a base editor constituted by each of the above adenosine deaminases is as follows.

An amino acid sequence of 004V2-nCas9 is as follows: 
MKRTADGSEFESPKKKRKVMYNAPRFFCRSAAVTEKEYQDEYWMRHALTLAKR
AREEGEVPVGAVLVLNNQVIGEGWNRAIGLHDPTAHAEIMALRQGGLVLQNYRLIDA
TLYVTFEPCVMCAGAMVHSRISRLVFGVRNSKRGAAGSLINVLNYPGMNHRVEITEGI
LAESCSAMLCDFYRWPREVFNALKKARQEEG
 
SGGSSGGSSGSETPGTSESATPESSGGSSGGSDKKYSIGLAIGTNSVGWAVITDEYK
VPSKKFKVLGNTDRHSIKKNLIGALLFDSGETAEATRLKRTARRRYTRRKNRICY
LQEIFSNEMAKVDDSFFHRLEESFLVEEDKKHERHPIFGNIVDEVAYHEKYPTIYH
LRKKLVDSTDKADLRLIYLALAHMIKFRGHFLIEGDLNPDNSDVDKLFIQLVQTY
NQLFEENPINASGVDAKAILSARLSKSRRLENLIAQLPGEKKNGLFGNLIALSLGL
TPNFKSNFDLAEDAKLQLSKDTYDDDLDNLLAQIGDQYADLFLAAKNLSDAILLS
DILRVNTEITKAPLSASMIKRYDEHHQDLTLLKALVRQQLPEKYKEIFFDQSKNG
YAGYIDGGASQEEFYKFIKPILEKMDGTEELLVKLNREDLLRKQRTFDNGSIPHQI
HLGELHAILRRQEDFYPFLKDNREKIEKILTFRIPYYVGPLARGNSRFAWMTRKS
 
EETITPWNFEEVVDKGASAQSFIERMTNFDKNLPNEKVLPKHSLLYEYFTVYNEL
TKVKYVTEGMRKPAFLSGEQKKAIVDLLFKTNRKVTVKQLKEDYFKKIECFDSV
EISGVEDRFNASLGTYHDLLKIIKDKDFLDNEENEDILEDIVLTLTLFEDREMIEER
LKTYAHLFDDKVMKQLKRRRYTGWGRLSRKLINGIRDKQSGKTILDFLKSDGFA
NRNFMQLIHDDSLTFKEDIQKAQVSGQGDSLHEHIANLAGSPAIKKGILQTVKVV
DELVKVMGRHKPENIVIEMARENQTTQKGQKNSRERMKRIEEGIKELGSQILKE
HPVENTQLQNEKLYLYYLQNGRDMYVDQELDINRLSDYDVDHIVPQSFLKDDSID
NKVLTRSDKNRGKSDNVPSEEVVKKMKNYWRQLLNAKLITQRKFDNLTKAERG
GLSELDKAGFIKRQLVETRQITKHVAQILDSRMNTKYDENDKLIREVKVITLKSK
LVSDFRKDFQFYKVREINNYHHAHDAYLNAVVGTALIKKYPKLESEFVYGDYKVY
DVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDW
DPKKYGGFDSPTVAYSVLVVAKVEKGKSKKLKSVKELLGITIMERSSFEKNPIDFL
EAKGYKEVKKDLIIKLPKYSLFELENGRKRMLASAGELQKGNELALPSKYVNFL
YLASHYEKLKGSPEDNEQKQLFVEQHKHYLDEIIEQISEFSKRVILADANLDKVLS
AYNKHRDKPIREQAENIIHLFTLTNLGAPAAFKYFDTTIDRKRYTSTKEVLDATLI
HQSITGLYETRIDLSQLGGDSGGSKRTADGSEFEPKKKRKV* (SEQ ID NO: 191),
 wherein, the bold sequence indicates a sequence derived from nCas9, the italic sequence indicates a linker sequence, the double underlined sequence indicates a nuclear localization sequence, the single underlined sequence is a sequence of an adenosine deaminase 004V2, and the asterisk indicates a stop codon position.

An amino acid sequence of 004V3-nCas9 is as follows: 
MKRTADGSEFESPKKKRKVMYNAPRFFCRSAAVSDHEFNDDFFMKRAITLAKRA
REEGEVPVGAVLVLNNQVIGEGWNRAIGLHDPTAHAEIMALRQGGLVLQNYRLIDATL
 
YVTFEPCVMCAGAMVHSRISRLVFGVRNSKRGAAGSLINVLNYPGMNHRVEITEGILA

ESCSAMLCDFYRWPREVFNALKKARQEEG
 
SGGSSGGSSGSETPGTSESATPESSGGSSGGSDKKYSIGLAIGTNSVGWAVITDEYK
VPSKKFKVLGNTDRHSIKKNLIGALLFDSGETAEATRLKRTARRRYTRRKNRICY
LQEIFSNEMAKVDDSFFHRLEESFLVEEDKKHERHPIFGNIVDEVAYHEKYPTIYH
LRKKLVDSTDKADLRLIYLALAHMIKFRGHFLIEGDLNPDNSDVDKLFIQLVQTY
NQLFEENPINASGVDAKAILSARLSKSRRLENLIAQLPGEKKNGLFGNLIALSLGL
TPNFKSNFDLAEDAKLQLSKDTYDDDLDNLLAQIGDQYADLFLAAKNLSDAILLS
DILRVNTEITKAPLSASMIKRYDEHHQDLTLLKALVRQQLPEKYKEIFFDQSKNG
YAGYIDGGASQEEFYKFIKPILEKMDGTEELLVKLNREDLLRKQRTFDNGSIPHQI
HLGELHAILRRQEDFYPFLKDNREKIEKILTFRIPYYVGPLARGNSRFAWMTRKS
EETITPWNFEEVVDKGASAQSFIERMTNFDKNLPNEKVLPKHSLLYEYFTVYNEL
TKVKYVTEGMRKPAFLSGEQKKAIVDLLFKTNRKVTVKQLKEDYFKKIECFDSV
EISGVEDRFNASLGTYHDLLKIIKDKDFLDNEENEDILEDIVLTLTLFEDREMIEER
LKTYAHLFDDKVMKQLKRRRYTGWGRLSRKLINGIRDKQSGKTILDFLKSDGFA
NRNFMQLIHDDSLTFKEDIQKAQVSGQGDSLHEHIANLAGSPAIKKGILQTVKVV
DELVKVMGRHKPENIVIEMARENQTTQKGQKNSRERMKRIEEGIKELGSQILKE
HPVENTQLQNEKLYLYYLQNGRDMYVDQELDINRLSDYDVDHIVPQSFLKDDSID
NKVLTRSDKNRGKSDNVPSEEVVKKMKNYWRQLLNAKLITQRKFDNLTKAERG
GLSELDKAGFIKRQLVETRQITKHVAQILDSRMNTKYDENDKLIREVKVITLKSK
LVSDFRKDFQFYKVREINNYHHAHDAYLNAVVGTALIKKYPKLESEFVYGDYKVY
DVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDW
DPKKYGGFDSPTVAYSVLVVAKVEKGKSKKLKSVKELLGITIMERSSFEKNPIDFL
 
EAKGYKEVKKDLIIKLPKYSLFELENGRKRMLASAGELQKGNELALPSKYVNFL
YLASHYEKLKGSPEDNEQKQLFVEQHKHYLDEIIEQISEFSKRVILADANLDKVLS
AYNKHRDKPIREQAENIIHLFTLTNLGAPAAFKYFDTTIDRKRYTSTKEVLDATLI
HQSITGLYETRIDLSQLGGDSGGSKRTADGSEFEPKKKRKV* (SEQ ID NO: 192),
 wherein, the bold sequence indicates a sequence derived from nCas9, the italic sequence indicates a linker sequence, the double underlined sequence indicates a nuclear localization sequence, the single underlined sequence is a sequence of a deaminase 004V3, and the asterisk indicates a stop codon position.

An amino acid sequence of 004V4-nCas9 is as follows: 
MKRTADGSEFESPKKKRKVMYNAPRFFCRSAAVSDHEFNDEYWMRHALTLARK
SREEGEVPVGAVLVLNNQVIGEGWNRAIGLHDPTAHAEIMALRQGGLVLQNYRLIDAT
LYVTFEPCVMCAGAMVHSRISRLVFGVRNSKRGAAGSLINVLNYPGMNHRVEITEGIL
AESCSAMLCDFYRWPREVFNALKKARQEEG
 
SGGSSGGSSGSETPGTSESATPESSGGSSGGSDKKYSIGLAIGTNSVGWAVITDEYK
VPSKKFKVLGNTDRHSIKKNLIGALLFDSGETAEATRLKRTARRRYTRRKNRICY
LQEIFSNEMAKVDDSFFHRLEESFLVEEDKKHERHPIFGNIVDEVAYHEKYPTIYH
LRKKLVDSTDKADLRLIYLALAHMIKFRGHFLIEGDLNPDNSDVDKLFIQLVQTY
NQLFEENPINASGVDAKAILSARLSKSRRLENLIAQLPGEKKNGLFGNLIALSLGL
TPNFKSNFDLAEDAKLQLSKDTYDDDLDNLLAQIGDQYADLFLAAKNLSDAILLS
DILRVNTEITKAPLSASMIKRYDEHHQDLTLLKALVRQQLPEKYKEIFFDQSKNG
YAGYIDGGASQEEFYKFIKPILEKMDGTEELLVKLNREDLLRKQRTFDNGSIPHQI
HLGELHAILRRQEDFYPFLKDNREKIEKILTFRIPYYVGPLARGNSRFAWMTRKS
EETITPWNFEEVVDKGASAQSFIERMTNFDKNLPNEKVLPKHSLLYEYFTVYNEL
TKVKYVTEGMRKPAFLSGEQKKAIVDLLFKTNRKVTVKQLKEDYFKKIECFDSV
 
EISGVEDRFNASLGTYHDLLKIIKDKDFLDNEENEDILEDIVLTLTLFEDREMIEER
LKTYAHLFDDKVMKQLKRRRYTGWGRLSRKLINGIRDKQSGKTILDFLKSDGFA
NRNFMQLIHDDSLTFKEDIQKAQVSGQGDSLHEHIANLAGSPAIKKGILQTVKVV
DELVKVMGRHKPENIVIEMARENQTTQKGQKNSRERMKRIEEGIKELGSQILKE
HPVENTQLQNEKLYLYYLQNGRDMYVDQELDINRLSDYDVDHIVPQSFLKDDSID
NKVLTRSDKNRGKSDNVPSEEVVKKMKNYWRQLLNAKLITQRKFDNLTKAERG
GLSELDKAGFIKRQLVETRQITKHVAQILDSRMNTKYDENDKLIREVKVITLKSK
LVSDFRKDFQFYKVREINNYHHAHDAYLNAVVGTALIKKYPKLESEFVYGDYKVY
DVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDW
DPKKYGGFDSPTVAYSVLVVAKVEKGKSKKLKSVKELLGITIMERSSFEKNPIDFL
EAKGYKEVKKDLIIKLPKYSLFELENGRKRMLASAGELQKGNELALPSKYVNFL
YLASHYEKLKGSPEDNEQKQLFVEQHKHYLDEIIEQISEFSKRVILADANLDKVLS
AYNKHRDKPIREQAENIIHLFTLTNLGAPAAFKYFDTTIDRKRYTSTKEVLDATLI
HQSITGLYETRIDLSQLGGDSGGSKRTADGSEFEPKKKRKV* (SEQ ID NO: 193),
 wherein, the bold sequence indicates a sequence derived from nCas9, the italic sequence indicates a linker sequence, the double underlined sequence indicates a nuclear localization sequence, the single underlined sequence is a sequence of a deaminase 004V4, and the asterisk indicates a stop codon position.

An amino acid sequence of 004V7-nCas9 is as follows: 
MKRTADGSEFESPKKKRKVMYNAPRFFCRSAAVSDHEFNDEYWMRHALTLAKR
AREEGEVPVGAVLVLNNQVIGEGWNRAIGLHDPTAHAEIMALRQGANAINSWRLIDA
TLYVTFEPCVMCAGAMVHSRISRLVFGVRNSKRGAAGSLINVLNYPGMNHRVEITEGI
LAESCSAMLCDFYRWPREVFNALKKARQEEG
 
SGGSSGGSSGSETPGTSESATPESSGGSSGGSDKKYSIGLAIGTNSVGWAVITDEYK
VPSKKFKVLGNTDRHSIKKNLIGALLFDSGETAEATRLKRTARRRYTRRKNRICY
LQEIFSNEMAKVDDSFFHRLEESFLVEEDKKHERHPIFGNIVDEVAYHEKYPTIYH
LRKKLVDSTDKADLRLIYLALAHMIKFRGHFLIEGDLNPDNSDVDKLFIQLVQTY
NQLFEENPINASGVDAKAILSARLSKSRRLENLIAQLPGEKKNGLFGNLIALSLGL
TPNFKSNFDLAEDAKLQLSKDTYDDDLDNLLAQIGDQYADLFLAAKNLSDAILLS
DILRVNTEITKAPLSASMIKRYDEHHQDLTLLKALVRQQLPEKYKEIFFDQSKNG
YAGYIDGGASQEEFYKFIKPILEKMDGTEELLVKLNREDLLRKQRTFDNGSIPHQI
HLGELHAILRRQEDFYPFLKDNREKIEKILTFRIPYYVGPLARGNSRFAWMTRKS
EETITPWNFEEVVDKGASAQSFIERMTNFDKNLPNEKVLPKHSLLYEYFTVYNEL
TKVKYVTEGMRKPAFLSGEQKKAIVDLLFKTNRKVTVKQLKEDYFKKIECFDSV
EISGVEDRFNASLGTYHDLLKIIKDKDFLDNEENEDILEDIVLTLTLFEDREMIEER
LKTYAHLFDDKVMKQLKRRRYTGWGRLSRKLINGIRDKQSGKTILDFLKSDGFA
NRNFMQLIHDDSLTFKEDIQKAQVSGQGDSLHEHIANLAGSPAIKKGILQTVKVV
DELVKVMGRHKPENIVIEMARENQTTQKGQKNSRERMKRIEEGIKELGSQILKE
HPVENTQLQNEKLYLYYLQNGRDMYVDQELDINRLSDYDVDHIVPQSFLKDDSID
NKVLTRSDKNRGKSDNVPSEEVVKKMKNYWRQLLNAKLITQRKFDNLTKAERG
GLSELDKAGFIKRQLVETRQITKHVAQILDSRMNTKYDENDKLIREVKVITLKSK
LVSDFRKDFQFYKVREINNYHHAHDAYLNAVVGTALIKKYPKLESEFVYGDYKVY
DVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDW
DPKKYGGFDSPTVAYSVLVVAKVEKGKSKKLKSVKELLGITIMERSSFEKNPIDFL
EAKGYKEVKKDLIIKLPKYSLFELENGRKRMLASAGELQKGNELALPSKYVNFL
YLASHYEKLKGSPEDNEQKQLFVEQHKHYLDEIIEQISEFSKRVILADANLDKVLS
 
AYNKHRDKPIREQAENIIHLFTLTNLGAPAAFKYFDTTIDRKRYTSTKEVLDATLI
HQSITGLYETRIDLSQLGGDSGGSKRTADGSEFEPKKKRKV* (SEQ ID NO: 194),
 wherein, the bold sequence indicates a sequence derived from nCas9, the italic sequence indicates a linker sequence, the double underlined sequence indicates a nuclear localization sequence, the single underlined sequence is a sequence of a deaminase 004V7, and the asterisk indicates a stop codon position.

An amino acid sequence of 004V8-nCas9 is as follows: 
MKRTADGSEFESPKKKRKVMYNAPRFFCRSAAVSDHEFNDEYWMRHALTLAKR
AREEGEVPVGAVLVLNNQVIGEGWNRAIGLHDPTAHAEIMALRQGGLVLQNYRLLGT
DLYVTFEPCVMCAGAMVHSRISRLVFGVRNSKRGAAGSLINVLNYPGMNHRVEITEGI
LAESCSAMLCDFYRWPREVFNALKKARQEEG
 
SGGSSGGSSGSETPGTSESATPESSGGSSGGSDKKYSIGLAIGTNSVGWAVITDEYK
VPSKKFKVLGNTDRHSIKKNLIGALLFDSGETAEATRLKRTARRRYTRRKNRICY
LQEIFSNEMAKVDDSFFHRLEESFLVEEDKKHERHPIFGNIVDEVAYHEKYPTIYH
LRKKLVDSTDKADLRLIYLALAHMIKFRGHFLIEGDLNPDNSDVDKLFIQLVQTY
NQLFEENPINASGVDAKAILSARLSKSRRLENLIAQLPGEKKNGLFGNLIALSLGL
TPNFKSNFDLAEDAKLQLSKDTYDDDLDNLLAQIGDQYADLFLAAKNLSDAILLS
DILRVNTEITKAPLSASMIKRYDEHHQDLTLLKALVRQQLPEKYKEIFFDQSKNG
YAGYIDGGASQEEFYKFIKPILEKMDGTEELLVKLNREDLLRKQRTFDNGSIPHQI
HLGELHAILRRQEDFYPFLKDNREKIEKILTFRIPYYVGPLARGNSRFAWMTRKS
EETITPWNFEEVVDKGASAQSFIERMTNFDKNLPNEKVLPKHSLLYEYFTVYNEL
TKVKYVTEGMRKPAFLSGEQKKAIVDLLFKTNRKVTVKQLKEDYFKKIECFDSV
EISGVEDRFNASLGTYHDLLKIIKDKDFLDNEENEDILEDIVLTLTLFEDREMIEER
LKTYAHLFDDKVMKQLKRRRYTGWGRLSRKLINGIRDKQSGKTILDFLKSDGFA
 
NRNFMQLIHDDSLTFKEDIQKAQVSGQGDSLHEHIANLAGSPAIKKGILQTVKVV
DELVKVMGRHKPENIVIEMARENQTTQKGQKNSRERMKRIEEGIKELGSQILKE
HPVENTQLQNEKLYLYYLQNGRDMYVDQELDINRLSDYDVDHIVPQSFLKDDSID
NKVLTRSDKNRGKSDNVPSEEVVKKMKNYWRQLLNAKLITQRKFDNLTKAERG
GLSELDKAGFIKRQLVETRQITKHVAQILDSRMNTKYDENDKLIREVKVITLKSK
LVSDFRKDFQFYKVREINNYHHAHDAYLNAVVGTALIKKYPKLESEFVYGDYKVY
DVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDW
DPKKYGGFDSPTVAYSVLVVAKVEKGKSKKLKSVKELLGITIMERSSFEKNPIDFL
EAKGYKEVKKDLIIKLPKYSLFELENGRKRMLASAGELQKGNELALPSKYVNFL
YLASHYEKLKGSPEDNEQKQLFVEQHKHYLDEIIEQISEFSKRVILADANLDKVLS
AYNKHRDKPIREQAENIIHLFTLTNLGAPAAFKYFDTTIDRKRYTSTKEVLDATLI
HQSITGLYETRIDLSQLGGDSGGSKRTADGSEFEPKKKRKV* (SEQ ID NO: 195),
 wherein, the bold sequence indicates a sequence derived from nCas9, the italic sequence indicates a linker sequence, the double underlined sequence indicates a nuclear localization sequence, the single underlined sequence is a sequence of a deaminase 004V8, and the asterisk indicates a stop codon position.

An amino acid sequence of 004V10-nCas9 is as follows: 
MKRTADGSEFESPKKKRKVMYNAPRFFCRSAAVSDHEFNDEYWMRHALTLAKR
AREEGEVPVGAVLVLNNQVIGEGWNRAIGLHDPTAHAEIMALRQGGLVLQNYRLIDA
TLYVTFEPCVMCAGAMVHSRLKKLVFGVRNSKRGAAGSLINVLNYPGMNHRVEITEG
ILAESCSAMLCDFYRWPREVFNALKKARQEEG
 
SGGSSGGSSGSETPGTSESATPESSGGSSGGSDKKYSIGLAIGTNSVGWAVITDEYK
VPSKKFKVLGNTDRHSIKKNLIGALLFDSGETAEATRLKRTARRRYTRRKNRICY
 
LQEIFSNEMAKVDDSFFHRLEESFLVEEDKKHERHPIFGNIVDEVAYHEKYPTIYH
LRKKLVDSTDKADLRLIYLALAHMIKFRGHFLIEGDLNPDNSDVDKLFIQLVQTY
NQLFEENPINASGVDAKAILSARLSKSRRLENLIAQLPGEKKNGLFGNLIALSLGL
TPNFKSNFDLAEDAKLQLSKDTYDDDLDNLLAQIGDQYADLFLAAKNLSDAILLS
DILRVNTEITKAPLSASMIKRYDEHHQDLTLLKALVRQQLPEKYKEIFFDQSKNG
YAGYIDGGASQEEFYKFIKPILEKMDGTEELLVKLNREDLLRKQRTFDNGSIPHQI
HLGELHAILRRQEDFYPFLKDNREKIEKILTFRIPYYVGPLARGNSRFAWMTRKS
EETITPWNFEEVVDKGASAQSFIERMTNFDKNLPNEKVLPKHSLLYEYFTVYNEL
TKVKYVTEGMRKPAFLSGEQKKAIVDLLFKTNRKVTVKQLKEDYFKKIECFDSV
EISGVEDRFNASLGTYHDLLKIIKDKDFLDNEENEDILEDIVLTLTLFEDREMIEER
LKTYAHLFDDKVMKQLKRRRYTGWGRLSRKLINGIRDKQSGKTILDFLKSDGFA
NRNFMQLIHDDSLTFKEDIQKAQVSGQGDSLHEHIANLAGSPAIKKGILQTVKVV
DELVKVMGRHKPENIVIEMARENQTTQKGQKNSRERMKRIEEGIKELGSQILKE
HPVENTQLQNEKLYLYYLQNGRDMYVDQELDINRLSDYDVDHIVPQSFLKDDSID
NKVLTRSDKNRGKSDNVPSEEVVKKMKNYWRQLLNAKLITQRKFDNLTKAERG
GLSELDKAGFIKRQLVETRQITKHVAQILDSRMNTKYDENDKLIREVKVITLKSK
LVSDFRKDFQFYKVREINNYHHAHDAYLNAVVGTALIKKYPKLESEFVYGDYKVY
DVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDW
DPKKYGGFDSPTVAYSVLVVAKVEKGKSKKLKSVKELLGITIMERSSFEKNPIDFL
EAKGYKEVKKDLIIKLPKYSLFELENGRKRMLASAGELQKGNELALPSKYVNFL
YLASHYEKLKGSPEDNEQKQLFVEQHKHYLDEIIEQISEFSKRVILADANLDKVLS
AYNKHRDKPIREQAENIIHLFTLTNLGAPAAFKYFDTTIDRKRYTSTKEVLDATLI
HQSITGLYETRIDLSQLGGDSGGSKRTADGSEFEPKKKRKV* (SEQ ID NO: 196),
 wherein, the bold sequence indicates a sequence derived from nCas9, the italic sequence indicates a linker sequence, the double underlined sequence indicates a nuclear localization sequence, the single underlined sequence is a sequence of a deaminase 004V10, and the asterisk indicates a stop codon position.

An amino acid sequence of 004V12-nCas9 is as follows: 
MKRTADGSEFESPKKKRKVMYNAPRFFCRSAAVSDHEFNDEYWMRHALTLAKR
AREEGEVPVGAVLVLNNQVIGEGWNRAIGLHDPTAHAEIMALRQGGLVLQNYRLIDA
TLYVTFEPCVMCAGAMVHSRISRLVFGVRNSKRGAAGSLIKIFHYPGMNHRVEITEGIL
AESCSAMLCDFYRWPREVFNALKKARQEEG
 
SGGSSGGSSGSETPGTSESATPESSGGSSGGSDKKYSIGLAIGTNSVGWAVITDEYK
VPSKKFKVLGNTDRHSIKKNLIGALLFDSGETAEATRLKRTARRRYTRRKNRICY
LQEIFSNEMAKVDDSFFHRLEESFLVEEDKKHERHPIFGNIVDEVAYHEKYPTIYH
LRKKLVDSTDKADLRLIYLALAHMIKFRGHFLIEGDLNPDNSDVDKLFIQLVQTY
NQLFEENPINASGVDAKAILSARLSKSRRLENLIAQLPGEKKNGLFGNLIALSLGL
TPNFKSNFDLAEDAKLQLSKDTYDDDLDNLLAQIGDQYADLFLAAKNLSDAILLS
DILRVNTEITKAPLSASMIKRYDEHHQDLTLLKALVRQQLPEKYKEIFFDQSKNG
YAGYIDGGASQEEFYKFIKPILEKMDGTEELLVKLNREDLLRKQRTFDNGSIPHQI
HLGELHAILRRQEDFYPFLKDNREKIEKILTFRIPYYVGPLARGNSRFAWMTRKS
EETITPWNFEEVVDKGASAQSFIERMTNFDKNLPNEKVLPKHSLLYEYFTVYNEL
TKVKYVTEGMRKPAFLSGEQKKAIVDLLFKTNRKVTVKQLKEDYFKKIECFDSV
EISGVEDRFNASLGTYHDLLKIIKDKDFLDNEENEDILEDIVLTLTLFEDREMIEER
LKTYAHLFDDKVMKQLKRRRYTGWGRLSRKLINGIRDKQSGKTILDFLKSDGFA
NRNFMQLIHDDSLTFKEDIQKAQVSGQGDSLHEHIANLAGSPAIKKGILQTVKVV
DELVKVMGRHKPENIVIEMARENQTTQKGQKNSRERMKRIEEGIKELGSQILKE
 
HPVENTQLQNEKLYLYYLQNGRDMYVDQELDINRLSDYDVDHIVPQSFLKDDSID
NKVLTRSDKNRGKSDNVPSEEVVKKMKNYWRQLLNAKLITQRKFDNLTKAERG
GLSELDKAGFIKRQLVETRQITKHVAQILDSRMNTKYDENDKLIREVKVITLKSK
LVSDFRKDFQFYKVREINNYHHAHDAYLNAVVGTALIKKYPKLESEFVYGDYKVY
DVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDW
DPKKYGGFDSPTVAYSVLVVAKVEKGKSKKLKSVKELLGITIMERSSFEKNPIDFL
EAKGYKEVKKDLIIKLPKYSLFELENGRKRMLASAGELQKGNELALPSKYVNFL
YLASHYEKLKGSPEDNEQKQLFVEQHKHYLDEIIEQISEFSKRVILADANLDKVLS
AYNKHRDKPIREQAENIIHLFTLTNLGAPAAFKYFDTTIDRKRYTSTKEVLDATLI
HQSITGLYETRIDLSQLGGDSGGSKRTADGSEFEPKKKRKV* (SEQ ID NO: 197),
 wherein, the bold sequence indicates a sequence derived from nCas9, the italic sequence indicates a linker sequence, the double underlined sequence indicates a nuclear localization sequence, the single underlined sequence is a sequence of a deaminase 004V12, and the asterisk indicates a stop codon position.

An amino acid sequence of 004V13-nCas9 is as follows: 
MKRTADGSEFESPKKKRKVMYNAPRFFCRSAAVSDHEFNDEYWMRHALTLAKR
AREEGEVPVGAVLVLNNQVIGEGWNRAIGLHDPTAHAEIMALRQGGLVLQNYRLIDA
TLYVTFEPCVMCAGAMVHSRISRLVFGVRNSKRGAAGSLINVLNYFALNHRVEITEGIL
AESCSAMLCDFYRWPREVFNALKKARQEEG
 
SGGSSGGSSGSETPGTSESATPESSGGSSGGSDKKYSIGLAIGTNSVGWAVITDEYK
VPSKKFKVLGNTDRHSIKKNLIGALLFDSGETAEATRLKRTARRRYTRRKNRICY
LQEIFSNEMAKVDDSFFHRLEESFLVEEDKKHERHPIFGNIVDEVAYHEKYPTIYH
LRKKLVDSTDKADLRLIYLALAHMIKFRGHFLIEGDLNPDNSDVDKLFIQLVQTY
 
NQLFEENPINASGVDAKAILSARLSKSRRLENLIAQLPGEKKNGLFGNLIALSLGL
TPNFKSNFDLAEDAKLQLSKDTYDDDLDNLLAQIGDQYADLFLAAKNLSDAILLS
DILRVNTEITKAPLSASMIKRYDEHHQDLTLLKALVRQQLPEKYKEIFFDQSKNG
YAGYIDGGASQEEFYKFIKPILEKMDGTEELLVKLNREDLLRKQRTFDNGSIPHQI
HLGELHAILRRQEDFYPFLKDNREKIEKILTFRIPYYVGPLARGNSRFAWMTRKS
EETITPWNFEEVVDKGASAQSFIERMTNFDKNLPNEKVLPKHSLLYEYFTVYNEL
TKVKYVTEGMRKPAFLSGEQKKAIVDLLFKTNRKVTVKQLKEDYFKKIECFDSV
EISGVEDRFNASLGTYHDLLKIIKDKDFLDNEENEDILEDIVLTLTLFEDREMIEER
LKTYAHLFDDKVMKQLKRRRYTGWGRLSRKLINGIRDKQSGKTILDFLKSDGFA
NRNFMQLIHDDSLTFKEDIQKAQVSGQGDSLHEHIANLAGSPAIKKGILQTVKVV
DELVKVMGRHKPENIVIEMARENQTTQKGQKNSRERMKRIEEGIKELGSQILKE
HPVENTQLQNEKLYLYYLQNGRDMYVDQELDINRLSDYDVDHIVPQSFLKDDSID
NKVLTRSDKNRGKSDNVPSEEVVKKMKNYWRQLLNAKLITQRKFDNLTKAERG
GLSELDKAGFIKRQLVETRQITKHVAQILDSRMNTKYDENDKLIREVKVITLKSK
LVSDFRKDFQFYKVREINNYHHAHDAYLNAVVGTALIKKYPKLESEFVYGDYKVY
DVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDW
DPKKYGGFDSPTVAYSVLVVAKVEKGKSKKLKSVKELLGITIMERSSFEKNPIDFL
EAKGYKEVKKDLIIKLPKYSLFELENGRKRMLASAGELQKGNELALPSKYVNFL
YLASHYEKLKGSPEDNEQKQLFVEQHKHYLDEIIEQISEFSKRVILADANLDKVLS
AYNKHRDKPIREQAENIIHLFTLTNLGAPAAFKYFDTTIDRKRYTSTKEVLDATLI
HQSITGLYETRIDLSQLGGDSGGSKRTADGSEFEPKKKRKV* (SEQ ID NO: 198),
 wherein, the bold sequence indicates a sequence derived from nCas9, the italic sequence indicates a linker sequence, the double underlined sequence indicates a nuclear localization sequence, the single underlined sequence is a sequence of an adenosine deaminase 004V13, and the asterisk at a C-terminal indicates a stop codon position.

Further, the present invention also provides mutants obtained after amino acid substitution at one or more sites based on the adenosine deaminase 005V1, including a plurality of mutants including deaminases 005V1-10-3, 005V1-11-5, 005V1-10-1, 005V1-11-2, 005V1-15-1, 005V1-11-3, 005V1-10-4, 005V1-11-4, 005V1-15-2, 005V1-2-7, 005V1-1-1, 005V1-1-2, 005V1-1-3, 005V1-1-4, 005V1-1-5, 005V1-1-6, 005V1-1-7, 005V1-1-8, 005V1-2-1, 005V1-2-2, 005V1-2-3, 005V1-2-5, 005V1-2-6, 005V1-2-8, 005V1-3-1, 005V1-3-2, 005V1-3-3, 005V1-3-4, 005V1-3-5, 005V1-3-7, 005V1-3-8, 005V1-4-1, 005V1-4-2, 005V1-4-3, 005V1-15-5, 005V1-5-4, 005V1-5-8, 005V1-10-5, 005V1-5-2, 005V1-3-6, 005V1-4-5, 005V1-4-7, 005V1-4-8, 005V1-5-1, 005V1-5-3, 005V1-5-5, 005V1-5-6, 005V1-5-7, 005V1-6-1, 005V1-6-2, 005V1-6-5, 005V1-6-6, 005V1-6-8, 005V1-7-1, 005V1-7-2, 005V1-7-3, 005V1-8-1, 005V1-8-2, 005V1-8-3, 005V1-8-4, 005V1-8-5, 005V1-9-1, 005V1-9-2, 005V1-9-3, 005V1-9-4, 005V1-9-5, and 005V1-10-2.

By searching for a suitable insertion site of the adenosine deaminase in the middle of the nCas9 protein, a plurality of chimeric base editor fusion proteins with improved editing efficiency are also obtained. The adenosine deaminase used includes adenosine deaminases 005V1, 005V1-10-1, and 005V1-10-3. In the base editor fusion protein containing the sequences of the above three adenosine deaminases, the nCas9 domain is selected as the nucleic acid programmable nucleotide binding domain, and an insertion position of the adenosine deaminase is between amino acid positions 583 and 584, 768 and 769, 770 and 771, 776 and 777, 793 and 794, 905 and 906, 919 and 920, 1048 to 1063, 1049 to 1062, 1249 and 1250, 1263 and 1264, or 1276 and 1277 of SEQ ID NO: 61.

In the present invention, the expression "retaining the polynucleotide binding and base editing activity of the amino acid sequence set forth in SEQ ID NO: 190", "retaining the polynucleotide binding and base editing activity of the amino acid sequence set forth in SEQ ID NO: 3" can mean completely retaining the polynucleotide binding and base editing activity of the base editor of the amino acid sequence set forth in SEQ ID NO: 190 or SEQ ID NO: 3, or partially retaining the activity thereof. In other embodiments, a base editor having a modified sequence may have a polynucleotide binding and base editing activity higher than that of the base editor of the amino acid sequence set forth in SEQ ID NO: 190 or SEQ ID NO: 3.

It should be understood that the base editor fusion protein of this disclosure can include one or more additional characteristics. For example, in some embodiments, the fusion protein can include an inhibitor, a cytoplasmic localization sequence, and an export sequence, such as a nuclear export sequence or other localization sequences, as well as a tag that can be used for solubilization, purification, or detection of the fusion. Suitable tags provided herein include, but are not limited to, a biotin carboxyl carrier protein (BCCP) tag, a myc tag, a calmodulin tag, a FLAG tag, a hemagglutinin (HA) tag, a polyhistidine tag also known as a histidine tag or a Histag, a maltose binding protein (MBP)-tag, a nus-tag, a glutathione-S-transferase (GST)-tag, a green fluorescent protein (GFP)-tag, a thioredoxin-tag, an S-tag, Softags (for example, Softag 1 and Softag 3), a strand tag, a biotin ligase tag, a Flash tag, a V5 tag, and a SBP tag. Other suitable sequences will be apparent to those skilled in the art. In some embodiments, the fusion protein includes one or more His tags.

### <Polynucleotide>

A third aspect of the present invention provides a polynucleotide encoding the adenosine deaminase according to the first aspect of the present invention or encoding the base editor fusion protein according to the second aspect of the present invention.

### <Expression Vector>

A fourth aspect of the present invention provides a vector including the polynucleotide according to the third aspect of the present invention. In some embodiments of the present invention, the vector is a mammalian expression vector. In some embodiments, the expression vector is selected from one or more of adeno-associated virus, retroviral vector, adenoviral vector, lentiviral vector, Sendai virus vector, and herpesvirus vector. In some embodiments, the vector includes a promoter.

### <Cell>

A fifth aspect of the present invention provides a cell including one or more of the adenosine deaminase according to the first aspect of the present invention, the base editor fusion protein according to the second aspect of the present invention, the polynucleotide according to the third aspect of the present invention, and the vector according to the fourth aspect of the present invention. In some embodiments of the present invention, the cell is a prokaryotic cell or a eukaryotic cell, and further, may be a bacterial cell, a plant cell, an insect cell, a human cell, or a mammalian cell.

### <Base Editor System>

A sixth aspect of the present invention provides a base editor system. In some embodiments, the base editor system includes the adenosine deaminase according to the first aspect of the present invention, a nucleic acid programmable nucleotide binding domain, and a guide polynucleotide.

In other embodiments, the base editor system includes the base editor fusion protein according to the second aspect of the present invention, and a guide polynucleotide.

In some embodiments of the present invention, the guide polynucleotide is a guide RNA (gRNA), which is a short synthetic RNA constituted by a backbone sequence required for Cas protein binding and a user-defined spacer sequence of about 20 nucleotides, and the spacer sequence defines a genomic target to be modified. Therefore, those skilled in the art can change a specific portion of the genome target of the Cas protein depending on specificity of a gRNA targeting sequence for the genomic target compared to a rest of a genome.

In some more specific embodiments of the present invention, the guide polynucleotide is a sgRNA, which is constituted by a backbone sequence required for Cas protein binding and a user-defined spacer sequence of about 20 nucleotides.

Different backbone sequences can be selected for Cas proteins of different sources or types. In some more specific embodiments of the present invention, a domain (SpCas9) that binds to the Cas9 protein, that is, the backbone sequence of the sgRNA is:

GTTTTAGAGCTAGAAATAGCAAGTTAAAATAAGGCTAGTCCGTTATCAACTTGA
AAAAGTGGCACCGAGTCGGTGC (SEQ ID NO: 199).

### <Pharmaceutical Composition, Kit, Delivery System, Use, and Method>

A seventh aspect of the present invention provides a pharmaceutical composition including: one or more of the adenosine deaminase according to the first aspect of the present invention, the base editor fusion protein according to the second aspect of the present invention, the polynucleotide according to the third aspect of the present invention, the expression vector according to the fourth aspect of the present invention, the cell according to the fifth aspect of the present invention, and the base editor system according to the sixth aspect of the present invention; and a pharmaceutically acceptable carrier.

**In** some embodiments, the pharmaceutically acceptable carrier may be a delivery vector, such as a lipid, a cationic lipid, or other polymers having a drug delivery function.

An eighth aspect of the present invention provides a kit, specifically a disease treatment kit including one or more of the adenosine deaminase according to the first aspect of the present invention, the base editor fusion protein according to the second aspect of the present invention, the polynucleotide according to the third aspect of the present invention, the expression vector according to the fourth aspect of the present invention, the cell according to the fifth aspect of the present invention, the base editor system according to the sixth aspect of the present invention, and the pharmaceutical composition according to the seventh aspect of the present invention.

A ninth aspect of the present invention provides a delivery system including: one or more of the adenosine deaminase according to the first aspect of the present invention, the base editor fusion protein according to the second aspect of the present invention, the polynucleotide according to the third aspect of the present invention, the vector according to the fourth aspect of the present invention, the cell according to the fifth aspect of the present invention, and the base editor system according to the sixth aspect of the present invention; and a delivery medium.

In some embodiments, the delivery medium may be nanoparticles, liposomes, exosomes, microvesicles or gene guns, cell-penetrating peptides, and the like.

A tenth aspect of the present invention provides a use of the adenosine deaminase according to the first aspect of the present invention in preparing a base editor or a base editor system.

An eleventh aspect of the present invention provides a use of the adenosine deaminase according to the first aspect of the present invention, the base editor fusion protein according to the second aspect of the present invention, the polynucleotide according to the third aspect of the present invention, the expression vector according to the fourth aspect of the present invention, the cell according to the fifth aspect of the present invention, the base editor system according to the sixth aspect of the present invention or the pharmaceutical composition according to the seventh aspect of the present invention, and the delivery system according to the ninth aspect of the present invention in preparing a drug for treating a disease associated with or caused by a point mutation.

In some embodiments, the drug can correct the point mutation. In some embodiments, the point mutation is G to A and/or C to T.

In some embodiments, the disease associated with or caused by the point mutation includes hypercholesterolemia, transthyretin amyloidosis, and β-hemoglobinopathy. In other embodiments, non-limiting examples of the disease include Meier-Gorlin syndrome; Seckel syndrome; Joubert syndrome; Leber congenital amaurosis; Charcot-Marie-Tooth disease, Type 2; Usher syndrome, Type 2C; spinocerebellar ataxia; long QT syndrome 2; Sjogren-Larsson syndrome; hereditary diabetes mellitus; neuroblastoma; Kallmann syndrome 1; Kallmann syndrome; metachromatic leukodystrophy; Rett syndrome; amyotrophic lateral sclerosis Type 10; and Li-Fraumeni syndrome.

A twelfth aspect of the present invention provides a base editing method of a nucleic acid, the method including a step of contacting a nucleic acid with the base editor system according to the sixth aspect of the present invention.

In some embodiments, the nucleic acid is DNA. Further, the nucleic acid is double-stranded DNA.

In some embodiments, the nucleic acid includes a target sequence associated with a disease.

In some embodiments, the target sequence includes a point mutation associated with a disease.

In some specific embodiments, the target sequence includes a point mutation associated with a disease or disorder from G to A or C to T, and deamination of a mutant A base results in a sequence that is not associated with the disease or disorder.

In some embodiments, the target sequence encodes a protein, and the point mutation is in a codon and results in a change in an amino acid encoded by the mutant codon compared to a wild-type codon.

In some embodiments, the target sequence is located at a splice site, and the point mutation results in a splicing change of an mRNA transcript compared to a wild-type transcript.

In some embodiments, the target sequence is located at a promoter of a gene, and the point mutation results in an increase in gene expression.

In some embodiments, the target sequence is located at a promoter of a gene, and the point mutation results in a decrease in gene expression.

In some embodiments, the nucleic acid is located in a genome of an organism.

In some embodiments, the organism is a prokaryotic organism or a eukaryotic organism, or a vertebrate or a mammal.

In some embodiments, the deamination of the mutant A base results in a change in the amino acid encoded by the mutant codon, or a codon encoding a wild-type amino acid, or a change in the mRNA transcript, or a wild-type mRNA transcript, or an increase in gene expression, or a decrease in gene expression.

In some embodiments, the contacting is performed in vitro.

In some embodiments, the contacting is performed in vivo in a subject.

In some embodiments, the subject has been diagnosed with a disease or disorder.

In some embodiments, the disease or disorder is associated with a point mutation in a proprotein convertase subtilisin/kexin type 9 (PCSK9) gene.

In some embodiments, the disease includes hypercholesterolemia, transthyretin amyloidosis, and β-hemoglobinopathy. In other embodiments, non-limiting examples of the disease include Meier-Gorlin syndrome; Seckel syndrome; Joubert syndrome; Leber congenital amaurosis; Charcot-Marie-Tooth disease, Type 2; Usher syndrome, Type 2C; spinocerebellar ataxia; long QT syndrome; Sjogren-Larsson syndrome; hereditary diabetes mellitus; neuroblastoma; Kallmann syndrome; metachromatic leukodystrophy; Rett syndrome; amyotrophic lateral sclerosis Type 10; and Li-Fraumeni syndrome.

A thirteenth aspect of the present invention provides a method for treating a disease associated with or caused by a point mutation. In some embodiments, the provided method includes administering to a subject afflicted with such a disease an effective amount of the base editor fusion protein according to the second aspect of the present invention, the base editor system according to the sixth aspect of the present invention, the pharmaceutical composition according to the seventh aspect of the present invention, the kit according to the eighth aspect of the present invention, or the delivery system according to the ninth aspect of the present invention that corrects a point mutation or introduces an inactive mutation into a disease-related gene.

### <Control>

In the following Examples, an efficient base editor ABE8e (Richter MF, Zhao KT, Eton E, Lapinaite A, Newby GA, Thuronyi BW, Wilson C, Koblan LW, Zeng J, Bauer DE, Doudna JA, Liu DR. Phage-assisted evolution of an adenine base editor with improved Cas domain compatibility and activity. Nat Biotechnol. 2020 Jul;38(7):883-891. doi: 10.1038/s41587-020-0453-z. Epub 2020 Mar 16. Erratum in: Nat Biotechnol. 2020 May 20;: PMID: 32433547; PMCID: PMC7357821.) evolved by the David R. Liu team is used as a comparison. Compared with the base editor including the adenosine deaminase provided by the present invention, the ABE8e is obtained based on the optimization of a deaminase component of ABE7.10 (Gaudelli NM, Komor AC, Rees HA, Packer MS, Badran AH, Bryson DI, Liu DR. Programmable base editing of A•T to G•C in genomic DNA without DNA cleavage. Nature. 2017 Nov 23;551(7681):464-471. doi: 10.1038/nature24644. Epub 2017 Oct 25. Erratum in: Nature. 2018 May 2;: PMID: 29160308; PMCID: PMC5726555.). According to experimental results obtained by the above team, the activity (expressed as first-order kinetics. deamination rate constants, (kapp)) is increased by 590 times relative to the ABE7.10.

Specifically, in the following Examples, an amino acid sequence of the ABE8e is as follows:
MKRTADGSEFESPKKKRKVSEVEFSHEYWMRHALTLAKRARDEREVPVGAVLVL NNRVIGEGWNRAIGLHDPTAHAEIMALRQGGLVMQNYRLIDATLYVTFEPCVMCAGA MIHSRIGRVVFGVRNSKRGAAGSLMNVLNYPGMNHRVEITEGILADECAALLCDFYR MPRQVFNAQKKAQSSIN*SGGSSGGSSGSETPGTSESATPESSGGSSGGS***DKKYSIGLAIG TNSVGWAVITDEYKVPSKKFKVLGNTDRHSIKKNLIGALLFDSGETAEATRLKRT ARRRYTRRKNRICYLQEIFSNEMAKVDDSFFHRLEESFLVEEDKKHERHPIFGNIV DEVAYHEKYPTIYHLRKKLVDSTDKADLRLIYLALAHMIKFRGHFLIEGDLNPDN SDVDKLFIQLVQTYNQLFEENPINASGVDAKAILSARLSKSRRLENLIAQLPGEKK NGLFGNLIALSLGLTPNFKSNFDLAEDAKLQLSKDTYDDDLDNLLAQIGDQYADL FLAAKNLSDAILLSDILRVNTEITKAPLSASMIKRYDEHHQDLTLLKALVRQQLPE KYKEIFFDQSKNGYAGYIDGGASQEEFYKFIKPILEKMDGTEELLVKLNREDLLR KQRTFDNGSIPHQIHLGELHAILRRQEDFYPFLKDNREKIEKILTFRIPYYVGPLA RGNSRFAWMTRKSEETITPWNFEEVVDKGASAQSFIERMTNFDKNLPNEKVLPK HSLLYEYFTVYNELTKVKYVTEGMRKPAFLSGEQKKAIVDLLFKTNRKVTVKQL KEDYFKKIECFDSVEISGVEDRFNASLGTYHDLLKIIKDKDFLDNEENEDILEDIVL TLTLFEDREMIEERLKTYAHLFDDKVMKQLKRRRYTGWGRLSRKLINGIRDKQS GKTILDFLKSDGFANRNFMQLIHDDSLTFKEDIQKAQVSGQGDSLHEHIANLAGS PAIKKGILQTVKVVDELVKVMGRHKPENIVIEMARENQTTQKGQKNSRERMKRI EEGIKELGSQILKEHPVENTQLQNEKLYLYYLQNGRDMYVDQELDINRLSDYDV DHIVPQSFLKDDSIDNKVLTRSDKNRGKSDNVPSEEVVKKMKNYWRQLLNAKLI TQRKFDNLTKAERGGLSELDKAGFIKRQLVETRQITKΣIVAQILDSRMNTKYDEN DKLIREVKVITLKSKLVSDFRKDFQFYKVREINNYHHAHDAYLNAVVGTALIKKY PKLESEFVYGDYKVYDVRKMIAKSEQEIGKATAKYFFYSNIMNFFKTEITLANGEI RKRPLIETNGETGEIVWDKGRDFATVRKVLSMPQVNIVKKTEVQTGGFSKESILP KRNSDKLIARKKDWDPKKYGGFDSPTVAYSVLVVAKVEKGKSKKLKSVKELLGI TIMERSSFEKNPIDFLEAKGYKEVKKDLIIKLPKYSLFELENGRKRMLASAGELQ KGNELALPSKYVNFLYLASHYEKLKGSPEDNEQKQLFVEQHKHYLDEIIEQISEFS KRVILADANLDKVLSAYNKHRDKPIREQAENIIHLFTLTNLGAPAAFKYFDTTIDR KRYTSTKEVLDATLIHQSITGLYETRIDLSQLGGD**SGGSKRTADGSEFEPKKKRKV* (SEQ ID NO: 200), wherein, the bold sequence indicates a sequence derived from nCas9, the italic sequence indicates a linker sequence, the double underlined sequence indicates a nuclear localization sequence, the single underlined sequence is a sequence of ecTadA* deaminase, and * at an end of a C-terminal indicates a stop codon position.

Correspondingly, a nucleotide sequence of the ABE8e is as follows:

atgaaacggacagccgacggaagcgagttcgagtcaccaaagaagaagcggaaagtctctgaggtggagttttcccacgagt
actggatgagacatgccctgaccctggccaagagggcacgggatgagagggaggtgcctgtgggagccgtgctggtgctgaacaat
agagtgatcggcgagggctggaacagagccatcggcctgcacgacccaacagcccatgccgaaattatggccctgagacagggcg
gcctggtcatgcagaactacagactgattgacgccaccctgtacgtgacattcgagccttgcgtgatgtgcgccggcgccatgatccact
ctaggatcggccgcgtggtgtttggcgtgaggaactcaaaaagaggcgccgcaggctccctgatgaacgtgctgaactaccccggcat
gaatcaccgcgtcgaaattaccgagggaatcctggcagatgaatgtgccgccctgctgtgcgatttctatcggatgcctagacaggtgtt
caatgctcagaagaaggcccagagctccatcaactccggaggatctagcggaggctcctctggctctgagacacctggcacaagcga

gagcgcaacacctgaaagcagcgggggcagcagcggggggtcagacaagaagtacagcatcggcctggccatcggcaccaactct
gtgggctgggccgtgatcaccgacgagtacaaggtgcccagcaagaaattcaaggtgctgggcaacaccgaccggcacagcatcaa
gaagaacctgatcggagccctgctgttcgacagcggcgaaacagccgaggccacccggctgaagagaaccgccagaagaagatac
accagacggaagaaccggatctgctatctgcaagagatcttcagcaacgagatggccaaggtggacgacagcttcttccacagactgg
aagagtccttcctggtggaagaggataagaagcacgagcggcaccccatcttcggcaacatcgtggacgaggtggcctaccacgaga
agtaccccaccatctaccacctgagaaagaaactggtggacagcaccgacaaggccgacctgcggctgatctatctggccctggccc
acatgatcaagttccggggccacttcctgatcgagggcgacctgaaccccgacaacagcgacgtggacaagctgttcatccagctggt
gcagacctacaaccagctgttcgaggaaaaccccatcaacgccagcggcgtggacgccaaggccatcctgtctgccagactgagcaa
gagcagacggctggaaaatctgatcgcccagctgcccggcgagaagaagaatggcctgttcggaaacctgattgccctgagcctggg
cctgacccccaacttcaagagcaacttcgacctggccgaggatgccaaactgcagctgagcaaggacacctacgacgacgacctgga
caacctgctggcccagatcggcgaccagtacgccgacctgtttctggccgccaagaacctgtccgacgccatcctgctgagcgacatc
ctgagagtgaacaccgagatcaccaaggcccccctgagcgcctctatgatcaagagatacgacgagcaccaccaggacctgaccctg
ctgaaagctctcgtgcggcagcagctgcctgagaagtacaaagagattttcttcgaccagagcaagaacggctacgccggctacattga
cggcggagccagccaggaagagttctacaagttcatcaagcccatcctggaaaagatggacggcaccgaggaactgctcgtgaagct
gaacagagaggacctgctgcggaagcagcggaccttcgacaacggcagcatcccccaccagatccacctgggagagctgcacgcc
attctgcggcggcaggaagatttttacccattcctgaaggacaaccgggaaaagatcgagaagatcctgaccttccgcatcccctactac
gtgggccctctggccaggggaaacagcagattcgcctggatgaccagaaagagcgaggaaaccatcaccccctggaacttcgagga
agtggtggacaagggcgcttccgcccagagcttcatcgagcggatgaccaacttcgataagaacctgcccaacgagaaggtgctgcc
caagcacagcctgctgtacgagtacttcaccgtgtataacgagctgaccaaagtgaaatacgtgaccgagggaatgagaaagcccgcc
ttcctgagcggcgagcagaaaaaggccatcgtggacctgctgttcaagaccaaccggaaagtgaccgtgaagcagctgaaagagga
ctacttcaagaaaatcgagtgcttcgactccgtggaaatctccggcgtggaagatcggttcaacgcctccctgggcacataccacgatct
gctgaaaattatcaaggacaaggacttcctggacaatgaggaaaacgaggacattctggaagatatcgtgctgaccctgacactgtttga
ggacagagagatgatcgaggaacggctgaaaacctatgcccacctgttcgacgacaaagtgatgaagcagctgaagcggcggagat
acaccggctggggcaggctgagccggaagctgatcaacggcatccgggacaagcagtccggcaagacaatcctggatttcctgaagt

ccgacggcttcgccaacagaaacttcatgcagctgatccacgacgacagcctgacctttaaagaggacatccagaaagcccaggtgtc
cggccagggcgatagcctgcacgagcacattgccaatctggccggcagccccgccattaagaagggcatcctgcagacagtgaagg
tggtggacgagctcgtgaaagtgatgggccggcacaagcccgagaacatcgtgatcgaaatggccagagagaaccagaccaccca
gaagggacagaagaacagccgcgagagaatgaagcggatcgaagagggcatcaaagagctgggcagccagatcctgaaagaaca
ccccgtggaaaacacccagctgcagaacgagaagctgtacctgtactacctgcagaatgggcgggatatgtacgtggaccaggaact
ggacatcaaccggctgtccgactacgatgtggaccatatcgtgcctcagagctttctgaaggacgactccatcgacaacaaggtgctga
ccagaagcgacaagaaccggggcaagagcgacaacgtgccctccgaagaggtcgtgaagaagatgaagaactactggcggcagct
gctgaacgccaagctgattacccagagaaagttcgacaatctgaccaaggccgagagaggcggcctgagcgaactggataaggccg
gcttcatcaagagacagctggtggaaacccggcagatcacaaagcacgtggcacagatcctggactcccggatgaacactaagtacg
acgagaatgacaagctgatccgggaagtgaaagtgatcaccctgaagtccaagctggtgtccgatttccggaaggatttccagttttaca
aagtgcgcgagatcaacaactaccaccacgcccacgacgcctacctgaacgccgtcgtgggaaccgccctgatcaaaaagtacccta
agctggaaagcgagttcgtgtacggcgactacaaggtgtacgacgtgcggaagatgatcgccaagagcgagcaggaaatcggcaag
gctaccgccaagtacttcttctacagcaacatcatgaactttttcaagaccgagattaccctggccaacggcgagatccggaagcggcct
ctgatcgagacaaacggcgaaaccggggagatcgtgtgggataagggccgggattttgccaccgtgcggaaagtgctgagcatgcc
ccaagtgaatatcgtgaaaaagaccgaggtgcagacaggcggcttcagcaaagagtctatcctgcccaagaggaacagcgataagct
gatcgccagaaagaaggactgggaccctaagaagtacggcggcttcgacagccccaccgtggcctattctgtgctggtggtggccaa
agtggaaaagggcaagtccaagaaactgaagagtgtgaaagagctgctggggatcaccatcatggaaagaagcagcttcgagaaga
atcccatcgactttctggaagccaagggctacaaagaagtgaaaaaggacctgatcatcaagctgcctaagtactccctgttcgagctgg
aaaacggccggaagagaatgctggcctctgccggcgaactgcagaagggaaacgaactggccctgccctccaaatatgtgaacttcc
tgtacctggccagccactatgagaagctgaagggctcccccgaggataatgagcagaaacagctgtttgtggaacagcacaagcacta
cctggacgagatcatcgagcagatcagcgagttctccaagagagtgatcctggccgacgctaatctggacaaagtgctgtccgcctaca
acaagcaccgggataagcccatcagagagcaggccgagaatatcatccacctgtttaccctgaccaatctgggagcccctgccgcctt
caagtactttgacaccaccatcgaccggaagaggtacaccagcaccaaagaggtgctggacgccaccctgatccaccagagcatcac
cggcctgtacgagacacggatcgacctgtctcagctgggaggtgactctggcggctcaaaaagaaccgccgacggcagcgaattcga

gcccaagaagaagaggaaagtctaa (SEQ ID NO: 201).

In the following Examples, the ABE8e and the napDNAbp used in the base editor including the adenosine deaminase provided by the present invention are both nCas9, and an amino acid sequence of the nCas9 is as follows:

DKKYSIGLAIGTNSVGWAVITDEYKVPSKKFKVLGNTDRHSIKKNLIGALLFDSGE
TAEATRLKRTARRRYTRRKNRICYLQEIFSNEMAKVDDSFFHRLEESFLVEEDKKHERH
PIFGNIVDEVAYHEKYPTIYHLRKKLVDSTDKADLRLIYLALAHMIKFRGHFLIEGDLN
PDNSDVDKLFIQLVQTYNQLFEENPINASGVDAKAILSARLSKSRRLENLIAQLPGEKK
NGLFGNLIALSLGLTPNFKSNFDLAEDAKLQLSKDTYDDDLDNLLAQIGDQYADLFLA
AKNLSDAILLSDILRVNTEITKAPLSASMIKRYDEHHQDLTLLKALVRQQLPEKYKEIFF
DQSKNGYAGYIDGGASQEEFYKFIKPILEKMDGTEELLVKLNREDLLRKQRTFDNGSIP
HQIHLGELHAILRRQEDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKVK
YVTEGMRKPAFLSGEQKKAIVDLLFKTNRKVTVKQLKEDYFKKIECFDSVEISGVEDR
FNASLGTYHDLLKIIKDKDFLDNEENEDILEDIVLTLTLFEDREMIEERLKTYAHLFDDK
VMKQLKRRRYTGWGRLSRKLINGIRDKQSGKTILDFLKSDGFANRNFMQLIHDDSLTF
KEDIQKAQVSGQGDSLHEHIANLAGSPAIKKGILQTVKVVDELVKVMGRHKPENIVIE
MARENQTTQKGQKNSRERMKRIEEGIKELGSQILKEHPVENTQLQNEKLYLYYLQNG
RDMYVDQELDINRLSDYDVDHIVPQSFLKDDSIDNKVLTRSDKNRGKSDNVPSEEVV
KKMKNYWRQLLNAKLITQRKFDNLTKAERGGLSELDKAGFIKRQLVETRQITKHVAQ
ILDSRMNTKYDENDKLIREVKVITLKSKLVSDFRKDFQFYKVREINNYHHAHDAYLNA
VVGTALIKKYPKLESEFVYGDYKVYDVRKMIAKSEQEIGKATAKYFFYSNIMNFFKTE
ITLANGEIRKRPLIETNGETGEIVWDKGRDFATVRKVLSMPQVNIVKKTEVQTGGFSKE
SILPKRNSDKLIARKKDWDPKKYGGFDSPTVAYSVLVVAKVEKGKSKKLKSVKELLGI

TIMERSSFEKNPIDFLEAKGYKEVKKDLIIKLPKYSLFELENGRKRMLASAGELQKGNE
LALPSKYVNFLYLASHYEKLKGSPEDNEQKQLFVEQHKHYLDEIIEQISEFSKRVILAD
ANLDKVLSAYNKHRDKPIREQAENIIHLFTLTNLGAPAAFKYFDTTIDRKRYTSTKEVL
DATLIHQSITGLYETRIDLSQLGGD (SEQ ID NO: 61)

A nucleotide sequence of the nCas9 is as follows:

gacaagaagtacagcatcggcctggccatcggcaccaactctgtgggctgggccgtgatcaccgacgagtacaaggtgccca
gcaagaaattcaaggtgctgggcaacaccgaccggcacagcatcaagaagaacctgatcggagccctgctgttcgacagcggcgaa
acagccgaggccacccggctgaagagaaccgccagaagaagatacaccagacggaagaaccggatctgctatctgcaagagatctt
cagcaacgagatggccaaggtggacgacagcttcttccacagactggaagagtccttcctggtggaagaggataagaagcacgagcg
gcaccccatcttcggcaacatcgtggacgaggtggcctaccacgagaagtaccccaccatctaccacctgagaaagaaactggtgga
cagcaccgacaaggccgacctgcggctgatctatctggccctggcccacatgatcaagttccggggccacttcctgatcgagggcgac
ctgaaccccgacaacagcgacgtggacaagctgttcatccagctggtgcagacctacaaccagctgttcgaggaaaaccccatcaacg
ccagcggcgtggacgccaaggccatcctgtctgccagactgagcaagagcagacggctggaaaatctgatcgcccagctgcccggc
gagaagaagaatggcctgttcggaaacctgattgccctgagcctgggcctgacccccaacttcaagagcaacttcgacctggccgagg
atgccaaactgcagctgagcaaggacacctacgacgacgacctggacaacctgctggcccagatcggcgaccagtacgccgacctg
tttctggccgccaagaacctgtccgacgccatcctgctgagcgacatcctgagagtgaacaccgagatcaccaaggcccccctgagcg
cctctatgatcaagagatacgacgagcaccaccaggacctgaccctgctgaaagctctcgtgcggcagcagctgcctgagaagtacaa
agagattttcttcgaccagagcaagaacggctacgccggctacattgacggcggagccagccaggaagagttctacaagttcatcaag
cccatcctggaaaagatggacggcaccgaggaactgctcgtgaagctgaacagagaggacctgctgcggaagcagcggaccttcga
caacggcagcatcccccaccagatccacctgggagagctgcacgccattctgcggcggcaggaagatttttacccattcctgaaggac
aaccgggaaaagatcgagaagatcctgaccttccgcatcccctactacgtgggccctctggccaggggaaacagcagattcgcctgg
atgaccagaaagagcgaggaaaccatcaccccctggaacttcgaggaagtggtggacaagggcgcttccgcccagagcttcatcga
gcggatgaccaacttcgataagaacctgcccaacgagaaggtgctgcccaagcacagcctgctgtacgagtacttcaccgtgtataac
gagctgaccaaagtgaaatacgtgaccgagggaatgagaaagcccgccttcctgagcggcgagcagaaaaaggccatcgtggacct

gctgttcaagaccaaccggaaagtgaccgtgaagcagctgaaagaggactacttcaagaaaatcgagtgcttcgactccgtggaaatct
ccggcgtggaagatcggttcaacgcctccctgggcacataccacgatctgctgaaaattatcaaggacaaggacttcctggacaatgag
gaaaacgaggacattctggaagatatcgtgctgaccctgacactgtttgaggacagagagatgatcgaggaacggctgaaaacctatg
cccacctgttcgacgacaaagtgatgaagcagctgaagcggcggagatacaccggctggggcaggctgagccggaagctgatcaac
ggcatccgggacaagcagtccggcaagacaatcctggatttcctgaagtccgacggcttcgccaacagaaacttcatgcagctgatcc
acgacgacagcctgacctttaaagaggacatccagaaagcccaggtgtccggccagggcgatagcctgcacgagcacattgccaatc
tggccggcagccccgccattaagaagggcatcctgcagacagtgaaggtggtggacgagctcgtgaaagtgatgggccggcacaag
cccgagaacatcgtgatcgaaatggccagagagaaccagaccacccagaagggacagaagaacagccgcgagagaatgaagcgg
atcgaagagggcatcaaagagctgggcagccagatcctgaaagaacaccccgtggaaaacacccagctgcagaacgagaagctgta
cctgtactacctgcagaatgggcgggatatgtacgtggaccaggaactggacatcaaccggctgtccgactacgatgtggaccatatcg
tgcctcagagctttctgaaggacgactccatcgacaacaaggtgctgaccagaagcgacaagaaccggggcaagagcgacaacgtg
ccctccgaagaggtcgtgaagaagatgaagaactactggcggcagctgctgaacgccaagctgattacccagagaaagttcgacaat
ctgaccaaggccgagagaggcggcctgagcgaactggataaggccggcttcatcaagagacagctggtggaaacccggcagatca
caaagcacgtggcacagatcctggactcccggatgaacactaagtacgacgagaatgacaagctgatccgggaagtgaaagtgatca
ccctgaagtccaagctggtgtccgatttccggaaggatttccagttttacaaagtgcgcgagatcaacaactaccaccacgcccacgac
gcctacctgaacgccgtcgtgggaaccgccctgatcaaaaagtaccctaagctggaaagcgagttcgtgtacggcgactacaaggtgt
acgacgtgcggaagatgatcgccaagagcgagcaggaaatcggcaaggctaccgccaagtacttcttctacagcaacatcatgaactt
tttcaagaccgagattaccctggccaacggcgagatccggaagcggcctctgatcgagacaaacggcgaaaccggggagatcgtgtg
ggataagggccgggattttgccaccgtgcggaaagtgctgagcatgccccaagtgaatatcgtgaaaaagaccgaggtgcagacagg
cggcttcagcaaagagtctatcctgcccaagaggaacagcgataagctgatcgccagaaagaaggactgggaccctaagaagtacgg
cggcttcgacagccccaccgtggcctattctgtgctggtggtggccaaagtggaaaagggcaagtccaagaaactgaagagtgtgaaa
gagctgctggggatcaccatcatggaaagaagcagcttcgagaagaatcccatcgactttctggaagccaagggctacaaagaagtga
aaaaggacctgatcatcaagctgcctaagtactccctgttcgagctggaaaacggccggaagagaatgctggcctctgccggcgaact
gcagaagggaaacgaactggccctgccctccaaatatgtgaacttcctgtacctggccagccactatgagaagctgaagggctccccc

gaggataatgagcagaaacagctgtttgtggaacagcacaagcactacctggacgagatcatcgagcagatcagcgagttctccaaga

gagtgatcctggccgacgctaatctggacaaagtgctgtccgcctacaacaagcaccgggataagcccatcagagagcaggccgaga

atatcatccacctgtttaccctgaccaatctgggagcccctgccgccttcaagtactttgacaccaccatcgaccggaagaggtacacca

gcaccaaagaggtgctggacgccaccctgatccaccagagcatcaccggcctgtacgagacacggatcgacctgtctcagctgggag

gtgac (SEQ ID NO: 62).

### Beneficial Effects of the Invention

According to the present invention, by performing site-directed mutation on a parental adenosine deaminase, an editing efficiency of the corresponding base editor is improved, an application prospect is expanded, and the potential utilization value is achieved. The base editor according to the present invention has a significantly higher editing efficiency on a PCSK9 target and other sites than a wild type, and has great potential in the field of treatment of diseases associated with or caused by a point mutation (such as hypercholesterolemia, transthyretin amyloidosis, and β-hemoglobinopathy). Through extensive exploration and research, for some base editors constituted by an adenosine deaminase and a nuclease, a chimeric position suitable for the adenosine deaminase of the present invention in the nCas9 protein is found, further improving the base editing efficiency.

In any of the embodiments of the present invention described herein, including the embodiments described only in Examples or claims or described only in one of the following aspects/parts, it should be understood that the embodiments may be combined with any other one or more embodiments of the present invention unless explicitly denied or the combination is inappropriate.

### BRIEF DESCRIPTION OF THE DRAWINGS

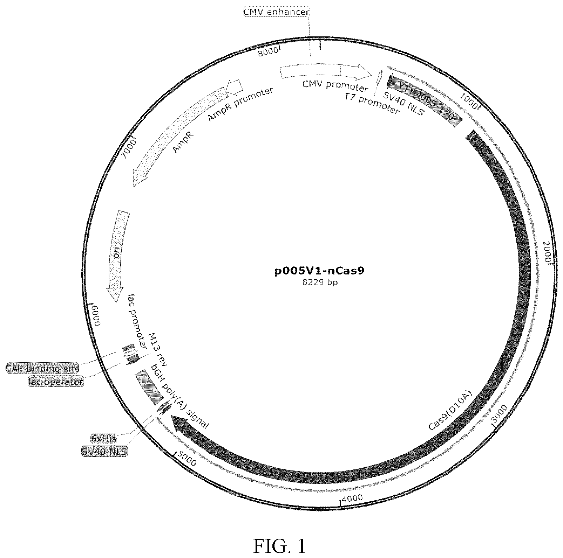

FIG. 1 is a plasmid map of 005V1-nCas9;
FIG. 2 shows editing efficiencies of the 005V1-nCas9 and each mutant base editor at a PCSK9 site, in which an editing targeting site is a PCSK9 gene, and cells used are 293T cells;
FIG. 3 shows a comparison of the editing efficiencies of the 005V1-nCas9 and each mutant base editor at the PCSK9 site, and in order to more clearly show the comparison of the editing efficiencies of the mutant base editor and the 005V1-nCas9, an editing efficiency value of the 005V1-nCas9 is set to 1, and the editing efficiency of each of the other mutant base editors is calculated in proportion;

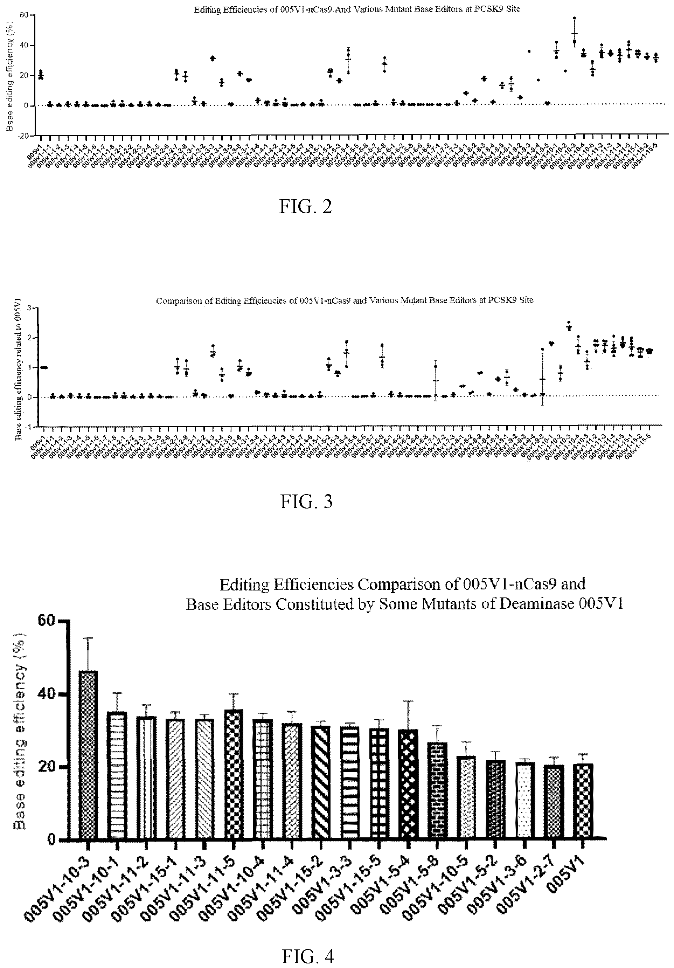

FIG. 4 shows a comparison of editing efficiencies of the 005V1-nCas9 and mutant base editors with editing efficiency, including a comparison of editing efficiencies of base editors such as 005V1-10-3-nCas9, 005V1-10-1-nCas9, 005V1-11-2-nCas9, 005V1-15-1-nCas9, 005V1-11-3-nCas9, 005V1-11-5-nCas9, 005V1-10-4-nCas9, 005V1-11-4-nCas9, 005V1-15-2-nCas9, 005V1-3-3-nCas9, 005V1-15-5-nCas9, 005V1-5-4-nCas9, 005V1-5-8-nCas9, 005V1-10-5-nCas9, 005V1-5-2-nCas9, 005V1-3-6-nCas9, 005V1-2-7-nCas9, and 005V1-nCas9, and for ease of display, only an adenosine deaminase name is marked in the figure to refer to the corresponding base editor;
FIG. 5 shows a comparison of editing efficiencies of the 005V1-nCas9 and some mutant base editors at different sites, in which different depths of color in the figure represent different editing efficiencies, and for ease of display, only the adenosine deaminase name is marked in the figure to refer to the corresponding base editor;
FIGS. 6A and 6B are a plasmid map of PHK09 (FIG. 6A) and a structure diagram of 004V1-nCas9 (FIG. 6B), respectively;

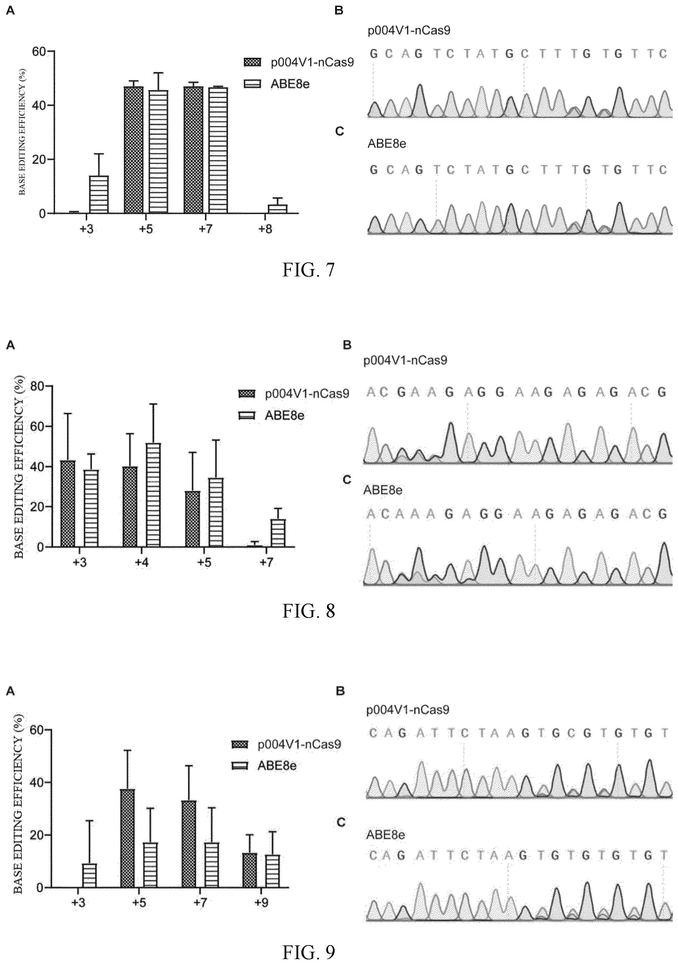

FIG. 7 shows an editing efficiency of the 004V1-nCas9 from A·T to G·C at a site 1, including A to C, in which A of FIG. 7 shows a comparison of editing efficiencies of the 004V1-nCas9 and ABE8e at the site 1, editing positions are adenine deoxynucleotides +3, +5, +7, and +8 from a 5' end of sgRNA, an error line represents mean ± SEM, and there are three biological replicates for each group of samples; B of FIG. 7 shows a sequencing result of the base editor 004V1-nCas9 at the site 1 after transfection; and C of FIG. 7 shows a sequencing result of the base editor ABE8e at the site 1 after transfection;
FIG. 8 shows an editing efficiency of the 004V1-nCas9 from A·T to G·C at a site 17, including A to C, in which A of FIG. 8 shows a comparison of editing efficiencies of the 004V1-nCas9 and ABE8e at the site 17, editing positions are adenine deoxynucleotides +3, +4, +5, and +7 from a 5' end of sgRNA, an error line represents mean ± SEM, and there are three biological replicates for each group of samples; B of FIG. 8 shows a sequencing result of the base editor 004V1-nCas9 at the site 17 after transfection; and C of FIG. 8 shows a sequencing result of the base editor ABE8e at the site 17 after transfection;
FIG. 9 shows an editing efficiency of the 004V1-nCas9 from A·T to G·C at a site 18, including A to C, in which A of FIG. 9 shows a comparison of editing efficiencies of the 004V1-nCas9 and ABE8e at the site 18, editing positions are adenine deoxynucleotides +3, +5, +7, and +9 from a 5' end of sgRNA, an error line represents mean ± SEM, and there are three biological replicates for each group of samples; B of FIG. 9 shows a sequencing result of the base editor 004V1-nCas9 at the site 18 after transfection; and C of FIG. 9 shows a sequencing result of the base editor ABE8e at the site 18 after transfection;
FIG. 10 shows an editing efficiency of the 004V1-nCas9 from A-T to G·C at the PCSK9 site, including A to C, FIG. 10A shows a comparison of editing efficiencies of the 004V1-nCas9 and ABE8e at the PCSK9 site, an editing position is an adenine deoxynucleotide +6 from a 5' end of sgRNA, an error line represents mean ± SEM, and there are three biological replicates for each group of samples; FIG. 10B shows a sequencing result of the base editor 004V1-nCas9 at the PCSK9 site after transfection; and FIG. 10C shows a sequencing result of the base editor ABE8e at the PCSK9 site after transfection;
FIG. 11 shows editing efficiencies of each adenosine deaminase 004V1 mutant-nCas9 and ABE8e at different sites;
FIG. 12 shows editing efficiencies of base editors constituted by other mutants of adenosine deaminase 004V1 at different sites;
FIG. 13 shows base editing efficiencies of a part of chimeric base editors and base editors with deaminase connected to an N-terminal or a C-terminal of nCas9 at the site 1;
FIG. 14 shows base editing efficiencies of a part of chimeric base editors and base editors with deaminase connected to an N-terminal or a C-terminal of nCas9 at the PCSK9 site; and
FIG. 15 shows base editing efficiencies of a part of chimeric base editors and base editors with deaminase connected to an N-terminal or a C-terminal of nCas9 at a FANCF site.

### DETAILED DESCRIPTION OF THE EMBODIMENTS

Unless otherwise specified, the experiments and methods described in the examples were basically performed according to conventional methods well known in the art and described in various references.For example, for general techniques of immunology, biochemistry, chemistry, molecular biology, microbiology, cell biology, genomics, recombinant DNA, and the like used in the present invention, see Sambrook, Fritsch, and Maniatis, "MOLECULAR CLONING: A LABORATORY MANUAL", 2nd edition (1989); "CURRENT PROTOCOLS IN MOLECULAR BIOLOGY" (F.M. Ausubel et al., ed., (1987)); "METHODS IN ENZYMOLOGY" series (Academic Press Corporation): "PCR 2: A PRACTICAL APPROACH" (M.J. MacPherson, B.D. Hames, and G.R. Taylor, ed. (1995)); "ANTIBODIES, A LABORATORY MANUAL" (1988) edited by Harlow and Lane; and "ANIMAL CELL CULTURE" (R.I. Freshney, ed. (1987)).

In addition, for those without specific conditions in the examples, they were carried out in accordance with the conventional conditions or the conditions recommended by the manufacturer. The reagents or instruments used without the manufacturer's indication were all conventional products that were purchased commercially. Those skilled in the art will appreciate that Examples describe the present invention by way of example and are not intended to limit the claimed scope of the present invention. All disclosures and other references mentioned herein are incorporated herein by reference in their entirety.

### Example 1: Preparation of Base Editors Constituted by Adenosine Deaminase 005V1 and Various Mutants

The applicant predicted a key amino acid site that may affect its biological function according to bioinformatics functional prediction, and mutated the amino acid site to obtain a plurality of adenosine deaminase mutants whose editing activity is significantly improved compared with the base editor constituted by the adenosine deaminase 005V1. Specific amino acid site mutation patterns of the deaminase 005V1 are shown in Tables 1a and 1b, and a mutation pattern of each mutant is shown in Table 2.

**Table 1a Amino acid Substitutions of deaminase 005V1**

| Mutatio n site | Substitution | Codon before mutation | Codon after mutation | Mutatio n site | Substitution s | Codon before mutation | Codon after mutation |
|---|---|---|---|---|---|---|---|
| 19 | K19L | AAG | CTT | 107 | S107E | TCA | GAA |
| 19 | K19F | AAG | TTT | 107 | S107T | TCA | ACT |
| 19 | K19R | AAG | CGA | 108 | K108E | AAA | GAA |
| 19 | K19H | AAG | CAC | 108 | K108G | AAA | GGC |
| 19 | K19Y | AAG | TAC | 108 | K108L | AAA | CTA |
| 20 | A20G | GCC | GGG | 108 | K108R | AAA | AGG |
| 20 | A20I | GCC | ATC | 108 | K108Y | AAA | TAC |
| 21 | R21G | CGG | GGG | 109 | R109L | AGA | CTT |
| 21 | R21E | CGG | GAG | 109 | R109K | AGA | AAG |
| 21 | R21T | CGG | ACT | 109 | R109G | AGA | GGA |
| 21 | R21F | CGG | TTT | 109 | R109I | AGA | ATA |
| 22 | E22S | GAA | TCA | 110 | G110A | GGC | GCT |
| 22 | E22D | GAA | GAC | 110 | G110M | GGC | ATG |
| 22 | E22R | GAA | CGC | 110 | G110R | GGC | CGA |
| 22 | E22L | GAA | CTC | 117 | N117T | AAC | ACC |
| 22 | E22T | GAA | ACT | 118 | V118G | GTG | GGG |
| 33 | V33L | GTG | CTG | 119 | L119R | CTG | CGG |
| 33 | V33I | GTG | ATT | 120 | N120G | AAC | GGC |
| 33 | V33P | GTG | CCT | 120 | N120D | AAC | GAC |
| 34 | L34A | CTG | GCG | 120 | N120K | AAC | AAA |
| 34 | L34M | CTG | ATG | 121 | Y121L | TAC | CTT |
| 34 | L34R | CTG | CGC | 121 | Y121S | TAC | AGT |
| 34 | L34I | CTG | ATA | 122 | P122A | CCC | GCT |
| 35 | D35E | GAT | GAA | 122 | P122K | CCC | AAA |
| 35 | D35R | GAT | AGA | 123 | G123L | GGC | TTA |
| 35 | D35G | GAT | GGA | 123 | G123S | GGC | TCC |
| 35 | D35P | GAT | CCA | 123 | G123T | GGC | ACG |
| 36 | D36G | GAT | GGG | 144 | C144A | GGC | GCG |
| 36 | D36N | GAT | AAC | | | | |

**Table 1b Amino acid Substitutions of deaminase 005V1**

| Mutation site | Mutati on pattern | Codon before mutation | Codon after mutation | Mutation site | Mutatio n pattern | Codon before mutation | Codon after mutation |
|---|---|---|---|---|---|---|---|
| 36 | D36P | GAT | CCA | 144 | C144F | TGC | TTC |
| 36 | D36Q | GAT | CAA | 144 | C144S | TGC | AGT |
| 36 | D36T | GAT | ACG | 144 | C144W | TGC | TGG |
| 46 | A46C | GCC | TGC | 145 | Q145A | CAG | GCG |
| 46 | A46N | GCC | AAT | 145 | Q145S | CAG | TCA |
| 46 | A46S | GCC | TCG | 145 | Q145R | CAG | AGG |
| 47 | I47G | ATC | GGC | 145 | Q145W | CAG | TGG |
| 47 | I47N | ATC | AAC | 146 | F146C | TTC | TGC |
| 47 | I47Y | ATC | TAC | 146 | F146R | TTC | CGC |
| 47 | I47V | ATC | GTA | 146 | F146V | TTC | GTC |
| 47 | I47R | ATC | AGA | 146 | F146Y | TTC | TAC |
| 48 | T48A | ACC | GCC | 147 | Y147G | TAT | GGT |
| 48 | T48Q | ACC | CAA | 147 | Y147L | TAT | TTA |
| 48 | T48R | ACC | CGA | 147 | Y147I | TAT | ATA |
| 48 | T48G | ACC | GGC | 147 | Y147W | TAT | TGG |
| 48 | T48V | ACC | GTT | 148 | Q148P | CAG | CCG |
| 48 | T48Y | ACC | TAT | 148 | Q148C | CAG | TGC |
| 49 | L49A | CTG | GCC | 148 | Q148A | CAG | GCA |
| 49 | L49K | CTG | AAA | 148 | Q148G | CAG | GGT |
| 49 | L49T | CTG | ACG | 149 | Q149M | CAG | ATG |
| 49 | L49H | CTG | CAT | 149 | Q149G | CAG | GGG |
| 49 | L49N | CTG | AAT | 149 | Q149L | CAG | CTC |
| 49 | L49S | CTG | TCA | 150 | P150R | CCT | CGC or CGA |
| 81 | T81N | ACC | AAC | 150 | P150L | CCT | CTT |
| 81 | T81I | ACC | ATC | 150 | P150C | CCT | TGT |
| 83 | E83A | GAG | CGC | 150 | P150V | CCT | GTG |
| 83 | E83Q | GAG | CAG | 151 | R151K | AGG | AAG |
| 84 | P84L | CCC | CTC | 152 | E152R | GAA | AGG |
| 84 | P84S | CCC | TCC | 152 | E152T | GAA | ACG |
| 104 | V104A | GTG | GCG | 152 | E152G | GAA | GGG |
| 104 | V104M | GTG | ATG | 152 | E152W | GAA | TGG |
| 105 | S105C | AGC | TGC | 153 | V153P | GTG | CCA |
| 105 | S105G | AGC | GGC | 153 | V153F | GTG | TTT |
| 105 | S105I | AGC | ATC | 153 | V153I | GTG | ATA |
| 105 | S105R | AGC | CGC | 153 | V153T | GTG | ACA |
| 106 | N106D | AAC | GAC | 154 | F154H | TTC | CAT |
| 106 | N106H | AAC | CAC | 154 | F154K | TTC | AAG |
| 107 | S107Y | TCA | TAC | 154 | F154L | TTC | CTG |
| 107 | S107R | TCA | AGG | 155 | N155T | AAT | ACG or ACT |
| 107 | S107K | TCA | AAG or AAA | 155 | N155R | AAT | CGC |
| 107 | S107A | TCA | GCA & GCT | 155 | N155H | AAT | CAC |

**Table 2 Mutation forms of various mutants of adenosine deaminase 005V1**

| Mutant | Mutation form | Mutant | Mutation form |
|---|---|---|---|
| 005V1-10-3 | Q148G + Q149M + P150R | 005V1-15-5 | S107A |
| 005V1-11-5 | E152R + V153P + F154H + N155T | 005V1-5-4 | V104M |
| 005V1-10-1 | Q148P + Q149G + P150L | 005V1-5-8 | S105C |
| 005V1-11-2 | E152T + V153F + N155T | 005V1-10-5 | Q148A + Q149G + P150C |
| 005V1-15-1 | S107R | 005V1-5-2 | S105G |
| 005V1-11-3 | E152G + V153I + F154K + N155R | 005V1-3-6 | I47R + T48G + L49T |
| 005V1-10-4 | Q148C + Q149L + P150R + R151K | 005V1-4-5 | E83A |
| 005V1-11-4 | E152W + V153T + F154L + N155H | 005V1-4-7 | T81N |
| 005V1-15-2 | S 107K | 005V1-4-8 | E83Q |
| 005V1-2-7 | D35G + D36N | 005V1-5-1 | N106D |
| 005V1-1-1 | K19F + R21G + E22R | 005V1-5-3 | V104A |
| 005V1-1-2 | K19L + R21E + E22T | 005V1-5-5 | S105I |
| 005V1-1-3 | K19R + R21T + E22R | 005V1-5-6 | N106H |
| 005V1-1-4 | K19H + R21F | 005V1-5-7 | S105R |
| 005V1-1-5 | E22L | 005V1-6-1 | K108R |
| 005V1-1-6 | K19L + A20G + R21F + E22S | 005V1-6-2 | S107T + K108G + R109L + G110A |
| 005V1-1-7 | K19Y + A20I + R21G + E22S | 005V1-6-5 | S107Y + K108E + R109G |
| 005V1-1-8 | R21F + E22D | 005V1-6-6 | S107E + K108Y + R109I + G110M |
| 005V1-2-1 | V33L + D35G | 005V1-6-8 | K108L + R109K + G110R |
| 005V1-2-2 | V33P + L34A + D35P + D36P | 005V1-7-1 | N117T |
| 005V1-2-3 | L34R + D35G + D36T | 005V1-7-2 | L119R |
| 005V1-2-5 | V33I + L34I + D36Q | 005V1-7-3 | V118G |
| 005V1-2-6 | L34M + D35E + D36N | 005V1-8-1 | Y121S |
| 005V1-2-8 | V33I + D35R + D36G | 005V1-8-2 | N120G +Y121S + P122K + G123S |
| 005V1-3-1 | A46S + T48G + L49N | 005V1-8-3 | N120D |
| 005V1-3-2 | A46N + I47N + T48Q + L49S | 005V1-8-4 | N120K + P122A + G123T |
| 005V1-3-3 | A46C + I47Y + T48G + L49H | 005V1-8-5 | N120G + Y121L + G123L |
| 005V1-3-4 | I47V + T48R + L49K | 005V1-9-1 | C144W + Q145A + F146C + Y147I |
| 005V1-3-5 | A46C + I47Y + T48A + L49H | 005V1-9-2 | C144A + Y147W |
| 005V1-3-7 | A46S + I47G + T48Y + L49A | 005V1-9-3 | C144F + Q145W + F146Y |
| 005V1-3-8 | A46N + I47R + T48V + L49S | 005V1-9-4 | C144S + Q145R + F146R + Y147G |
| 005V1-4-1 | T81I | 005V1-9-5 | C144F + Q145S + F146V + Y147L |
| 005V1-4-2 | P84L | 005V1-10-2 | Q148G + Q149G + P150V + R151K |
| 005V1-4-3 | P84S | | |

Base editors of different deaminase variants were generated by PCR-based site-directed mutagenesis. A specific method was to amplify a DNA sequence encoding a 005V1-nCas9 base editor with 4 to 6 amino acids near a mutation site as the center, introduce a sequence to be mutated on a primer at the same time, and obtain different mutant base editors by homologous recombination or enzyme ligation of the amplified fragments. PCR primers involved in the mutant base editors are shown in Tables 3-1, 3-2, and 3-3.

**Table 3-1 PCR primers used for each mutant base editor**

| Base editor variants | Primer (5'-3') |
|---|---|
| 005V1-10-3 | F: ggctacggtctcatgcCAGTTCtatGGTATGCGCAGAGAAgtgttcaatGCTGAGCG (SEQ ID NO. 5) |
| | R: ggctacggtctcaGgcaCAGCATCTCGCTGCAC (SEQ ID NO. 6) |
| 005V1-11-5 | F: ggctacggtctcaCAGCAGcctAGGAGGCCACATACTGCTGAGCGTGAGGCTAGG CG (SEQ ID NO. 7) |
| | R: ggctacggtctcaGCTGataGAACTGgcaCAGC (SEQ ID NO. 8) |
| 005V1-10-1 | F: ggctacggtctcatgcCAGTTCtatCCGGGTCTTCGCGAAgtgttcaatGCTGAGCG (SEQ ID NO. 9) |
| | R: ggctacggtctcaGgcaCAGCATCTCGCTGCAC (SEQ ID NO. 10) |
| 005V1-11-2 | F: ggctacggtctcaCAGCAGcctAGGACGTTTTTTACGGCTGAGCGTGAGGCTAGGC G (SEQ ID NO. 11) |
| | R: ggctacggtctcaGCTGataGAACTGgcaCAGC (SEQ ID NO. 12) |
| 005V1-15-1 | F: ggctacggtctcaGGTCTACGGCgtgAGGaactcaAAAagaGGCGCCGC (SEQ ID NO. 13) |
| | R: ggctacggtctcaGACCAGTCTCTTGATCCTGC (SEQ ID NO. 14) |
| 005V1-11-3 | F: ggctacggtctcaCAGCAGcctAGGGGGATAAAGCGCGCTGAGCGTGAGGCTAGG CG (SEQ ID NO. 15) |
| | R: ggctacggtctcaGCTGataGAACTGgcaCAGC (SEQ ID NO. 16) |
| 005V1-10-4 | F: ggctacggtctcatgcCAGTTCtatTGCCTCCGAAAGGAAgtgttcaatGCTGAGCG (SEQ ID NO. 17) |
| | R: ggctacggtctcaGgcaCAGCATCTCGCTGCAC (SEQ ID NO. 18) |
| 005V1-11-4 | F: ggctacggtctcaCAGCAGcctAGGTGGACACTGCACGCTGAGCGTGAGGCTAGG CG (SEQ ID NO. 19) |
| | R: ggctacggtctcaGCTGataGAACTGgcaCAGCATCTCGCTGC (SEQ ID NO. 20) |
| 005V1-15-2 | F: ggctacggtctcaGGTCTACGGCgtgAAAaactcaAAAagaGGCGCCGC (SEQ ID NO. 21) |
| | R: ggctacggtctcaGACCAGTCTCTTGATCCTGCTGTGCACCATG (SEQ ID NO. 22) |
| 005V1-3-3 | F: TAGGACAGGGATGGAATAGATGCTACGGCCATCACGACCCCACCGCCCAC GC (SEQ ID NO. 23) |
| | R: TCTATTCCATCCCTGTCCTA (SEQ ID NO. 24) |
| 005V1-15-5 | F: ggctacggtctcaGGTCTACGGCgtgGCTaactcaAAAagaGGCGCCGC (SEQ ID NO. 25) |
| | R: ggctacggtctcaGACCAGTCTCTTGATCCTGCTGTGCACCATG (SEQ ID NO. 26) |
| 005V1-5-4 | F: GGATCAAGAGACTGGTCTACGGCATGAGCAACtcaAAAagaGGCGCCGCCG G (SEQ ID NO. 27) |
| | R: GCCGTAGACCAGTCTCTT (SEQ ID NO. 28) |
| 005V1-5-8 | F: GGATCAAGAGACTGGTCTACGGCGTGTGCAACtcaAAAagaGGCGCCGCCG |
| | G (SEQ ID NO. 29) |
| | R: acGCCGTAGACCAGTCTCTTGATCCTGCTG (SEQ ID NO. 30) |
| 005V1-10-5 | F: ggctacggtctcatgcCAGTTCtatGCAGGGTGTCGTGAAgtgttcaatGCTGAGCG (SEQ ID NO. 31) |
| | R: ggctacggtctcaGgcaCAGCATCTCGCTGCAC (SEQ ID NO. 32) |
| 005V1-5-2 | F: GATCAAGAGACTGGTCTACGTGAGCAACtcaAAAagaGGC (SEQ ID NO. 33) |
| | R: CGTAGACCAGTCTCTTGATC (SEQ ID NO. 34) |
| 005V1-3-6 | F: ACAGGGATGGAATAGAGCCAGAGGAACGCACGACCCCACCGCCCACG (SEQ ID NO. 35) |
| | R: TCTATTCCATCCCTGTCCTA (SEQ ID NO. 36) |
| 005V1-2-7 | F: CCAGTGGGCGCTATTCTGGTCTTGGGCAACGAAGTGATAGGACAGGGATG (SEQ ID NO. 37) |
| | R: CAGAATAGCGCCCACTGGAA (SEQ ID NO. 38) |

**Table 3-2 PCR primers used for each mutant base editor**

| Base editor variants | Primer (5'-3') |
|---|---|
| 005V1-1-1 | F: CTGGATGAAACAGGCACTCGTTTGCAGGGAGACAGGGAGAAGTTCCAG TGG (SEQ ID NO. 39) |
| | R: CGAGTGCCTGTTTCATCCAG (SEQ ID NO. 40) |
| 005V1-1-2 | F: CTGGATGAAACAGGCACTCGCTTGCGGAGACTCAGGGAGAAGTTCCAG TGGG (SEQ ID NO. 41) |
| | R: CGAGTGCCTGTTTCATCCAG (SEQ ID NO. 42) |
| 005V1-1-3 | F: CTGGATGAAACAGGCACTCGCGAGCGACTCGCCAGGGAGAAGTTCCAG TGGG (SEQ ID NO. 43) |
| | R: CGAGTGCCTGTTTCATCCAG (SEQ ID NO. 44) |
| 005V1-1-4 | F: CTGGATGAAACAGGCACTCGCACGCGTTTGAACAGGGAGAAGTTCCAG T (SEQ ID NO. 45) |
| | R: CGAGTGCCTGTTTCATCCAG (SEQ ID NO. 46) |
| 005V1-1-5 | F: AACAGGCACTCGAAGGCCCGTCTCCAGGGAGAAGTTCCAGTGGG (SEQ ID NO. 47) |
| | R: CGAGTGCCTGTTTCATCCAG (SEQ ID NO. 48) |
| 005V1-1-6 | F: CTGGATGAAACAGGCACTCGCTCGGGTTTTCACAGGGAGAAGTTCCAG TGG (SEQ ID NO. 49) |
| | R: CGAGTGCCTGTTTCATCCAG (SEQ ID NO. 50) |
| 005V1-1-7 | F: CTGGATGAAACAGGCACTCGTACATCGGATCCCAGGGAGAAGTTCCAGT GGG (SEQ ID NO. 51) |
| | R: CGAGTGCCTGTTTCATCCAG (SEQ ID NO. 52) |
| 005V1-1-8 | F: GGATGAAACAGGCACTCGAAAGCATTTGACCAGGGAGAAGTTCCAGTG GG (SEQ ID NO. 53) |
| | R: CGAGTGCCTGTTTCATCCAG (SEQ ID NO. 54) |
| 005V1-2-1 | F: TTCCAGTGGGCGCTATTCTGCTGTTAGGAGACGAAGTGATAGGACAGGG ATG (SEQ ID NO. 55) |
| | R: CAGAATAGCGCCCACTGGAA (SEQ ID NO. 56) |
| 005V1-2-2 | F: TTCCAGTGGGCGCTATTCTGCCTGCGCCACCAGAAGTGATAGGACAGGG ATG (SEQ ID NO. 57) |
| | R: CAGAATAGCGCCCACTGGAA (SEQ ID NO. 58) |
| 005V1-2-3 | F: CCAGTGGGCGCTATTCTGGTCCGCGGGACGGAAGTGATAGGACAGGGAT G (SEQ ID NO. 59) |
| | R: CAGAATAGCGCCCACTGGAA (SEQ ID NO. 60) |
| 005V1-2-5 | F: TTCCAGTGGGCGCTATTCTGATAATAGATCAAGAAGTGATAGGACAGGG ATG (SEQ ID NO. 63) |
| | R: CAGAATAGCGCCCACTGGAA (SEQ ID NO. 64) |
| 005V1-2-6 | F: CCAGTGGGCGCTATTCTGGTTATGGAAAATGAAGTGATAGGACAGGGA (SEQ ID NO. 65) |
| | R: CAGAATAGCGCCCACTGGAA (SEQ ID NO. 66) |
| 005V1-2-8 | F: TTCCAGTGGGCGCTATTCTGATTCTAAGAGGGGAAGTGATAGGACAGGG |
| | ATG (SEQ ID NO. 67) |
| | R: CAGAATAGCGCCCACTGGAA (SEQ ID NO. 68) |
| 005V1-3-1 | F: TAGGACAGGGATGGAATAGATCGATTGGCAATCACGACCCCACCGCCCA CGC (SEQ ID NO. 69) |
| | R: TCTATTCCATCCCTGTCCTA (SEQ ID NO. 70) |
| 005V1-3-2 | F: TAGGACAGGGATGGAATAGAAATAACCAATCACACGACCCCACCGCCCA CGC (SEQ ID NO. 71) |
| | R: TCTATTCCATCCCTGTCCTA (SEQ ID NO. 72) |
| 005V1-3-4 | F: GGACAGGGATGGAATAGAGCTGTACGAAAACACGACCCCACCGCCCAC GC (SEQ ID NO. 73) |
| | R: TCTATTCCATCCCTGTCCTA (SEQ ID NO. 74) |
| 005V1-3-5 | F: TAGGACAGGGATGGAATAGATGTTACGCCCATCACGACCCCACCGCCCA CGC (SEQ ID NO. 75) |
| | R: TCTATTCCATCCCTGTCCTA (SEQ ID NO. 76) |
| 005V1-3-7 | F: TAGGACAGGGATGGAATAGATCGGGCTATGCCCACGACCCCACCGCCCA CGC (SEQ ID NO. 77) |
| | R: TCTATTCCATCCCTGTCCTA (SEQ ID NO. 78) |
| 005V1-3-8 | F: TAGGACAGGGATGGAATAGAAACCGCGTTTCACACGACCCCACCGCCC ACGC (SEQ ID NO. 79) |
| | R: TCTATTCCATCCCTGTCCTA (SEQ ID NO. 80) |
| 005V1-4-1 | F: ACGCCACCCTGTACGTGATCTTCGAGCCCTGCGTGAT (SEQ ID NO. 81) |
| | R: TCACGTACAGGGTGGCGT (SEQ ID NO. 82) |
| 005V1-4-2 | F: TGTACGTGACCTTCGAGCTCTGCGTGATGTGCGCCGG (SEQ ID NO. 83) |
| | R: GCTCgaaGGTCACGTACA (SEQ ID NO. 84) |
| 005V1-4-3 | F: CTGTACGTGACCTTCGAGTCCTGCGTGATGTGCGCCG (SEQ ID NO. 85) |
| | R: CTCgaaGGTCACGTACAG (SEQ ID NO. 86) |
| 005V1-9-1 | F: ggctacggtctcaAGCGAGATGCTGTGGGCGTGCATACAGCAGcctAGGGAAgtgtt (SEQ ID NO. 87) |
| | R: ggctacggtctcaCGCTGCACTCGTTGGCCATCACGCCGGCGGT (SEQ ID NO. 88) |
| 005V1-9-2 | F: ggctacggtctcaAGCGAGATGCTGGCGCAATTTTGGCAGCAGcctAGGGAAgtgtt (SEQ ID NO. 89) |
| | R: ggctacggtctcaCGCTGCACTCGTTGGCCATCACGCCGGCGGT (SEQ ID NO. 90) |
| 005V1-9-3 | F: ggctacggtctcaAGCGAGATGCTGTTCTGGTACTATCAGCAGcctAGGGAAgtgtt (SEQ ID NO. 91) |
| | R: ggctacggtctcaCGCTGCACTCGTTGGCCATCACGCCGGCGGT (SEQ ID NO. 92) |
| 005V1-9-4 | F: ggctacggtctcaAGCGAGATGCTGAGTAGGCGCGGTCAGCAGcctAGGGAAgtgt t (SEQ ID NO. 93) |
| | R: ggctacggtctcaCGCTGCACTCGTTGGCCATCACGCCGGCGGT (SEQ ID NO. 94) |

**Table 3-3 PCR primers used for each mutant base editor**

| Base editor variants | Primer (5'-3') |
|---|---|
| 005V1-4-5 | F: CCCTGTACGTGACCTTCGCGCCCTGCGTGATGTGCGC (SEQ ID NO. 95) |
| | R: CgaaGGTCACGTACAGGG (SEQ ID NO. 96) |
| 005V1-4-7 | F: ACGCCACCCTGTACGTGAACTTCGAGCCCTGCGTGAT (SEQ ID NO. 97) |
| | R: TCACGTACAGGGTGGCGT (SEQ ID NO. 98) |
| 005V1-4-8 | F: ACCCTGTACGTGACCTTCCAGCCCTGCGTGATGTGCG (SEQ ID NO. 99) |
| | R: TCACGTACAGGGTGGCGT (SEQ ID NO. 100) |
| 005V1-5-1 | F: GAGACTGGTCTACGGCGTAAGCAACtcaAAAagaGGC (SEQ ID NO. 101) |
| | R: acGCCGTAGACCAGTCTC (SEQ ID NO. 102) |
| 005V1-5-3 | F: AGAGACTGGTCTACGGCGCGAGCAACtcaAAAagaGG (SEQ ID NO. 103) |
| | R: AGAGACTGGTCTACGGCg (SEQ ID NO. 104) |
| 005V1-5-5 | F: GACTGGTCTACGGCGTGATCAACtcaAAAagaGGCGC (SEQ ID NO. 105) |
| | R: TcacGCCGTAGACCAGTC (SEQ ID NO. 106) |
| 005V1-5-6 | F: CTGGTCTACGGCGTGAGCCACtcaAAAagaGGCGCCG (SEQ ID NO. 107) |
| | R: GCTcacGCCGTAGACCAG (SEQ ID NO. 108) |
| 005V1-5-7 | F: AGACTGGTCTACGGCGTGCGCAACtcaAAAagaGGCG (SEQ ID NO. 109) |
| | R: cacGCCGTAGACCAGTCT (SEQ ID NO. 110) |
| 005V1-6-1 | F: CTACGGCgtgAGCaacTCAAGGCGGGGAGCCGCCGGCAGCCTGCTGaa (SEQ ID NO. 111) |
| | R: TtgagttGCTcacGCCGTAG (SEQ ID NO. 112) |
| 005V1-6-2 | F: TGGTCTACGGCgtgAGCaacACTGGCCTTGCTGCCGCCGGCAGCCTGCTGaa (SEQ ID NO. 113) |
| | R: gttGCTcacGCCGTAGACCA (SEQ ID NO. 114) |
| 005V1-6-5 | F: GGTCTACGGCgtgAGCaacTACGAAGGAGGAGCCGCCGGCAGCCTGCTGaa (SEQ ID NO. 115) |
| | R: TtgagttGCTcacGCCGTAG (SEQ ID NO. 116) |
| 005V1-6-6 | F: TGGTCTACGGCgtgAGCaacGAATACATAATGGCCGCCGGCAGCCTGCTGaa (SEQ ID NO. 117) |
| | R: TtgagttGCTcacGCCGTAG (SEQ ID NO. 118) |
| 005V1-6-8 | F: TGGTCTACGGCgtgAGCaacAGTCTAAAGCGGGCCGCCGGCAGCCTGCTGaa (SEQ ID NO. 119) |
| | R: TtgagttGCTcacGCCGTAG (SEQ ID NO. 120) |
| 005V1-7-1 | F: ggctacggtctcaGaGGCGCCGCCGGCAGCCTGCTGACCGTGCTGaactacCCCGGC ATGAACCA (SEQ ID NO. 121) |
| | R: ggctacggtctcaCCtctTTTtgagttGCTcacGCCGTAGACCAGTCTC (SEQ ID NO. 122) |
| 005V1-7-2 | F: ggctacggtctcaGaGGCGCCGCCGGCAGCCTGCTGAACGTGCGGaactacCCCGG CATGAACCA (SEQ ID NO. 123) |
| | R: ggctacggtctcaCCtctTTTtgagttGCTcacGCCGTAGACCAGTCTC (SEQ ID NO. 124) |
| 005V1-7-3 | F: ggctacggtctcaGaGGCGCCGCCGGCAGCCTGCTGAACGGGCTGaactacCCCGG CATGAACCA (SEQ ID NO. 125) |
| | R: ggctacggtctcaCCtctTTTtgagttGCTcacGCCGTAGACCAGTCTC (SEQ ID NO. 126) |
| 005V1-8-1 | F: ggctacggtctcaCTGaacGTGCTGAACTCCCCCGGCATGAACCACCAGATCGAG |
| | AT (SEQ ID NO. 127) |
| | R: ggctacggtctcatCAGCAGGCTGCCGGCGGCGCCtctTTTtgagttGC (SEQ ID NO. 128) |
| 005V1-8-2 | F: ggctacggtctcaCTGaacGTGCTGGGCAGTAAATCCATGAACCACCAGATCGAG AT (SEQ ID NO. 129) |
| | R: ggctacggtctcatCAGCAGGCTGCCGGCGGCGCCtctTTTtgagttGC (SEQ ID NO. 130) |
| 005V1-8-3 | F: ggctacggtctcaCTGaacGTGCTGGACTACCCCGGCATGAACCACCAGATCGAG AT (SEQ ID NO. 131) |
| | R: ggctacggtctcatCAGCAGGCTGCCGGCGGCGCCtctTTTtgagttGC (SEQ ID NO. 132) |
| 005V1-8-4 | F: ggctacggtctcaCTGaacGTGCTGAAATATGCTACGATGAACCACCAGATCGAGA T (SEQ ID NO. 133) |
| | R: ggctacggtctcatCAGCAGGCTGCCGGCGGCGCCtctTTTtgagttGC (SEQ ID NO. 134) |
| 005V1-8-5 | F: ggctacggtctcaCTGaacGTGCTGGGCCTTCCGTTAATGAACCACCAGATCGAGA T (SEQ ID NO. 135) |
| | R: ggctacggtctcatCAGCAGGCTGCCGGCGGCGCCtctTTTtgagttGC (SEQ ID NO. 136) |
| 005V1-9-5 | F: ggctacggtctcaAGCGAGATGCTGTTTTCAGTCTTACAGCAGcctAGGGAAgtgtt (SEQ ID NO. 137) |
| | R: ggctacggtctcaCGCTGCACTCGTTGGCCATCACGCCGGCGGT (SEQ ID NO. 138) |
| 005V1-10-2 | F: ggctacggtctcatgcCAGTTCtatGGGGGGGTGAAGGAAgtgttcaatGCTGAGCG (SEQ ID NO. 139) |
| | R: ggctacggtctcaGgcaCAGCATCTCGCTGCAC (SEQ ID NO. 140) |

The applicant obtains the corresponding base editor variants through site-directed mutagenesis of deaminase 005V1-nCas9 by PCR, and a specific method is as follows.

As described above, the applicant designs two mutation primers near the mutation site and inserts a sequence to be mutated into the mutation primers, and the mutation primers used are shown in Tables 2 and 3. A full-length DNA sequence of a plasmid was amplified using 2 × Phanta Flash Master Mix enzyme (Vazyme, P520) (according to the instructions), and a residual original template was digested with DpnI enzyme (NEB, R1076L) after amplification. Then, a target fragment was end-digested and ligated using BsaI-HF^{®}v2 (NEB, R3733L) and T4 DNA ligase (NEB, M0202L). After conversion, the target fragment was sent to Boshang Biotechnology Co., Ltd. for sequencing. The sequencing results showed that different mutants of 005V1-nCas9 with correct sequences had been obtained.

### Example 2 Verification of Editing Activity of Base Editors Constructed by 005V1-nCas9 and Various Deaminase variants

### (1) A construction process of a sgRNA expression vector (sgRNA plasmid) is as follows.

PCSK9-sgRNA was designed for a PCSK9 target, site 1-sgRNA, site 8-sgRNA, site 16-sgRNA, and site 18-sgRNA were designed according to site 1, site 8, site 16, and site 18 targets (see Gaudelli NM, Komor AC, Rees HA, Packer MS, Badran AH, Bryson DI, Liu DR. Programmable base editing of A•T to G•C in genomic DNA without DNA cleavage. Nature. 2017 Nov 23;551(7681):464-471. doi: 10.1038/nature24644. Epub 2017 Oct 25. Erratum in: Nature. 2018 May 2;: PMID: 29160308; PMCID: PMC5726555.), and specific sgRNAs are shown in Table 4.

**Table 4**

| SgRNA name | Sequence | Target |
|---|---|---|
| PCSK9-sgRNA | cccgcaccttggcgcagcgg (SEQ ID NO. 141) | PCSK9 |
| site 1-sgRNA | gaacacaaagcatagactgc (SEQ ID NO. 142) | site 1 |
| site 8-sgRNA | taaacaaagcatagactga (SEQ ID NO. 143) | site 8 |
| site 16-sgRNA | ggaataaatcatagaatcc (SEQ ID NO. 144) | site 16 |
| site 18-sgRNA | acacacacacttagaatctg (SEQ ID NO. 145) | site 18 |

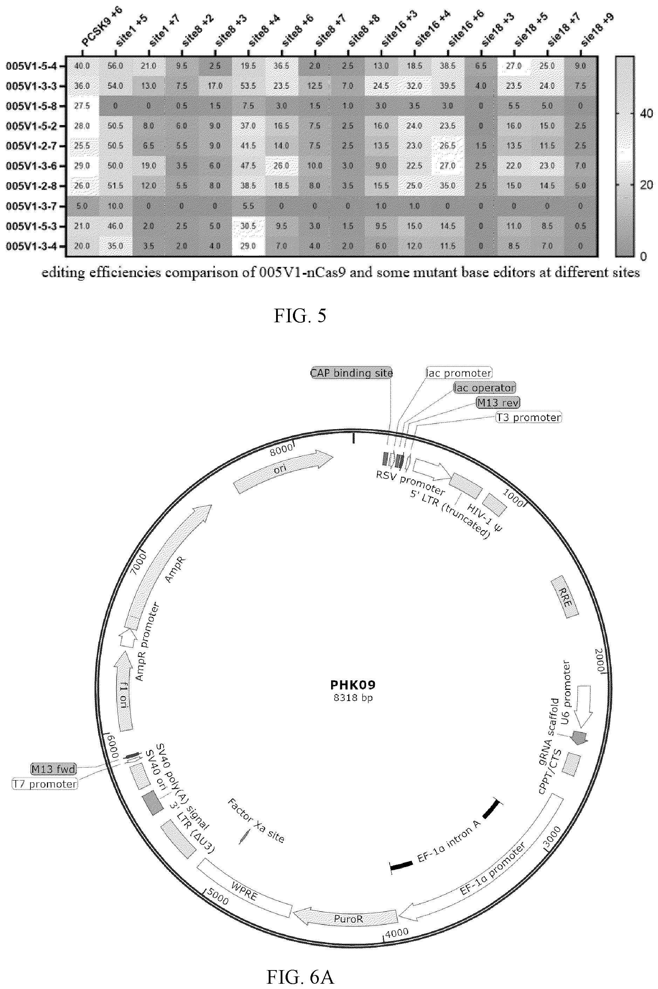

According to a target sequence, sgRNA was designed and oligonucleotides (oligos) were synthesized, and sgRNA sequences used are shown in SEQ ID NO: 141 to SEQ ID NO: 145. A CACC sequence was added to a 5' end of an upstream sequence of each sgRNA, and an AAAC sequence was added to a 5' end of a downstream sequence. Therefore, an upstream sequence form of each sgRNA used for synthesis was: 5'-CACCXXXXXXXXXXXXXXXXXXXX(20nt)-3', and a downstream sequence form was: 5'-AAACXXXXXXXXXXXXXXXXXXXX(20nt)-3'. After synthesis, the upstream and downstream sequences were annealed by a preset program (95°C, 5 min; 95°C to 85°C at -2°C/s; 85°C to 25°C at -0.1°C/s; maintained at 4°C), and annealed products were connected to a PHK09 vector (plasmid map is shown in FIG. 6A, which is owned by the laboratory and contains a backbone sequence of sgRNA) linearized by BsmBI (NEB: R0739L). A sequence of the PHK09 vector is shown below.

TGAGCTGATACCGCTCGCCGCAGCCGAACGACCGAGCGCAGCGAGTCAGTGAGCG

AGGAAGCGGAAGAGCGCCCAATACGCAAACCGCCTCTCCCCGCGCGTTGGCCGAT

TCATTAATGCAGCTGGCACGACAGGTTTCCCGACTGGAAAGCGGGCAGTGAGCGC

AACGCAATTAATGTGAGTTAGCTCACTCATTAGGCACCCCAGGCTTTACACTTTATG

CTTCCGGCTCGTATGTTGTGTGGAATTGTGAGCGGATAACAATTTCACACAGGAAA

CAGCTATGACCATGATTACGCCAAGCGCGCAATTAACCCTCACTAAAGGGAACAAA

AGCTGGAGCTGCAAGCTTAATGTAGTCTTATGCAATACTCTTGTAGTCTTGCAACAT

GGTAACGATGAGTTAGCAACATGCCTTACAAGGAGAGAAAAAGCACCGTGCATGC

CGATTGGTGGAAGTAAGGTGGTACGATCGTGCCTTATTAGGAAGGCAACAGACGGG

TCTGACATGGATTGGACGAACCACTGAATTGCCGCATTGCAGAGATATTGTATTTAA

GTGCCTAGCTCGATACATAAACGGGTCTCTCTGGTTAGACCAGATCTGAGCCTGGG

AGCTCTCTGGCTAACTAGGGAACCCACTGCTTAAGCCTCAATAAAGCTTGCCTTGA

GTGCTTCAAGTAGTGTGTGCCCGTCTGTTGTGTGACTCTGGTAACTAGAGATCCCTC

AGACCCTTTTAGTCAGTGTGGAAAATCTCTAGCAGTGGCGCCCGAACAGGGACTTG
AAAGCGAAAGGGAAACCAGAGGAGCTCTCTCGACGCAGGACTCGGCTTGCTGAA
GCGCGCACGGCAAGAGGCGAGGGGCGGCGACTGGTGAGTACGCCAAAAATTTTG
ACTAGCGGAGGCTAGAAGGAGAGAGATGGGTGCGAGAGCGTCAGTATTAAGCGGG
GGAGAATTAGATCGCGATGGGAAAAAATTCGGTTAAGGCCAGGGGGAAAGAAAAA
ATATAAATTAAAACATATAGTATGGGCAAGCAGGGAGCTAGAACGATTCGCAGTTAA
TCCTGGCCTGTTAGAAACATCAGAAGGCTGTAGACAAATACTGGGACAGCTACAAC
CATCCCTTCAGACAGGATCAGAAGAACTTAGATCATTATATAATACAGTAGCAACCC
TCTATTGTGTGCATCAAAGGATAGAGATAAAAGACACCAAGGAAGCTTTAGACAAG
ATAGAGGAAGAGCAAAACAAAAGTAAGACCACCGCACAGCAAGCGGCCGCTGAT
CTTCAGACCTGGAGGAGGAGATATGAGGGACAATTGGAGAAGTGAATTATATAAAT
ATAAAGTAGTAAAAATTGAACCATTAGGAGTAGCACCCACCAAGGCAAAGAGAAG
AGTGGTGCAGAGAGAAAAAAGAGCAGTGGGAATAGGAGCTTTGTTCCTTGGGTTC
TTGGGAGCAGCAGGAAGCACTATGGGCGCAGCGTCAATGACGCTGACGGTACAGG
CCAGACAATTATTGTCTGGTATAGTGCAGCAGCAGAACAATTTGCTGAGGGCTATTG
AGGCGCAACAGCATCTGTTGCAACTCACAGTCTGGGGCATCAAGCAGCTCCAGGC
AAGAATCCTGGCTGTGGAAAGATACCTAAAGGATCAACAGCTCCTGGGGATTTGGG
GTTGCTCTGGAAAACTCATTTGCACCACTGCTGTGCCTTGGAATGCTAGTTGGAGT
AATAAATCTCTGGAACAGATTTGGAATCACACGACCTGGATGGAGTGGGACAGAG
AAATTAACAATTACACAAGCTTAATACACTCCTTAATTGAAGAATCGCAAAACCAG
CAAGAAAAGAATGAACAAGAATTATTGGAATTAGATAAATGGGCAAGTTTGTGGAA
TTGGTTTAACATAACAAATTGGCTGTGGTATATAAAATTATTCATAATGATAGTAGGA
GGCTTGGTAGGTTTAAGAATAGTTTTTGCTGTACTTTCTATAGTGAATAGAGTTAGG
CAGGGATATTCACCATTATCGTTTCAGACCCACCTCCCAACCCCGAGGGGACCCAG

AGAGGGCCTATTTCCCATGATTCCTTCATATTTGCATATACGATACAAGGCTGTTAGA
GAGATAATTAGAATTAATTTGACTGTAAACACAAAGATATTAGTACAAAATACGTGA
CGTAGAAAGTAATAATTTCTTGGGTAGTTTGCAGTTTTAAAATTATGTTTTAAAATGG
ACTATCATATGCTTACCGTAACTTGAAAGTATTTCGATTTCTTGGCTTTATATATCTTG
TGGAAAGGACGAAACACCGGAGACGGACGTCTCTGTTTTAGAGCTAGAAATAGCA
AGTTAAAATAAGGCTAGTCCGTTATCAACTTGAAAAAGTGGCACCGAGTCGGTGCT
TTTTTAAGCTTGGCGTAACTAGATCTTGAGACAAATGGCAGTATTCATCCACAATTT
TAAAAGAAAAGGGGGGATTGGGGGGTACAGTGCAGGGGAAAGAATAGTAGACATA
ATAGCAACAGACATACAAACTAAAGAATTACAAAAACAAATTACAAAAATTCAAAA
TTTTCGGGTTTATTACAGGGACAGCAGAGATCCACTTTGGCGCCGGCTCGAGGGGG
CCCGGGTGCAAAGATGGATAAAGTTTTAAACAGAGAGGAATCTTTGCAGCTAATGG
ACCTTCTAGGTCTTGAAAGGAGTGGGAATTGGCTCCGGTGCCCGTCAGTGGGCAG
AGCGCACATCGCCCACAGTCCCCGAGAAGTTGGGGGGAGGGGTCGGCAATTGATC
CGGTGCCTAGAGAAGGTGGCGCGGGGTAAACTGGGAAAGTGATGTCGTGTACTGG
CTCCGCCTTTTTCCCGAGGGTGGGGGAGAACCGTATATAAGTGCAGTAGTCGCCGT
GAACGTTCTTTTTCGCAACGGGTTTGCCGCCAGAACACAGGTAAGTGCCGTGTGTG
GTTCCCGCGGGCCTGGCCTCTTTACGGGTTATGGCCCTTGCGTGCCTTGAATTACTT
CCACCTGGCTGCAGTACGTGATTCTTGATCCCGAGCTTCGGGTTGGAAGTGGGTGG
GAGAGTTCGAGGCCTTGCGCTTAAGGAGCCCCTTCGCCTCGTGCTTGAGTTGAGGC
CTGGCCTGGGCGCTGGGGCCGCCGCGTGCGAATCTGGTGGCACCTTCGCGCCTGTC
TCGCTGCTTTCGATAAGTCTCTAGCCATTTAAAATTTTTGATGACCTGCTGCGACGC
TTTTTTTCTGGCAAGATAGTCTTGTAAATGCGGGCCAAGATCTGCACACTGGTATTT
CGGTTTTTGGGGCCGCGGGCGGCGACGGGGCCCGTGCGTCCCAGCGCACATGTTC
GGCGAGGCGGGGCCTGCGAGCGCGGCCACCGAGAATCGGACGGGGGTAGTCTCA

AGCTGGCCGGCCTGCTCTGGTGCCTGGCCTCGCGCCGCCGTGTATCGCCCCGCCCT
GGGCGGCAAGGCTGGCCCGGTCGGCACCAGTTGCGTGAGCGGAAAGATGGCCGCT
TCCCGGCCCTGCTGCAGGGAGCTCAAAATGGAGGACGCGGCGCTCGGGAGAGCG
GGCGGGTGAGTCACCCACACAAAGGAAAAGGGCCTTTCCGTCCTCAGCCGTCGCT
TCATGTGACTCCACGGAGTACCGGGCGCCGTCCAGGCACCTCGATTAGTTCTCGAG
CTTTTGGAGTACGTCGTCTTTAGGTTGGGGGGAGGGGTTTTATGCGATGGAGTTTCC
CCACACTGAGTGGGTGGAGACTGAAGTTAGGCCAGCTTGGCACTTGATGTAATTCT
CCTTGGAATTTGCCCTTTTTGAGTTTGGATCTTGGTTCATTCTCAAGCCTCAGACAG
TGGTTCAAAGTTTTTTTCTTCCATTTCAGGTGTCGTGACGTACGGCCACCATGACCG
AGTACAAGCCCACGGTGCGCCTCGCCACCCGCGACGACGTCCCCAGGGCCGTACG
CACCCTCGCCGCCGCGTTCGCCGACTACCCCGCCACGCGCCACACCGTCGATCCGG
ACCGCCACATCGAGCGGGTCACCGAGCTGCAAGAACTCTTCCTCACGCGCGTCGG
GCTCGACATCGGCAAGGTGTGGGTCGCGGACGACGGCGCCGCCGTGGCGGTCTGG
ACCACGCCGGAGAGCGTCGAAGCGGGGGCGGTGTTCGCCGAGATCGGCCCGCGC
ATGGCCGAGTTGAGCGGTTCCCGGCTGGCCGCGCAGCAACAGATGGAAGGCCTCC
TGGCGCCGCACCGGCCCAAGGAGCCCGCGTGGTTCCTGGCCACCGTCGGAGTCTC
GCCCGACCACCAGGGCAAGGGTCTGGGCAGCGCCGTCGTGCTCCCCGGAGTGGAG
GCGGCCGAGCGCGCCGGGGTGCCCGCCTTCCTGGAGACCTCCGCGCCCCGCAACC
TCCCCTTCTACGAGCGGCTCGGCTTCACCGTCACCGCCGACGTCGAGGTGCCCGAA
GGACCGCGCACCTGGTGCATGACCCGCAAGCCCGGTGCCTGAACGCGTTAAGTCG
ACAATCAACCTCTGGATTACAAAATTTGTGAAAGATTGACTGGTATTCTTAACTATG
TTGCTCCTTTTACGCTATGTGGATACGCTGCTTTAATGCCTTTGTATCATGCTATTGCT
TCCCGTATGGCTTTCATTTTCTCCTCCTTGTATAAATCCTGGTTGCTGTCTCTTTATGA
GGAGTTGTGGCCCGTTGTCAGGCAACGTGGCGTGGTGTGCACTGTGTTTGCTGAC

GCAACCCCCACTGGTTGGGGCATTGCCACCACCTGTCAGCTCCTTTCCGGGACTTT
CGCTTTCCCCCTCCCTATTGCCACGGCGGAACTCATCGCCGCCTGCCTTGCCCGCTG
CTGGACAGGGGCTCGGCTGTTGGGCACTGACAATTCCGTGGTGTTGTCGGGGAAA
TCATCGTCCTTTCCTTGGCTGCTCGCCTGTGTTGCCACCTGGATTCTGCGCGGGACG
TCCTTCTGCTACGTCCCTTCGGCCCTCAATCCAGCGGACCTTCCTTCCCGCGGCCTG
CTGCCGGCTCTGCGGCCTCTTCCGCGTCTTCGCCTTCGCCCTCAGACGAGTCGGAT
CTCCCTTTGGGCCGCCTCCCCGCGTCGACTTTAAGACCAATGACTTACAAGGCAGC
TGTAGATCTTAGCCACTTTTTAAAAGAAAAGGGGGGACTGGAAGGGCTAATTCACT
CCCAACGAAGACAAGATCTGCTTTTTGCTTGTACTGGGTCTCTCTGGTTAGACCAG
ATCTGAGCCTGGGAGCTCTCTGGCTAACTAGGGAACCCACTGCTTAAGCCTCAATA
AAGCTTGCCTTGAGTGCTTCAAGTAGTGTGTGCCCGTCTGTTGTGTGACTCTGGTA
ACTAGAGATCCCTCAGACCCTTTTAGTCAGTGTGGAAAATCTCTAGCAGTACGTATA
GTAGTTCATGTCATCTTATTATTCAGTATTTATAACTTGCAAAGAAATGAATATCAGA
GAGTGAGAGGAACTTGTTTATTGCAGCTTATAATGGTTACAAATAAAGCAATAGCAT
CACAAATTTCACAAATAAAGCATTTTTTTCACTGCATTCTAGTTGTGGTTTGTCCAA
ACTCATCAATGTATCTTATCATGTCTGGCTCTAGCTATCCCGCCCCTAACTCCGCCCA
TCCCGCCCCTAACTCCGCCCAGTTCCGCCCATTCTCCGCCCCATGGCTGACTAATTT
TTTTTATTTATGCAGAGGCCGAGGCCGCCTCGGCCTCTGAGCTATTCCAGAAGTAGT
GAGGAGGCTTTTTTGGAGGCCTAGGGACGTACCCAATTCGCCCTATAGTGAGTCGT
ATTACGCGCGCTCACTGGCCGTCGTTTTACAACGTCGTGACTGGGAAAACCCTGGC
GTTACCCAACTTAATCGCCTTGCAGCACATCCCCCTTTCGCCAGCTGGCGTAATAGC
GAAGAGGCCCGCACCGATCGCCCTTCCCAACAGTTGCGCAGCCTGAATGGCGAAT
GGGACGCGCCCTGTAGCGGCGCATTAAGCGCGGCGGGTGTGGTGGTTACGCGCAG
CGTGACCGCTACACTTGCCAGCGCCCTAGCGCCCGCTCCTTTCGCTTTCTTCCCTTC

CTTTCTCGCCACGTTCGCCGGCTTTCCCCGTCAAGCTCTAAATCGGGGGCTCCCTTT
AGGGTTCCGATTTAGTGCTTTACGGCACCTCGACCCCAAAAAACTTGATTAGGGTG
ATGGTTCACGTAGTGGGCCATCGCCCTGATAGACGGTTTTTCGCCCTTTGACGTTGG
AGTCCACGTTCTTTAATAGTGGACTCTTGTTCCAAACTGGAACAACACTCAACCCT
ATCTCGGTCTATTCTTTTGATTTATAAGGGATTTTGCCGATTTCGGCCTATTGGTTAA
AAAATGAGCTGATTTAACAAAAATTTAACGCGAATTTTAACAAAATATTAACGCTTA
CAATTTAGGTGGCACTTTTCGGGGAAATGTGCGCGGAACCCCTATTTGTTTATTTTT
CTAAATACATTCAAATATGTATCCGCTCATGAGACAATAACCCTGATAAATGCTTCAA
TAATATTGAAAAAGGAAGAGTATGAGTATTCAACATTTCCGTGTCGCCCTTATTCCC
TTTTTTGCGGCATTTTGCCTTCCTGTTTTTGCTCACCCAGAAACGCTGGTGAAAGTA
AAAGATGCTGAAGATCAGTTGGGTGCACGAGTGGGTTACATCGAACTGGATCTCAA
CAGCGGTAAGATCCTTGAGAGTTTTCGCCCCGAAGAACGTTTTCCAATGATGAGCA
CTTTTAAAGTTCTGCTATGTGGCGCGGTATTATCCCGTATTGACGCCGGGCAAGAGC
AACTCGGTCGCCGCATACACTATTCTCAGAATGACTTGGTTGAGTACTCACCAGTC
ACAGAAAAGCATCTTACGGATGGCATGACAGTAAGAGAATTATGCAGTGCTGCCAT
AACCATGAGTGATAACACTGCGGCCAACTTACTTCTGACAACGATCGGAGGACCGA
AGGAGCTAACCGCTTTTTTGCACAACATGGGGGATCATGTAACTCGCCTTGATCGTT
GGGAACCGGAGCTGAATGAAGCCATACCAAACGACGAGCGTGACACCACGATGCC
TGTAGCAATGGCAACAACGTTGCGCAAACTATTAACTGGCGAACTACTTACTCTAG
CTTCCCGGCAACAATTAATAGACTGGATGGAGGCGGATAAAGTTGCAGGACCACTT
CTGCGCTCGGCCCTTCCGGCTGGCTGGTTTATTGCTGATAAATCTGGAGCCGGTGA
GCGTGGGTCTCGCGGTATCATTGCAGCACTGGGGCCAGATGGTAAGCCCTCCCGTA
TCGTAGTTATCTACACGACGGGGAGTCAGGCAACTATGGATGAACGAAATAGACAG
ATCGCTGAGATAGGTGCCTCACTGATTAAGCATTGGTAACTGTCAGACCAAGTTTAC

TCATATATACTTTAGATTGATTTAAAACTTCATTTTTAATTTAAAAGGATCTAGGTGAA
GATCCTTTTTGATAATCTCATGACCAAAATCCCTTAACGTGAGTTTTCGTTCCACTG
AGCGTCAGACCCCGTAGAAAAGATCAAAGGATCTTCTTGAGATCCTTTTTTTCTGC
GCGTAATCTGCTGCTTGCAAACAAAAAAACCACCGCTACCAGCGGTGGTTTGTTTG
CCGGATCAAGAGCTACCAACTCTTTTTCCGAAGGTAACTGGCTTCAGCAGAGCGCA
GATACCAAATACTGTTCTTCTAGTGTAGCCGTAGTTAGGCCACCACTTCAAGAACTC
TGTAGCACCGCCTACATACCTCGCTCT
GCTAATCCTGTTACCAGTGGCTGCTGCCAGTGGCGATAAGTCGTGTCTTACCGGGTT
GGACTCAAGACGATAGTTACCGGATAAGGCGCAGCGGTCGGGCTGAACGGGGGGT
TCGTGCACACAGCCCAGCTTGGAGCGAACGACCTACACCGAACTGAGATACCTAC
AGCGTGAGCTATGAGAAAGCGCCACGCTTCCCGAAGGGAGAAAGGCGGACAGGT
ATCCGGTAAGCGGCAGGGTCGGAACAGGAGAGCGCACGAGGGAGCTTCCAGGGG
GAAACGCCTGGTATCTTTATAGTCCTGTCGGGTTTCGCCACCTCTGACTTGAGCGTC
GATTTTTGTGATGCTCGTCAGGGGGGCGGAGCCTATGGAAAAACGCCAGCAACGC
GGCCTTTTTACGGTTCCTGGCCTTTTGCTGGCCTTTTGCTCACATGTTCTTTCCTGCG
TTATCCCCTGATTCTGTGGATAACCGTATTACCGCCTTTGAG (SEQ ID NO: 168).

Systems used in the construction of the sgRNA plasmid are as follows.

A linearization system of the PHK09 vector is as follows: 3 µg of PHK09 vector, 6 µL of buffer (NEB: R0539L), 2 µL of BsmBI, ddH₂O made up to 60 µL, and enzyme digestion carried out at 37°C overnight.

A connection system of the sgRNA annealing product and the linearized vector is as follows: 1 µL of T4 ligase buffer (NEB: M0202L), 20 ng of linearized vector, 5 µL of annealed oligo fragment (10 µM), 0.5 µL of T4 ligase (NEB: M0202L), ddH₂O made up to 10 µL, and connection performed at 16°C overnight.

The connected vector was converted into E. coli DH5a competent cells (Weidi Biotechnology Co., Ltd., DL1001). A specific procedure is as follows: the DH5α competent cells were taken out from -80°C and were quickly inserted into ice. After 5 minutes, a bacterial block was melted, a connected product was added and gently mixed well by hand at a bottom of a centrifuge tube, and then allowed to stand for 25 minutes in ice. The heat shock was performed in a water bath at 42°C for 45 seconds, and the mixture was quickly placed back to ice and allowed to stand for 2 minutes. 700 µl of sterile LB medium without antibiotics was added to the centrifuge tube, mixed well, and recovered at 37°C, 200 rpm for 60 minutes. The cells were collected by centrifugation at 5000 rpm for one minute. About 100 µl of a supernatant was taken and gently blown to resuspend a bacterial block and spread on an LB medium containing Amp antibiotics. A plate was placed upside down in a 37°C incubator for overnight culture. Single colonies were picked, and after sequencing confirmation, positive clones were shaken and a plasmid was extracted (TIANGEN, DP120-01), and a concentration was measured. The plasmid was stored in a -20°C refrigerator for later use.

### (2) Cell Culture and Transfection

HEK293T cells (purchased from ATCC) were inoculated in a DMEM medium (Gibco, 11965092) supplemented with 10% FBS (v/v), containing 1% Penicillin Streptomycin (v/v) (Gibco, 15140122), and cultured in a 37°C cell culture incubator containing 5% CO₂. The cells used for transfection were inoculated in a 24-well cell culture plate the day before and cultured. The cells were observed the next day and transfected when the cells grew to a cell density of about 80%. The amount of plasmids transfected in each well of the 24-well plate was 0.4 µg of 005V1-nCas9 plasmid and each 005V1 mutant-nCas9, and 0.4 µg of sgRNA plasmid.

The plasmids were mixed, and then diluted with 25 µl of reduced serum medium (Basal Media, L530KJ), 2 µl of p3000 reagent was added, the mixture was blown and mixed well as a reagent A, and allowed to stand for 5 minutes. At the same time, 2 µl of Lipofectamine 3000 transfection reagent (Thermo, 11668019) was diluted with 25 µl of reduced serum medium and mixed well as a reagent B, and allowed to stand for 5 minutes. The above reagent A and reagent B were mixed and blown evenly, and allowed to stand for 20 minutes. After the standing was finished, the mixed reagent was added dropwise to the cells of the 24-well plate to be transfected and placed back to the 37°C incubator for culture. After 6 hours of transfection, the culture medium was replaced with a DMEM medium containing 10% FBS. After 48 hours of transfection, the cells were collected for editing efficiency detection.

(3) The collected cells were subjected to genomic extraction (TIANGEN, DP304-03), and primers were designed according to experimental requirements, and identification primer sequences used are shown in Table 5.

**Table 5 Design of identification primer for each target**

| Primer names | Sequence | Target |
|---|---|---|
| PCSK9-forward primer | gctagccttgcgttccg (SEQ ID NO. 146) | PCSK9 |
| PCSK9-reverse primer | gtccccaagatcgtgccaa (SEQ ID NO. 147) | |
| site 1-forward primer | cctcagcattcagccactaa (SEQ ID NO. 148) | site 1 |
| site 1-reverse primer | agaggcccattaacgtttgg (SEQ ID NO. 149) | |
| site 8-forward primer | ttccttctacggcagaaacc (SEQ ID NO. 150) | site 8 |
| site 8-reverse primer | aacaagtagcaacaggaggg (SEQ ID NO. 151) | |
| site 16-forward primer | gggaacaaaccaggcataca (SEQ ID NO. 152) | site 16 |
| site 16-reverse primer | cctcaaggaacaacctgtcc (SEQ ID NO. 153) | |
| site 18-forward primer | gagaggctgccaagctaaat (SEQ ID NO. 154) | site 18 |
| site 18-reverse primer | tggagctcaagatcacgttg (SEQ ID NO. 155) | |

A genome was used as a template, PCR amplification was performed on a sequence near a target, and an amplified PCR product was used for high-throughput deep sequencing (GENEWIZ, Inc) or Sanger sequencing (Boshang Biotechnology (Shanghai) Co., Ltd.) to identify the editing efficiency. A system used for target site sequence amplification is as follows: 25 µL of 2 × Taq Master Mix (Vazyme, P112-03), 1 µL of Primer-F (10 pmol/µL), 1 µL of Primer-R (10 pmol/µL), 1 µL of template, and ddH₂O made up to 50 µL.

### Gene editing effect detection:

For a calculation method of gene editing efficiency, see Kluesner MG, Nedveck DA, Lahr WS, Garbe JR, Abrahante JE, Webber BR, Moriarity BS. EditR: A Method to Quantify Base Editing from Sanger Sequencing. CRISPR J. 2018 Jun;1(3):239-250. doi: 10.1089/crispr.2018.0014. PMID: 31021262; PMCID: PMC6694769.

In this example, a structure of a base editor including the adenosine deaminase provided by the present invention and the nCas9 is as follows:
NH₂-[NLS]-[adenosine deaminase]-linker-[nCas9]-[NLS]-COOH. However, this structure is only used for example and is not used to limit the structure of the base editor. Examples of this structure can be found in SEQ ID NO: 3 and SEQ ID NO: 190.

This example counts an editing efficiency of each base editor on the PCSK9 target (efficiency of mutation of adenine A to guanine G), and measures and compares the editing efficiencies of the base editors. The editing efficiency of the base editor constituted by 005V1-nCas9 and each deaminase variant is shown in Tables 6 to 9.

**Table 6**

| Base editor | Editing efficiency (three replicates) | Average editing efficiency | Ratio to editing efficiency of 005V1-nCas9 |
|---|---|---|---|
| 005V1-nCas9 | 19%, 18%, 21% | 19.33% | 1.00 |
| 005V1-1-1-nCas9 | 0%, 2%, 0% | 0.67% | 0.03 |
| 005V1-1-2-nCas9 | 0%, 1%, 0% | 0.33% | 0.02 |
| 005V1-1-3-nCas9 | 0%, 1%, 2% | 1% | 0.05 |
| 005V1-1-4-nCas9 | 0%, 2%, 0% | 0.67% | 0.03 |
| 005V1-1-5-nCas9 | 0%, 2%, 0% | 0.67% | 0.03 |
| 005V1-1-6-nCas9 | 0%, 0%, 0% | 0% | 0.00 |
| 005V1-1-7-nCas9 | 0%, 0%, 0% | 0% | 0.00 |
| 005V1-1-8-nCas9 | 3%, 0%, 0% | 1% | 0.05 |
| 005V1-2-1-nCas9 | 0%, 3%, 0% | 1% | 0.05 |
| 005V1-2-2-nCas9 | 0%, 1%, 0% | 0.33% | 0.02 |
| 005V1-2-3-nCas9 | 0%, 2%, 0% | 0.67% | 0.03 |
| 005V1-2-5-nCas9 | 1%, 0%, 0% | 0.33% | 0.02 |
| 005V1-2-6-nCas9 | 0%, 0%, 0% | 0% | 0.00 |

**Table 7**

| Base editor | Editing efficiency (three replicates) | Average editing efficiency | Ratio to editing efficiency of 005V1-nCas9 |
|---|---|---|---|
| 005V1-2-7-nCas9 | 22%, 17%, 23% | 20.67% | 1.07 |
| 005V1-2-8-nCas9 | 19%, 16%, 22% | 19% | 0.98 |
| 005V1-3-1-nCas9 | 5%, 2%, 1% | 2.67% | 0.14 |
| 005V1-3-2-nCas9 | 0%, 2%, 1% | 1% | 0.05 |
| 005V1-3-3-nCas9 | 32%, 30%, 31% | 31% | 1.60 |
| 005V1-3-4-nCas9 | 13%, 15%, 17% | 15% | 0.78 |
| 005V1-3-5-nCas9 | 0%, 1%, 1% | 0.67% | 0.03 |
| 005V1-3-6-nCas9 | 21%, 20%, 22% | 21% | 1.09 |
| 005V1-3-7-nCas9 | 17%, 16%, 17% | 16.67% | 0.86 |
| 005V1-3-8-nCas9 | 4%, 3%, 2% | 3% | 0.16 |
| 005V1-4-1-nCas9 | 2%, 2%, 0% | 1.33% | 0.07 |
| 005v1-4-2-nCas9 | 0%, 2%, 0% | 0.67% | 0.03 |
| 005V1-4-3-nCas9 | 0%, 4%, 0% | 1.33% | 0.07 |
| 005V1-4-5-nCas9 | 0%, 0%, 0% | 0% | 0.00 |
| 005V1-4-7-nCas9 | 1%, 0%, 0% | 0.33% | 0.02 |

**Table 8**

| Base editor | Editing efficiency (three replicates) | Average editing efficiency | Ratio to editing efficiency of 005V1-nCas9 |
|---|---|---|---|
| 005V1-4-8-nCas9 | 1%, 0%, 0% | 0.33% | 0.02 |
| 005V1-5-1-nCas9 | 0%, 3%, 0% | 1% | 0.05 |
| 005V1-5-2-nCas9 | 23%, 19%, 23% | 21.67% | 1.12 |
| 005V1-5-3-nCas9 | 16%, 17%, 15% | 16% | 0.83 |
| 005V1-5-4-nCas9 | 36%, 21%, 33% | 30% | 1.55 |
| 005V1-5-5-nCas9 | 0%, 0%, 0% | 0% | 0.00 |
| 005V1-5-6-nCas9 | 0%, 0%, 0% | 0% | 0.00 |
| 005V1-5-7-nCas9 | 2%, 0%, 0% | 0.67% | 0.03 |
| 005V1-5-8-nCas9 | 27%, 22%, 31% | 26.67% | 1.38 |
| 005V1-6-1-nCas9 | 0%, 3%, 1% | 1.33% | 0.07 |
| 005V1-6-2-nCas9 | 0%, 2%, 0% | 0.67% | 0.03 |
| 005V1-6-5-nCas9 | 0%, 0%, 0% | 0% | 0.00 |
| 005V1-6-6-nCas9 | 0%, 0%, 0% | 0% | 0.00 |
| 005V1-6-8-nCas9 | 0%, 0%, 0% | 0% | 0.00 |
| 005V1-7-1-nCas9 | 0%, 0%, 0% | 0% | 0.00 |
| 005V1-7-2-nCas9 | 0%, 0%, 0% | 0% | 0.00 |
| 005V1-7-3-nCas9 | 0%, 2%, 0% | 0.67% | 0.03 |
| 005V1-8-1-nCas9 | 8%, 7%, 7% | 7.33% | 0.38 |
| 005V1-8-2-nCas9 | 3%, 2%, 1% | 2% | 0.10 |
| 005V1-8-3-nCas9 | 18%, 16%, 16% | 16.67% | 0.86 |
| 005V1-8-4-nCas9 | 2%, 1%, 1% | 1.33% | 0.07 |
| 005V1-8-5-nCas9 | 14%, 11%, 13% | 12.67% | 0.66 |

**Table 9**

| Base editor | Editing efficiency (three replicates) | Average editing efficiency | Ratio to editing efficiency of 005V1-nCas9 |
|---|---|---|---|
| 005V1-9-1-nCas9 | 10%, 17%, 13% | 13.33% | 0.69 |
| 005V1-9-2-nCas9 | 4%, 5%, 3% | 4% | 0.21 |
| 005V1-9-3-nCas9 | 0%, 1%, 1% | 0.67% | 0.03 |
| 005V1-9-4-nCas9 | 16%, 11%, 15% | 14% | 0.72 |
| 005V1-9-5-nCas9 | 1%, 1%, 0% | 0.67% | 0.03 |
| 005V1-10-1-nCas9 | 41%, 34%, 31% | 35.33% | 1.83 |
| 005V1-10-2-nCas9 | 22%, 12%, 13% | 15.67% | 0.81 |
| 005V1-10-3-nCas9 | 57%, 42%, 41% | 46.67% | 2.41 |
| 005V1-10-4-nCas9 | 32%, 36%, 33% | 33.67% | 1.74 |
| 005V1-10-5-nCas9 | 23%, 28%, 21% | 24% | 1.24 |
| 005V1-11-2-nCas9 | 39%, 35%, 33% | 35.67% | 1.85 |
| 005V1-11-3-nCas9 | 33%, 33%, 35% | 33.67% | 1.74 |
| 005V1-11-4-nCas9 | 32%, 36%, 30% | 32.67% | 1.69 |
| 005V1-11-5-nCas9 | 36%, 32%, 32% | 33.33% | 1.72 |
| 005V1-15-1-nCas9 | 33%, 33%, 35% | 33.67% | 1.74 |
| 005V1-15-2-nCas9 | 31%, 31%, 33% | 31.67% | 1.64 |
| 005V1-15-5-nCas9 | 29%, 29%, 28% | 28.67% | 1.48 |

The editing efficiency and comparison of the editor constituted by 005V1-nCas9 and each mutant are shown in FIGS. 2 to 5.

FIG. 2 shows editing efficiencies of the 005V1-nCas9 and each 005V1 mutant-nCas9 at a PCSK9 site from A·T to G·C, an editing position is an adenine deoxynucleotide +6 from a 5' end of sgRNA, an error line represents mean ± SEM, and there are 3 to 5 biological replicates for each group of samples.

As shown in FIG. 3, the editing efficiencies of 005V1-10-3-nCas9, 005V1-11-2-nCas9, 005V1-10-1-nCas9, 005V1-10-4-nCas9, 005V1-11-3-nCas9, 005V1-15-1-nCas9, 005V1-11-5-nCas9, 005V1-11-4-nCas9, 005V1-15-2-nCas9, 005V1-3-3-nCas9, 005V1-5-4-nCas9, 005V1-15-5-nCas9, 005V1-5-8-nCas9, 005V1-10-5-nCas9, 005V1-5-2-nCas9, 005V1-3-6-nCas9, and 005V1-2-7-nCas9 at the PCSK9 site are significantly improved, and an average editing efficiency is above 20%, which is 1.07 to 2.41 times higher than that of the original 005V1-nCas9.

As shown in FIG. 4, among the mutants with significantly improved editing efficiency at the PCSK9 site, the editing efficiency of the 005V1-10-3-nCas9 can reach 46.67%. The editing efficiencies of the 005V1-11-2-nCas9 and the 005V1-10-1-nCas9 also reach 35.67% and 35.33%, respectively. The editing efficiencies of the base editors constituted by most other mutants in the figure are also higher than that of the 005V1-nCas9.

As shown in FIG. 5, the editing windows of the 005V1-5-4-nCas9, 005V1-3-3-nCas9, 005V1-5-2-nCas9, 005V1-2-7-nCas9, 005V1-3-6-nCas9, 005V1-2-8-nCas9, 005V1-5-3-nCas9, and 005V1-3-4-nCas9 at the site 1 (+5 position, +7 position), the site 8 (+2 position, +3 position, +4 position, +6 position) and the site 16 (+3 position, +4 position, +6 position) are significantly narrowed. While the editing window of the above base editor at the site 1 is narrowed, the adenine deoxynucleotide at the +5 position of the 5' end of the sgRNA in the window still maintains extremely high editing activity, which can achieve efficient and accurate editing and has great potential application value.

### Example 3

In this example, the adenosine deaminase 004V1 was used, and was subjected to fusion expression with nCas9 to form a new adenine base editor 004V1-nCas9 with a narrower editing window.

In this example, a construction strategy of the adenine base editor 004V1-nCas9 is to replace the adenosine deaminase in ABE8e with 004V1, thereby obtaining a new adenine base editor 004V1-nCas9.

In the following example, a structure of the base editor including the adenosine deaminase provided by the present invention and the nCas9 is as follows:
NH₂-[NLS]-[adenosine deaminase]-linker-[napDNAbp]-[NLS]-COOH. However, this structure is only used for example and is not used to limit the structure of the base editor.

In the following example, the sgRNA used is shown in Table 10, and identification primer sequences used are shown in Table 11.

**Table 10**

| sgRNA name | Sequence | Target |
|---|---|---|
| site 1-sgRNA | Gaacacaaagcatagactgc (SEQ ID NO: 142) | site 1 |
| site 17-sgRNA | Acaaagaggaagagagacg (SEQ ID NO: 169) | site 17 |
| site 18-sgRNA | Acacacacacttagaatctg (SEQ ID NO: 145) | site 18 |

**Table 11**

| Primer name | Sequence | Target |
|---|---|---|
| site 1-forward primer | Cctcagcattcagccactaa (SEQ ID NO: 148) | site 1 |
| site 1-reverse primer | Agaggcccattaacgtttgg (SEQ ID NO: 149) | |
| site 17-forward primer | Ctggctgaccaagagtgaag (SEQ ID NO: 166) | site 17 |
| site 17-reverse primer | Aatgagcctctggtggagat (SEQ ID NO: 167) | |
| site 18-forward primer | Gagaggctgccaagctaaat (SEQ ID NO: 154) | site 18 |
| site 18-reverse primer | Tggagctcaagatcacgttg (SEQ ID NO: 155) | |

The specific construction of the sgRNA and the base editor is as follows.
1. A construction method of a sgRNA expression vector (sgRNA plasmid) is the same as that in Example 2.
2. Construction of Adenine Base Editor 004V1-nCas9 Expression Vector (004V1-nCas9 Plasmid)

In this example, adenine base editor expression vector 004V1-nCas9 was prepared. A nucleotide sequence of the adenosine deaminase 004V1 is shown as SEQ ID NO: 156.

The nucleotide sequence of the 004V1 is as follows:

atgtacaatgcccctagattcttttgtagatctgctgccgtgtctgatcatgagtttaacgatgagtactggatgagacacgccctga

cactggccaagagggctcgggaggaaggagaggtgccagtgggagctgtgctggtgctgaataatcaggtcattggagaaggatgg

aatagagccatcggcctgcacgaccccaccgcccacgccgagatcatggccctgagacagggcggcctggtgctgcagaactaccg

gctgatcgacgccaccctgtacgtgaccttcgagccctgcgtgatgtgcgccggcgccatggtgcatagcagaatcagcagactggtg

ttcggcgtgagaaactcaaagagaggcgccgccggcagcctgatcaacgtgctgaactaccccggcatgaaccacagagtggagat

caccgagggcatcctggccgagagctgcagcgccatgctgtgcgacttctataggtggcctagggaggtgttcaatgccctgaagaag

gccagacaggaggagggc (SEQ ID NO: 156)

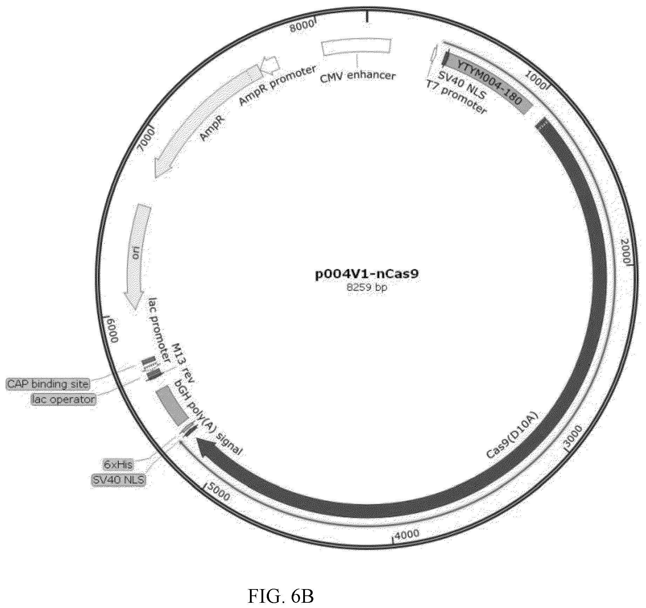

The above nucleotide sequence of the deaminase 004V1 has been codon-optimized according to the preference of human codon usage. Sangon Biotech (Shanghai) Co., Ltd. was commissioned to complete artificial synthesis of the 540bp deaminase 004V1 gene and replace nucleotides 63 to 560 of an ABE8e sequence with the synthesized gene. A plasmid map of 004V1-nCas9 is shown in FIG. 6B. (Correspondingly, an amino acid sequence of the 004V1-nCas9 is shown in SEQ ID NO: 190.)

A nucleotide sequence of the 004V1-nCas9 is as follows:

atgaaacggacagccgacggaagcgagttcgagtcaccaaagaagaagcggaaagtcatgtacaatgcccctagattcttttgt

agatctgctgccgtgtctgatcatgagtttaacgatgagtactggatgagacacgccctgacactggccaagagggctcgggaggaag

gagaggtgccagtgggagctgtgctggtgctgaataatcaggtcattggagaaggatggaatagagccatcggcctgcacgacccca

ccgcccacgccgagatcatggccctgagacagggcggcctggtgctgcagaactaccggctgatcgacgccaccctgtacgtgacct

tcgagccctgcgtgatgtgcgccggcgccatggtgcatagcagaatcagcagactggtgttcggcgtgagaaactcaaagagaggcg

ccgccggcagcctgatcaacgtgctgaactaccccggcatgaaccacagagtggagatcaccgagggcatcctggccgagagctgc

agcgccatgctgtgcgacttctataggtggcctagggaggtgttcaatgccctgaagaaggccagacaggaggagggctccggagga

tctagcggaggctcctctggctctgagacacctggcacaagcgagagcgcaacacctgaaagcagcgggggcagcagcggggggt

cagacaagaagtacagcatcggcctggccatcggcaccaactctgtgggctgggccgtgatcaccgacgagtacaaggtgcccagc
aagaaattcaaggtgctgggcaacaccgaccggcacagcatcaagaagaacctgatcggagccctgctgttcgacagcggcgaaac
agccgaggccacccggctgaagagaaccgccagaagaagatacaccagacggaagaaccggatctgctatctgcaagagatcttca
gcaacgagatggccaaggtggacgacagcttcttccacagactggaagagtccttcctggtggaagaggataagaagcacgagcgg
caccccatcttcggcaacatcgtggacgaggtggcctaccacgagaagtaccccaccatctaccacctgagaaagaaactggtggaca
gcaccgacaaggccgacctgcggctgatctatctggccctggcccacatgatcaagttccggggccacttcctgatcgagggcgacct
gaaccccgacaacagcgacgtggacaagctgttcatccagctggtgcagacctacaaccagctgttcgaggaaaaccccatcaacgc
cagcggcgtggacgccaaggccatcctgtctgccagactgagcaagagcagacggctggaaaatctgatcgcccagctgcccggcg
agaagaagaatggcctgttcggaaacctgattgccctgagcctgggcctgacccccaacttcaagagcaacttcgacctggccgagga
tgccaaactgcagctgagcaaggacacctacgacgacgacctggacaacctgctggcccagatcggcgaccagtacgccgacctgtt
tctggccgccaagaacctgtccgacgccatcctgctgagcgacatcctgagagtgaacaccgagatcaccaaggcccccctgagcgc
ctctatgatcaagagatacgacgagcaccaccaggacctgaccctgctgaaagctctcgtgcggcagcagctgcctgagaagtacaaa
gagattttcttcgaccagagcaagaacggctacgccggctacattgacggcggagccagccaggaagagttctacaagttcatcaagc
ccatcctggaaaagatggacggcaccgaggaactgctcgtgaagctgaacagagaggacctgctgcggaagcagcggaccttcgac
aacggcagcatcccccaccagatccacctgggagagctgcacgccattctgcggcggcaggaagatttttacccattcctgaaggaca
accgggaaaagatcgagaagatcctgaccttccgcatcccctactacgtgggccctctggccaggggaaacagcagattcgcctggat
gaccagaaagagcgaggaaaccatcaccccctggaacttcgaggaagtggtggacaagggcgcttccgcccagagcttcatcgagc
ggatgaccaacttcgataagaacctgcccaacgagaaggtgctgcccaagcacagcctgctgtacgagtacttcaccgtgtataacga
gctgaccaaagtgaaatacgtgaccgagggaatgagaaagcccgccttcctgagcggcgagcagaaaaaggccatcgtggacctgc
tgttcaagaccaaccggaaagtgaccgtgaagcagctgaaagaggactacttcaagaaaatcgagtgcttcgactccgtggaaatctcc
ggcgtggaagatcggttcaacgcctccctgggcacataccacgatctgctgaaaattatcaaggacaaggacttcctggacaatgagga
aaacgaggacattctggaagatatcgtgctgaccctgacactgtttgaggacagagagatgatcgaggaacggctgaaaacctatgcc
cacctgttcgacgacaaagtgatgaagcagctgaagcggcggagatacaccggctggggcaggctgagccggaagctgatcaacg
gcatccgggacaagcagtccggcaagacaatcctggatttcctgaagtccgacggcttcgccaacagaaacttcatgcagctgatccac

gacgacagcctgacctttaaagaggacatccagaaagcccaggtgtccggccagggcgatagcctgcacgagcacattgccaatctg
gccggcagccccgccattaagaagggcatcctgcagacagtgaaggtggtggacgagctcgtgaaagtgatgggccggcacaagc
ccgagaacatcgtgatcgaaatggccagagagaaccagaccacccagaagggacagaagaacagccgcgagagaatgaagcgga
tcgaagagggcatcaaagagctgggcagccagatcctgaaagaacaccccgtggaaaacacccagctgcagaacgagaagctgtac
ctgtactacctgcagaatgggcgggatatgtacgtggaccaggaactggacatcaaccggctgtccgactacgatgtggaccatatcgt
gcctcagagctttctgaaggacgactccatcgacaacaaggtgctgaccagaagcgacaagaaccggggcaagagcgacaacgtgc
cctccgaagaggtcgtgaagaagatgaagaactactggcggcagctgctgaacgccaagctgattacccagagaaagttcgacaatct
gaccaaggccgagagaggcggcctgagcgaactggataaggccggcttcatcaagagacagctggtggaaacccggcagatcaca
aagcacgtggcacagatcctggactcccggatgaacactaagtacgacgagaatgacaagctgatccgggaagtgaaagtgatcacc
ctgaagtccaagctggtgtccgatttccggaaggatttccagttttacaaagtgcgcgagatcaacaactaccaccacgcccacgacgc
ctacctgaacgccgtcgtgggaaccgccctgatcaaaaagtaccctaagctggaaagcgagttcgtgtacggcgactacaaggtgtac
gacgtgcggaagatgatcgccaagagcgagcaggaaatcggcaaggctaccgccaagtacttcttctacagcaacatcatgaacttttt
caagaccgagattaccctggccaacggcgagatccggaagcggcctctgatcgagacaaacggcgaaaccggggagatcgtgtgg
gataagggccgggattttgccaccgtgcggaaagtgctgagcatgccccaagtgaatatcgtgaaaaagaccgaggtgcagacaggc
ggcttcagcaaagagtctatcctgcccaagaggaacagcgataagctgatcgccagaaagaaggactgggaccctaagaagtacggc
ggcttcgacagccccaccgtggcctattctgtgctggtggtggccaaagtggaaaagggcaagtccaagaaactgaagagtgtgaaag
agctgctggggatcaccatcatggaaagaagcagcttcgagaagaatcccatcgactttctggaagccaagggctacaaagaagtgaa
aaaggacctgatcatcaagctgcctaagtactccctgttcgagctggaaaacggccggaagagaatgctggcctctgccggcgaactg
cagaagggaaacgaactggccctgccctccaaatatgtgaacttcctgtacctggccagccactatgagaagctgaagggctcccccg
aggataatgagcagaaacagctgtttgtggaacagcacaagcactacctggacgagatcatcgagcagatcagcgagttctccaagag
agtgatcctggccgacgctaatctggacaaagtgctgtccgcctacaacaagcaccgggataagcccatcagagagcaggccgagaa
tatcatccacctgtttaccctgaccaatctgggagcccctgccgccttcaagtactttgacaccaccatcgaccggaagaggtacaccag
caccaaagaggtgctggacgccaccctgatccaccagagcatcaccggcctgtacgagacacggatcgacctgtctcagctgggagg
tgactctggcggctcaaaaagaaccgccgacggcagcgaattcgagcccaagaagaagaggaaagtc (SEQ ID NO: 157).

### 3. Cell Culture and Transfection

HEK293T cells (purchased from ATCC) were inoculated in a DMEM medium (Gibco, 11965092) supplemented with 10% FBS (v/v), containing 1% Penicillin Streptomycin (v/v) (Gibco, 15140122), and cultured in a 37°C cell culture incubator containing 5% CO₂. The cells used for transfection were inoculated in a 24-well cell culture plate the day before and cultured. The cells were observed the next day and transfected when the cells grew to a cell density of about 80%. The amount of plasmids transfected in each well of the 24-well plate was 0.4 µg of p004V1-nCas9 plasmid, and 0.4 µg of sgRNA plasmid. The plasmids were mixed, and then diluted with 25 µl of reduced serum medium (Basal Media, L530KJ), 2 µl of p3000 reagent was added, the mixture was blown and mixed well as a reagent A, and allowed to stand for 5 minutes. At the same time, 2 µl of Lipofectamine 3000 transfection reagent (Thermo, 11668019) was diluted with 25 µl of reduced serum medium and mixed well as a reagent B, and allowed to stand for 5 minutes. The above reagent A and reagent B were mixed and blown evenly, and allowed to stand for 20 minutes. After the standing was finished, the mixed reagent was added dropwise to the cells of the 24-well plate to be transfected and placed back to the 37°C incubator for culture. After 6 hours of transfection, the culture medium was replaced with a DMEM medium containing 10% FBS. After 48 hours of transfection, the cells were collected for editing efficiency detection.

### 4. Detection of Editing Efficiency of Adenine Base Editor 004V1-nCas9 in This example at Endogenous Gene Sites

The cells described in "3. Cell Culture and Transfection" were subjected to genomic extraction (TIANGEN, DP304-03). Primers were designed according to experimental requirements, and identification primer sequences used are shown in SEQ ID NO: 148, SEQ ID NO: 149, SEQ ID NO: 166, SEQ ID NO: 167, SEQ ID NO: 154, and SEQ ID NO: 155 in Table 11. A genome was used as a template, PCR amplification was performed on a sequence near a target, and an amplified PCR product was used for high-throughput deep sequencing (GENEWIZ, Inc) or Sanger sequencing (Boshang Biotechnology (Shanghai) Co., Ltd.) to identify the editing efficiency. A system used for target site sequence amplification is as follows: 25 µL of 2 × Taq Master Mix (Vazyme, P112-03), 1 µL of Primer-F (10 pmol/µL), 1 µL of Primer-R (10 pmol/µL), 1 µL of template, and ddH₂O made up to 50 µL.

A gene editing effect test process is as follows.

The 004V1-nCas9 plasmid was co-transfected with sgRNA plasmids at different sites into HEK293T cells (purchased from ATCC). Compared with an ABE8e plasmid (addgene, Plasmid #138489), it was found that at sites 1 and 17, an editing efficiency of the 004V1-nCas9 was similar to that of the ABE8e (FIG. 7, FIG. 8), and the editing efficiency of the 004V1-nCas9 at a site 18 was significantly better than that of the ABE8e (FIG. 9).

As for editing windows, the editing windows of the 004V1-nCas9 at the sites 1, 17, and 18 were all smaller than those of the ABE8e (see FIGS. 7 to 9).

For a calculation method of gene editing efficiency, see Kluesner MG, Nedveck DA, Lahr WS, Garbe JR, Abrahante JE, Webber BR, Moriarity BS. EditR: A Method to Quantify Base Editing from Sanger Sequencing. CRISPR J. 2018 Jun;1(3):239-250. doi: 10.1089/crispr.2018.0014. PMID: 31021262; PMCID: PMC6694769. Relevant results of this example are shown in FIGS. 7 to 9.

### Example 4

In this example, the adenine base editor 004V1-nCas9 obtained in Example 3 was applied to disease treatment.

Proprotein convertase subtilisin/kexin type 9 (PCSK9) is the ninth member of the kexin-like proprotein convertase subtilisin family, which is constituted by 692 amino acid residues. As a negative regulator of low density lipoprotein receptor (LDLR), excessive PCSK9 can accelerate its degradation after binding to LDLR on a surface of hepatocytes, resulting in decreased uptake of low density lipoprotein cholesterol (LDL-C) by hepatocytes, thereby increasing a level of LDL-C in a peripheral circulation, and ultimately increasing a cholesterol level in the blood.

### 1. Construction of sgRNA Expression Vector (sgRNA Plasmid)

A construction method of the sgRNA plasmid targeting PCSK9 in this example is as described in Example 1. A sgRNA sequence used is shown in SEQ ID NO: 141.

PCSK9-sgRNA: Cccgcaccttggcgcagcgg (SEQ ID NO: 141).

### 2. Cell Culture and Transfection

Culture and transfection methods of HEK293T cells in this example are the same as those in Example 3.

### 3. Detection of Editing Efficiency of Optimized Base Editing Tool at PCSK9 Site

An editing efficiency detection method in this example is the same as that described in Example 1, and identification primer sequences used are shown in SEQ ID NO: 146 and SEQ ID NO: 147.
PCSK9-forward primer: Gctagccttgcgttccg (SEQ ID NO: 146), and
PCSK9-reverse primer: Gtccccaagatcgtgccaa (SEQ ID NO: 147).

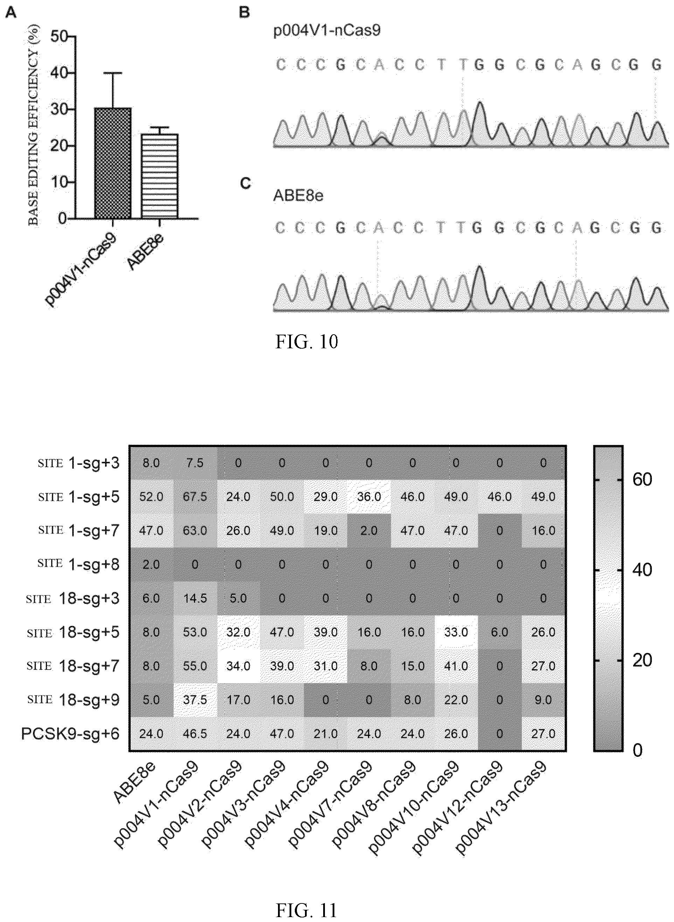

In this example, the adenine base editor expression vector p004V1-nCas9 and the sgRNA plasmid targeting PCSK9 were co-transfected into the HEK293T cells. As shown in FIG. 10, compared with the ABE8e adenine base editor, an editing efficiency of the 004V1-nCas9 is better than that of the ABE8e. This shows that the base editor 004V1-nCas9 can be targeted to the treatment of hypercholesterolemia caused by high expression of PCSK9.

### Example 5

In this example, mutants of an adenosine deaminase 004V1, that is, an adenosine deaminase 004V2, an adenosine deaminase 004V3, an adenosine deaminase 004V4, an adenosine deaminase 004V7, an adenosine deaminase 004V8, an adenosine deaminase 004V10, an adenosine deaminase 004V12, and an adenosine deaminase 004V13 were used, and an adenine base editor expression vector and a sgRNA expression vector were constructed by the same method as in Example 3 or Example 4. Cell culture and transfection were performed, and an editing efficiency was detected.

Amino acid sequence information on a base editor constituted by each of the above mutants is as follows:
004V2-nCas9 (SEQ ID NO: 191), 004V3-nCas9 (SEQ ID NO: 192), 004V4-nCas9 (SEQ ID NO: 193), 004V7-nCas9 (SEQ ID NO: 194), 004V8-nCas9 (SEQ ID NO: 195), 004V10-nCas9 (SEQ ID NO: 196), 004V12-nCas9 (SEQ ID NO: 197), and 004V13-nCas9 (SEQ ID NO: 198).

Base editing efficiency results are shown in FIG. 11. As shown in FIG. 11, compared with ABE8e, editing windows of the 004V2-nCas9, 004V3-nCas9, 004V4-nCas9, 004V7-nCas9, 004V8-nCas9, 004V10-nCas9, 004V12-nCas9, and 004V13-nCas9 at a site 1 are significantly narrowed (+5, +7). Among them, the 004V12-nCas9 has the narrowest editing window, with editing activity only at position +5, and has a base editing efficiency of 46%, which can achieve efficient and accurate editing. At a site 18, editing efficiencies of different mutants of the 004V1-nCas9 are significantly better than that of the ABE8e. For a PCSK9 site, editing efficiencies of the 004V1-nCas9 and the 004V3-nCas9 are also better than that of the ABE8e.

A nucleotide sequence of the base editor constituted by each adenosine deaminase is as follows.

A nucleotide sequence of the 004V2-nCas9 is as follows:

atgaaacggacagccgacggaagcgagttcgagtcaccaaagaagaagcggaaagtcatgtacaatgcccctagattcttttgt

agatctgctgccgtgacagagaaggaatatcaggatgagtactggatgagacacgccctgacactggccaagagggctcgggagga

aggagaggtgccagtgggagctgtgctggtgctgaataatcaggtcattggagaaggatggaatagagccatcggcctgcacgaccc

caccgcccacgccgagatcatggccctgagacagggcggcctggtgctgcagaactaccggctgatcgacgccaccctgtacgtga

ccttcgagccctgcgtgatgtgcgccggcgccatggtgcatagcagaatcagcagactggtgttcggcgtgagaaactcaaagagag

gcgccgccggcagcctgatcaacgtgctgaactaccccggcatgaaccacagagtggagatcaccgagggcatcctggccgagagc

tgcagcgccatgctgtgcgacttctataggtggcctagggaggtgttcaatgccctgaagaaggccagacaggaggagggctccgga

ggatctagcggaggctcctctggctctgagacacctggcacaagcgagagcgcaacacctgaaagcagcgggggcagcagcgggg

ggtcagacaagaagtacagcatcggcctggccatcggcaccaactctgtgggctgggccgtgatcaccgacgagtacaaggtgccca

gcaagaaattcaaggtgctgggcaacaccgaccggcacagcatcaagaagaacctgatcggagccctgctgttcgacagcggcgaa

acagccgaggccacccggctgaagagaaccgccagaagaagatacaccagacggaagaaccggatctgctatctgcaagagatctt

cagcaacgagatggccaaggtggacgacagcttcttccacagactggaagagtccttcctggtggaagaggataagaagcacgagcg

gcaccccatcttcggcaacatcgtggacgaggtggcctaccacgagaagtaccccaccatctaccacctgagaaagaaactggtgga

cagcaccgacaaggccgacctgcggctgatctatctggccctggcccacatgatcaagttccggggccacttcctgatcgagggcgac

ctgaaccccgacaacagcgacgtggacaagctgttcatccagctggtgcagacctacaaccagctgttcgaggaaaaccccatcaacg

ccagcggcgtggacgccaaggccatcctgtctgccagactgagcaagagcagacggctggaaaatctgatcgcccagctgcccggc

gagaagaagaatggcctgttcggaaacctgattgccctgagcctgggcctgacccccaacttcaagagcaacttcgacctggccgagg

atgccaaactgcagctgagcaaggacacctacgacgacgacctggacaacctgctggcccagatcggcgaccagtacgccgacctg

tttctggccgccaagaacctgtccgacgccatcctgctgagcgacatcctgagagtgaacaccgagatcaccaaggcccccctgagcg

cctctatgatcaagagatacgacgagcaccaccaggacctgaccctgctgaaagctctcgtgcggcagcagctgcctgagaagtacaa

agagattttcttcgaccagagcaagaacggctacgccggctacattgacggcggagccagccaggaagagttctacaagttcatcaag

cccatcctggaaaagatggacggcaccgaggaactgctcgtgaagctgaacagagaggacctgctgcggaagcagcggaccttcga

caacggcagcatcccccaccagatccacctgggagagctgcacgccattctgcggcggcaggaagatttttacccattcctgaaggac

aaccgggaaaagatcgagaagatcctgaccttccgcatcccctactacgtgggccctctggccaggggaaacagcagattcgcctgg

atgaccagaaagagcgaggaaaccatcaccccctggaacttcgaggaagtggtggacaagggcgcttccgcccagagcttcatcga

gcggatgaccaacttcgataagaacctgcccaacgagaaggtgctgcccaagcacagcctgctgtacgagtacttcaccgtgtataac

gagctgaccaaagtgaaatacgtgaccgagggaatgagaaagcccgccttcctgagcggcgagcagaaaaaggccatcgtggacct

gctgttcaagaccaaccggaaagtgaccgtgaagcagctgaaagaggactacttcaagaaaatcgagtgcttcgactccgtggaaatct

ccggcgtggaagatcggttcaacgcctccctgggcacataccacgatctgctgaaaattatcaaggacaaggacttcctggacaatgag

gaaaacgaggacattctggaagatatcgtgctgaccctgacactgtttgaggacagagagatgatcgaggaacggctgaaaacctatg

cccacctgttcgacgacaaagtgatgaagcagctgaagcggcggagatacaccggctggggcaggctgagccggaagctgatcaac

ggcatccgggacaagcagtccggcaagacaatcctggatttcctgaagtccgacggcttcgccaacagaaacttcatgcagctgatcc

acgacgacagcctgacctttaaagaggacatccagaaagcccaggtgtccggccagggcgatagcctgcacgagcacattgccaatc

tggccggcagccccgccattaagaagggcatcctgcagacagtgaaggtggtggacgagctcgtgaaagtgatgggccggcacaag

cccgagaacatcgtgatcgaaatggccagagagaaccagaccacccagaagggacagaagaacagccgcgagagaatgaagcgg

atcgaagagggcatcaaagagctgggcagccagatcctgaaagaacaccccgtggaaaacacccagctgcagaacgagaagctgta

cctgtactacctgcagaatgggcgggatatgtacgtggaccaggaactggacatcaaccggctgtccgactacgatgtggaccatatcg

tgcctcagagctttctgaaggacgactccatcgacaacaaggtgctgaccagaagcgacaagaaccggggcaagagcgacaacgtg

ccctccgaagaggtcgtgaagaagatgaagaactactggcggcagctgctgaacgccaagctgattacccagagaaagttcgacaat

ctgaccaaggccgagagaggcggcctgagcgaactggataaggccggcttcatcaagagacagctggtggaaacccggcagatca

caaagcacgtggcacagatcctggactcccggatgaacactaagtacgacgagaatgacaagctgatccgggaagtgaaagtgatca

ccctgaagtccaagctggtgtccgatttccggaaggatttccagttttacaaagtgcgcgagatcaacaactaccaccacgcccacgac

gcctacctgaacgccgtcgtgggaaccgccctgatcaaaaagtaccctaagctggaaagcgagttcgtgtacggcgactacaaggtgt

acgacgtgcggaagatgatcgccaagagcgagcaggaaatcggcaaggctaccgccaagtacttcttctacagcaacatcatgaactt

tttcaagaccgagattaccctggccaacggcgagatccggaagcggcctctgatcgagacaaacggcgaaaccggggagatcgtgtg

ggataagggccgggattttgccaccgtgcggaaagtgctgagcatgccccaagtgaatatcgtgaaaaagaccgaggtgcagacagg

cggcttcagcaaagagtctatcctgcccaagaggaacagcgataagctgatcgccagaaagaaggactgggaccctaagaagtacgg
cggcttcgacagccccaccgtggcctattctgtgctggtggtggccaaagtggaaaagggcaagtccaagaaactgaagagtgtgaaa
gagctgctggggatcaccatcatggaaagaagcagcttcgagaagaatcccatcgactttctggaagccaagggctacaaagaagtga
aaaaggacctgatcatcaagctgcctaagtactccctgttcgagctggaaaacggccggaagagaatgctggcctctgccggcgaact
gcagaagggaaacgaactggccctgccctccaaatatgtgaacttcctgtacctggccagccactatgagaagctgaagggctccccc
gaggataatgagcagaaacagctgtttgtggaacagcacaagcactacctggacgagatcatcgagcagatcagcgagttctccaaga
gagtgatcctggccgacgctaatctggacaaagtgctgtccgcctacaacaagcaccgggataagcccatcagagagcaggccgaga
atatcatccacctgtttaccctgaccaatctgggagcccctgccgccttcaagtactttgacaccaccatcgaccggaagaggtacacca
gcaccaaagaggtgctggacgccaccctgatccaccagagcatcaccggcctgtacgagacacggatcgacctgtctcagctgggag
gtgactctggcggctcaaaaagaaccgccgacggcagcgaattcgagcccaagaagaagaggaaagtc (SEQ ID NO: 158).

A nucleotide sequence of the 004V3-nCas9 is as follows:

atgaaacggacagccgacggaagcgagttcgagtcaccaaagaagaagcggaaagtcatgtacaatgcccctagattcttttgt
agatctgctgccgtgtctgatcatgagtttaacgatgattttttcatgaagcgcgcaattacactggccaagagggctcgggaggaagga
gaggtgccagtgggagctgtgctggtgctgaataatcaggtcattggagaaggatggaatagagccatcggcctgcacgaccccacc
gcccacgccgagatcatggccctgagacagggcggcctggtgctgcagaactaccggctgatcgacgccaccctgtacgtgaccttc
gagccctgcgtgatgtgcgccggcgccatggtgcatagcagaatcagcagactggtgttcggcgtgagaaactcaaagagaggcgcc
gccggcagcctgatcaacgtgctgaactaccccggcatgaaccacagagtggagatcaccgagggcatcctggccgagagctgcag
cgccatgctgtgcgacttctataggtggcctagggaggtgttcaatgccctgaagaaggccagacaggaggagggctccggaggatct
agcggaggctcctctggctctgagacacctggcacaagcgagagcgcaacacctgaaagcagcgggggcagcagcggggggtca
gacaagaagtacagcatcggcctggccatcggcaccaactctgtgggctgggccgtgatcaccgacgagtacaaggtgcccagcaa
gaaattcaaggtgctgggcaacaccgaccggcacagcatcaagaagaacctgatcggagccctgctgttcgacagcggcgaaacag
ccgaggccacccggctgaagagaaccgccagaagaagatacaccagacggaagaaccggatctgctatctgcaagagatcttcagc
aacgagatggccaaggtggacgacagcttcttccacagactggaagagtccttcctggtggaagaggataagaagcacgagcggcac
cccatcttcggcaacatcgtggacgaggtggcctaccacgagaagtaccccaccatctaccacctgagaaagaaactggtggacagc

accgacaaggccgacctgcggctgatctatctggccctggcccacatgatcaagttccggggccacttcctgatcgagggcgacctga

accccgacaacagcgacgtggacaagctgttcatccagctggtgcagacctacaaccagctgttcgaggaaaaccccatcaacgcca

gcggcgtggacgccaaggccatcctgtctgccagactgagcaagagcagacggctggaaaatctgatcgcccagctgcccggcgag

aagaagaatggcctgttcggaaacctgattgccctgagcctgggcctgacccccaacttcaagagcaacttcgacctggccgaggatg

ccaaactgcagctgagcaaggacacctacgacgacgacctggacaacctgctggcccagatcggcgaccagtacgccgacctgtttc

tggccgccaagaacctgtccgacgccatcctgctgagcgacatcctgagagtgaacaccgagatcaccaaggcccccctgagcgcct

ctatgatcaagagatacgacgagcaccaccaggacctgaccctgctgaaagctctcgtgcggcagcagctgcctgagaagtacaaag

agattttcttcgaccagagcaagaacggctacgccggctacattgacggcggagccagccaggaagagttctacaagttcatcaagcc

catcctggaaaagatggacggcaccgaggaactgctcgtgaagctgaacagagaggacctgctgcggaagcagcggaccttcgaca

acggcagcatcccccaccagatccacctgggagagctgcacgccattctgcggcggcaggaagatttttacccattcctgaaggacaa

ccgggaaaagatcgagaagatcctgaccttccgcatcccctactacgtgggccctctggccaggggaaacagcagattcgcctggatg

accagaaagagcgaggaaaccatcaccccctggaacttcgaggaagtggtggacaagggcgcttccgcccagagcttcatcgagcg

gatgaccaacttcgataagaacctgcccaacgagaaggtgctgcccaagcacagcctgctgtacgagtacttcaccgtgtataacgagc

tgaccaaagtgaaatacgtgaccgagggaatgagaaagcccgccttcctgagcggcgagcagaaaaaggccatcgtggacctgctgt

tcaagaccaaccggaaagtgaccgtgaagcagctgaaagaggactacttcaagaaaatcgagtgcttcgactccgtggaaatctccgg

cgtggaagatcggttcaacgcctccctgggcacataccacgatctgctgaaaattatcaaggacaaggacttcctggacaatgaggaaa

acgaggacattctggaagatatcgtgctgaccctgacactgtttgaggacagagagatgatcgaggaacggctgaaaacctatgccca

cctgttcgacgacaaagtgatgaagcagctgaagcggcggagatacaccggctggggcaggctgagccggaagctgatcaacggc

atccgggacaagcagtccggcaagacaatcctggatttcctgaagtccgacggcttcgccaacagaaacttcatgcagctgatccacga

cgacagcctgacctttaaagaggacatccagaaagcccaggtgtccggccagggcgatagcctgcacgagcacattgccaatctggc

cggcagccccgccattaagaagggcatcctgcagacagtgaaggtggtggacgagctcgtgaaagtgatgggccggcacaagccc

gagaacatcgtgatcgaaatggccagagagaaccagaccacccagaagggacagaagaacagccgcgagagaatgaagcggatc

gaagagggcatcaaagagctgggcagccagatcctgaaagaacaccccgtggaaaacacccagctgcagaacgagaagctgtacct

gtactacctgcagaatgggcgggatatgtacgtggaccaggaactggacatcaaccggctgtccgactacgatgtggaccatatcgtgc

ctcagagctttctgaaggacgactccatcgacaacaaggtgctgaccagaagcgacaagaaccggggcaagagcgacaacgtgccc
tccgaagaggtcgtgaagaagatgaagaactactggcggcagctgctgaacgccaagctgattacccagagaaagttcgacaatctga
ccaaggccgagagaggcggcctgagcgaactggataaggccggcttcatcaagagacagctggtggaaacccggcagatcacaaa
gcacgtggcacagatcctggactcccggatgaacactaagtacgacgagaatgacaagctgatccgggaagtgaaagtgatcaccct
gaagtccaagctggtgtccgatttccggaaggatttccagttttacaaagtgcgcgagatcaacaactaccaccacgcccacgacgcct
acctgaacgccgtcgtgggaaccgccctgatcaaaaagtaccctaagctggaaagcgagttcgtgtacggcgactacaaggtgtacga
cgtgcggaagatgatcgccaagagcgagcaggaaatcggcaaggctaccgccaagtacttcttctacagcaacatcatgaactttttca
agaccgagattaccctggccaacggcgagatccggaagcggcctctgatcgagacaaacggcgaaaccggggagatcgtgtgggat
aagggccgggattttgccaccgtgcggaaagtgctgagcatgccccaagtgaatatcgtgaaaaagaccgaggtgcagacaggcgg
cttcagcaaagagtctatcctgcccaagaggaacagcgataagctgatcgccagaaagaaggactgggaccctaagaagtacggcgg
cttcgacagccccaccgtggcctattctgtgctggtggtggccaaagtggaaaagggcaagtccaagaaactgaagagtgtgaaagag
ctgctggggatcaccatcatggaaagaagcagcttcgagaagaatcccatcgactttctggaagccaagggctacaaagaagtgaaaa
aggacctgatcatcaagctgcctaagtactccctgttcgagctggaaaacggccggaagagaatgctggcctctgccggcgaactgca
gaagggaaacgaactggccctgccctccaaatatgtgaacttcctgtacctggccagccactatgagaagctgaagggctcccccgag
gataatgagcagaaacagctgtttgtggaacagcacaagcactacctggacgagatcatcgagcagatcagcgagttctccaagagag
tgatcctggccgacgctaatctggacaaagtgctgtccgcctacaacaagcaccgggataagcccatcagagagcaggccgagaatat
catccacctgtttaccctgaccaatctgggagcccctgccgccttcaagtactttgacaccaccatcgaccggaagaggtacaccagca
ccaaagaggtgctggacgccaccctgatccaccagagcatcaccggcctgtacgagacacggatcgacctgtctcagctgggaggtg
actctggcggctcaaaaagaaccgccgacggcagcgaattcgagcccaagaagaagaggaaagtc (SEQ ID NO: 159).

A nucleotide sequence of the 004V4-nCas9 is as follows:

atgaaacggacagccgacggaagcgagttcgagtcaccaaagaagaagcggaaagtcatgtacaatgcccctagattcttttgt

agatctgctgccgtgtctgatcatgagtttaacgatgagtactggatgagacacgccctgacactggccagaaagtctcgggaggaagg

agaggtgccagtgggagctgtgctggtgctgaataatcaggtcattggagaaggatggaatagagccatcggcctgcacgaccccac

cgcccacgccgagatcatggccctgagacagggcggcctggtgctgcagaactaccggctgatcgacgccaccctgtacgtgacctt

cgagccctgcgtgatgtgcgccggcgccatggtgcatagcagaatcagcagactggtgttcggcgtgagaaactcaaagagaggcgc

cgccggcagcctgatcaacgtgctgaactaccccggcatgaaccacagagtggagatcaccgagggcatcctggccgagagctgca

gcgccatgctgtgcgacttctataggtggcctagggaggtgttcaatgccctgaagaaggccagacaggaggagggctccggaggat

ctagcggaggctcctctggctctgagacacctggcacaagcgagagcgcaacacctgaaagcagcgggggcagcagcggggggtc

agacaagaagtacagcatcggcctggccatcggcaccaactctgtgggctgggccgtgatcaccgacgagtacaaggtgcccagca

agaaattcaaggtgctgggcaacaccgaccggcacagcatcaagaagaacctgatcggagccctgctgttcgacagcggcgaaaca

gccgaggccacccggctgaagagaaccgccagaagaagatacaccagacggaagaaccggatctgctatctgcaagagatcttcag

caacgagatggccaaggtggacgacagcttcttccacagactggaagagtccttcctggtggaagaggataagaagcacgagcggca

ccccatcttcggcaacatcgtggacgaggtggcctaccacgagaagtaccccaccatctaccacctgagaaagaaactggtggacag

caccgacaaggccgacctgcggctgatctatctggccctggcccacatgatcaagttccggggccacttcctgatcgagggcgacctg

aaccccgacaacagcgacgtggacaagctgttcatccagctggtgcagacctacaaccagctgttcgaggaaaaccccatcaacgcc

agcggcgtggacgccaaggccatcctgtctgccagactgagcaagagcagacggctggaaaatctgatcgcccagctgcccggcga

gaagaagaatggcctgttcggaaacctgattgccctgagcctgggcctgacccccaacttcaagagcaacttcgacctggccgaggat

gccaaactgcagctgagcaaggacacctacgacgacgacctggacaacctgctggcccagatcggcgaccagtacgccgacctgttt

ctggccgccaagaacctgtccgacgccatcctgctgagcgacatcctgagagtgaacaccgagatcaccaaggcccccctgagcgcc

tctatgatcaagagatacgacgagcaccaccaggacctgaccctgctgaaagctctcgtgcggcagcagctgcctgagaagtacaaag

agattttcttcgaccagagcaagaacggctacgccggctacattgacggcggagccagccaggaagagttctacaagttcatcaagcc

catcctggaaaagatggacggcaccgaggaactgctcgtgaagctgaacagagaggacctgctgcggaagcagcggaccttcgaca

acggcagcatcccccaccagatccacctgggagagctgcacgccattctgcggcggcaggaagatttttacccattcctgaaggacaa

ccgggaaaagatcgagaagatcctgaccttccgcatcccctactacgtgggccctctggccaggggaaacagcagattcgcctggatg

accagaaagagcgaggaaaccatcaccccctggaacttcgaggaagtggtggacaagggcgcttccgcccagagcttcatcgagcg

gatgaccaacttcgataagaacctgcccaacgagaaggtgctgcccaagcacagcctgctgtacgagtacttcaccgtgtataacgagc

tgaccaaagtgaaatacgtgaccgagggaatgagaaagcccgccttcctgagcggcgagcagaaaaaggccatcgtggacctgctgt

tcaagaccaaccggaaagtgaccgtgaagcagctgaaagaggactacttcaagaaaatcgagtgcttcgactccgtggaaatctccgg

cgtggaagatcggttcaacgcctccctgggcacataccacgatctgctgaaaattatcaaggacaaggacttcctggacaatgaggaaa

acgaggacattctggaagatatcgtgctgaccctgacactgtttgaggacagagagatgatcgaggaacggctgaaaacctatgccca

cctgttcgacgacaaagtgatgaagcagctgaagcggcggagatacaccggctggggcaggctgagccggaagctgatcaacggc

atccgggacaagcagtccggcaagacaatcctggatttcctgaagtccgacggcttcgccaacagaaacttcatgcagctgatccacga

cgacagcctgacctttaaagaggacatccagaaagcccaggtgtccggccagggcgatagcctgcacgagcacattgccaatctggc

cggcagccccgccattaagaagggcatcctgcagacagtgaaggtggtggacgagctcgtgaaagtgatgggccggcacaagccc

gagaacatcgtgatcgaaatggccagagagaaccagaccacccagaagggacagaagaacagccgcgagagaatgaagcggatc

gaagagggcatcaaagagctgggcagccagatcctgaaagaacaccccgtggaaaacacccagctgcagaacgagaagctgtacct

gtactacctgcagaatgggcgggatatgtacgtggaccaggaactggacatcaaccggctgtccgactacgatgtggaccatatcgtgc

ctcagagctttctgaaggacgactccatcgacaacaaggtgctgaccagaagcgacaagaaccggggcaagagcgacaacgtgccc

tccgaagaggtcgtgaagaagatgaagaactactggcggcagctgctgaacgccaagctgattacccagagaaagttcgacaatctga

ccaaggccgagagaggcggcctgagcgaactggataaggccggcttcatcaagagacagctggtggaaacccggcagatcacaaa

gcacgtggcacagatcctggactcccggatgaacactaagtacgacgagaatgacaagctgatccgggaagtgaaagtgatcaccct

gaagtccaagctggtgtccgatttccggaaggatttccagttttacaaagtgcgcgagatcaacaactaccaccacgcccacgacgcct

acctgaacgccgtcgtgggaaccgccctgatcaaaaagtaccctaagctggaaagcgagttcgtgtacggcgactacaaggtgtacga

cgtgcggaagatgatcgccaagagcgagcaggaaatcggcaaggctaccgccaagtacttcttctacagcaacatcatgaactttttca

agaccgagattaccctggccaacggcgagatccggaagcggcctctgatcgagacaaacggcgaaaccggggagatcgtgtgggat

aagggccgggattttgccaccgtgcggaaagtgctgagcatgccccaagtgaatatcgtgaaaaagaccgaggtgcagacaggcgg

cttcagcaaagagtctatcctgcccaagaggaacagcgataagctgatcgccagaaagaaggactgggaccctaagaagtacggcgg

cttcgacagccccaccgtggcctattctgtgctggtggtggccaaagtggaaaagggcaagtccaagaaactgaagagtgtgaaagag

ctgctggggatcaccatcatggaaagaagcagcttcgagaagaatcccatcgactttctggaagccaagggctacaaagaagtgaaaa

aggacctgatcatcaagctgcctaagtactccctgttcgagctggaaaacggccggaagagaatgctggcctctgccggcgaactgca

gaagggaaacgaactggccctgccctccaaatatgtgaacttcctgtacctggccagccactatgagaagctgaagggctcccccgag

gataatgagcagaaacagctgtttgtggaacagcacaagcactacctggacgagatcatcgagcagatcagcgagttctccaagagag

tgatcctggccgacgctaatctggacaaagtgctgtccgcctacaacaagcaccgggataagcccatcagagagcaggccgagaatat
catccacctgtttaccctgaccaatctgggagcccctgccgccttcaagtactttgacaccaccatcgaccggaagaggtacaccagca
ccaaagaggtgctggacgccaccctgatccaccagagcatcaccggcctgtacgagacacggatcgacctgtctcagctgggaggtg
actctggcggctcaaaaagaaccgccgacggcagcgaattcgagcccaagaagaagaggaaagtc (SEQ ID NO: 160).

A nucleotide sequence of the 004V7-nCas9 is as follows:

atgaaacggacagccgacggaagcgagttcgagtcaccaaagaagaagcggaaagtcatgtacaatgcccctagattcttttgt
agatctgctgccgtgtctgatcatgagtttaacgatgagtactggatgagacacgccctgacactggccaagagggctcgggaggaag
gagaggtgccagtgggagctgtgctggtgctgaataatcaggtcattggagaaggatggaatagagccatcggcctgcacgacccca
ccgcccacgccgagatcatggccctgagacagggcgccaatgccattaacagctggcggctgatcgacgccaccctgtacgtgacct
tcgagccctgcgtgatgtgcgccggcgccatggtgcatagcagaatcagcagactggtgttcggcgtgagaaactcaaagagaggcg
ccgccggcagcctgatcaacgtgctgaactaccccggcatgaaccacagagtggagatcaccgagggcatcctggccgagagctgc
agcgccatgctgtgcgacttctataggtggcctagggaggtgttcaatgccctgaagaaggccagacaggaggagggctccggagga
tctagcggaggctcctctggctctgagacacctggcacaagcgagagcgcaacacctgaaagcagcgggggcagcagcggggggt
cagacaagaagtacagcatcggcctggccatcggcaccaactctgtgggctgggccgtgatcaccgacgagtacaaggtgcccagc
aagaaattcaaggtgctgggcaacaccgaccggcacagcatcaagaagaacctgatcggagccctgctgttcgacagcggcgaaac
agccgaggccacccggctgaagagaaccgccagaagaagatacaccagacggaagaaccggatctgctatctgcaagagatcttca
gcaacgagatggccaaggtggacgacagcttcttccacagactggaagagtccttcctggtggaagaggataagaagcacgagcgg
caccccatcttcggcaacatcgtggacgaggtggcctaccacgagaagtaccccaccatctaccacctgagaaagaaactggtggaca
gcaccgacaaggccgacctgcggctgatctatctggccctggcccacatgatcaagttccggggccacttcctgatcgagggcgacct
gaaccccgacaacagcgacgtggacaagctgttcatccagctggtgcagacctacaaccagctgttcgaggaaaaccccatcaacgc
cagcggcgtggacgccaaggccatcctgtctgccagactgagcaagagcagacggctggaaaatctgatcgcccagctgcccggcg
agaagaagaatggcctgttcggaaacctgattgccctgagcctgggcctgacccccaacttcaagagcaacttcgacctggccgagga
tgccaaactgcagctgagcaaggacacctacgacgacgacctggacaacctgctggcccagatcggcgaccagtacgccgacctgtt
tctggccgccaagaacctgtccgacgccatcctgctgagcgacatcctgagagtgaacaccgagatcaccaaggcccccctgagcgc

ctctatgatcaagagatacgacgagcaccaccaggacctgaccctgctgaaagctctcgtgcggcagcagctgcctgagaagtacaaa

gagattttcttcgaccagagcaagaacggctacgccggctacattgacggcggagccagccaggaagagttctacaagttcatcaagc

ccatcctggaaaagatggacggcaccgaggaactgctcgtgaagctgaacagagaggacctgctgcggaagcagcggaccttcgac

aacggcagcatcccccaccagatccacctgggagagctgcacgccattctgcggcggcaggaagatttttacccattcctgaaggaca

accgggaaaagatcgagaagatcctgaccttccgcatcccctactacgtgggccctctggccaggggaaacagcagattcgcctggat

gaccagaaagagcgaggaaaccatcaccccctggaacttcgaggaagtggtggacaagggcgcttccgcccagagcttcatcgagc

ggatgaccaacttcgataagaacctgcccaacgagaaggtgctgcccaagcacagcctgctgtacgagtacttcaccgtgtataacga

gctgaccaaagtgaaatacgtgaccgagggaatgagaaagcccgccttcctgagcggcgagcagaaaaaggccatcgtggacctgc

tgttcaagaccaaccggaaagtgaccgtgaagcagctgaaagaggactacttcaagaaaatcgagtgcttcgactccgtggaaatctcc

ggcgtggaagatcggttcaacgcctccctgggcacataccacgatctgctgaaaattatcaaggacaaggacttcctggacaatgagga

aaacgaggacattctggaagatatcgtgctgaccctgacactgtttgaggacagagagatgatcgaggaacggctgaaaacctatgcc

cacctgttcgacgacaaagtgatgaagcagctgaagcggcggagatacaccggctggggcaggctgagccggaagctgatcaacg

gcatccgggacaagcagtccggcaagacaatcctggatttcctgaagtccgacggcttcgccaacagaaacttcatgcagctgatccac

gacgacagcctgacctttaaagaggacatccagaaagcccaggtgtccggccagggcgatagcctgcacgagcacattgccaatctg

gccggcagccccgccattaagaagggcatcctgcagacagtgaaggtggtggacgagctcgtgaaagtgatgggccggcacaagc

ccgagaacatcgtgatcgaaatggccagagagaaccagaccacccagaagggacagaagaacagccgcgagagaatgaagcgga

tcgaagagggcatcaaagagctgggcagccagatcctgaaagaacaccccgtggaaaacacccagctgcagaacgagaagctgtac

ctgtactacctgcagaatgggcgggatatgtacgtggaccaggaactggacatcaaccggctgtccgactacgatgtggaccatatcgt

gcctcagagctttctgaaggacgactccatcgacaacaaggtgctgaccagaagcgacaagaaccggggcaagagcgacaacgtgc

cctccgaagaggtcgtgaagaagatgaagaactactggcggcagctgctgaacgccaagctgattacccagagaaagttcgacaatct

gaccaaggccgagagaggcggcctgagcgaactggataaggccggcttcatcaagagacagctggtggaaacccggcagatcaca

aagcacgtggcacagatcctggactcccggatgaacactaagtacgacgagaatgacaagctgatccgggaagtgaaagtgatcacc

ctgaagtccaagctggtgtccgatttccggaaggatttccagttttacaaagtgcgcgagatcaacaactaccaccacgcccacgacgc

ctacctgaacgccgtcgtgggaaccgccctgatcaaaaagtaccctaagctggaaagcgagttcgtgtacggcgactacaaggtgtac

gacgtgcggaagatgatcgccaagagcgagcaggaaatcggcaaggctaccgccaagtacttcttctacagcaacatcatgaacttttt
caagaccgagattaccctggccaacggcgagatccggaagcggcctctgatcgagacaaacggcgaaaccggggagatcgtgtgg
gataagggccgggattttgccaccgtgcggaaagtgctgagcatgccccaagtgaatatcgtgaaaaagaccgaggtgcagacaggc
ggcttcagcaaagagtctatcctgcccaagaggaacagcgataagctgatcgccagaaagaaggactgggaccctaagaagtacggc
ggcttcgacagccccaccgtggcctattctgtgctggtggtggccaaagtggaaaagggcaagtccaagaaactgaagagtgtgaaag
agctgctggggatcaccatcatggaaagaagcagcttcgagaagaatcccatcgactttctggaagccaagggctacaaagaagtgaa
aaaggacctgatcatcaagctgcctaagtactccctgttcgagctggaaaacggccggaagagaatgctggcctctgccggcgaactg
cagaagggaaacgaactggccctgccctccaaatatgtgaacttcctgtacctggccagccactatgagaagctgaagggctcccccg
aggataatgagcagaaacagctgtttgtggaacagcacaagcactacctggacgagatcatcgagcagatcagcgagttctccaagag
agtgatcctggccgacgctaatctggacaaagtgctgtccgcctacaacaagcaccgggataagcccatcagagagcaggccgagaa
tatcatccacctgtttaccctgaccaatctgggagcccctgccgccttcaagtactttgacaccaccatcgaccggaagaggtacaccag
caccaaagaggtgctggacgccaccctgatccaccagagcatcaccggcctgtacgagacacggatcgacctgtctcagctgggagg
tgactctggcggctcaaaaagaaccgccgacggcagcgaattcgagcccaagaagaagaggaaagtc (SEQ ID NO: 161).

A nucleotide sequence of the 004V8-nCas9 is as follows:

atgaaacggacagccgacggaagcgagttcgagtcaccaaagaagaagcggaaagtcatgtacaatgcccctagattcttttgt
agatctgctgccgtgtctgatcatgagtttaacgatgagtactggatgagacacgccctgacactggccaagagggctcgggaggaag
gagaggtgccagtgggagctgtgctggtgctgaataatcaggtcattggagaaggatggaatagagccatcggcctgcacgacccca
ccgcccacgccgagatcatggccctgagacagggcggcctggtgctgcagaactaccggctgctgggaacagacctgtacgtgacct
tcgagccctgcgtgatgtgcgccggcgccatggtgcatagcagaatcagcagactggtgttcggcgtgagaaactcaaagagaggcg
ccgccggcagcctgatcaacgtgctgaactaccccggcatgaaccacagagtggagatcaccgagggcatcctggccgagagctgc
agcgccatgctgtgcgacttctataggtggcctagggaggtgttcaatgccctgaagaaggccagacaggaggagggctccggagga
tctagcggaggctcctctggctctgagacacctggcacaagcgagagcgcaacacctgaaagcagcgggggcagcagcggggggt
cagacaagaagtacagcatcggcctggccatcggcaccaactctgtgggctgggccgtgatcaccgacgagtacaaggtgcccagc
aagaaattcaaggtgctgggcaacaccgaccggcacagcatcaagaagaacctgatcggagccctgctgttcgacagcggcgaaac

agccgaggccacccggctgaagagaaccgccagaagaagatacaccagacggaagaaccggatctgctatctgcaagagatcttca

gcaacgagatggccaaggtggacgacagcttcttccacagactggaagagtccttcctggtggaagaggataagaagcacgagcgg

caccccatcttcggcaacatcgtggacgaggtggcctaccacgagaagtaccccaccatctaccacctgagaaagaaactggtggaca

gcaccgacaaggccgacctgcggctgatctatctggccctggcccacatgatcaagttccggggccacttcctgatcgagggcgacct

gaaccccgacaacagcgacgtggacaagctgttcatccagctggtgcagacctacaaccagctgttcgaggaaaaccccatcaacgc

cagcggcgtggacgccaaggccatcctgtctgccagactgagcaagagcagacggctggaaaatctgatcgcccagctgcccggcg

agaagaagaatggcctgttcggaaacctgattgccctgagcctgggcctgacccccaacttcaagagcaacttcgacctggccgagga

tgccaaactgcagctgagcaaggacacctacgacgacgacctggacaacctgctggcccagatcggcgaccagtacgccgacctgtt

tctggccgccaagaacctgtccgacgccatcctgctgagcgacatcctgagagtgaacaccgagatcaccaaggcccccctgagcgc

ctctatgatcaagagatacgacgagcaccaccaggacctgaccctgctgaaagctctcgtgcggcagcagctgcctgagaagtacaaa

gagattttcttcgaccagagcaagaacggctacgccggctacattgacggcggagccagccaggaagagttctacaagttcatcaagc

ccatcctggaaaagatggacggcaccgaggaactgctcgtgaagctgaacagagaggacctgctgcggaagcagcggaccttcgac

aacggcagcatcccccaccagatccacctgggagagctgcacgccattctgcggcggcaggaagatttttacccattcctgaaggaca

accgggaaaagatcgagaagatcctgaccttccgcatcccctactacgtgggccctctggccaggggaaacagcagattcgcctggat

gaccagaaagagcgaggaaaccatcaccccctggaacttcgaggaagtggtggacaagggcgcttccgcccagagcttcatcgagc

ggatgaccaacttcgataagaacctgcccaacgagaaggtgctgcccaagcacagcctgctgtacgagtacttcaccgtgtataacga

gctgaccaaagtgaaatacgtgaccgagggaatgagaaagcccgccttcctgagcggcgagcagaaaaaggccatcgtggacctgc

tgttcaagaccaaccggaaagtgaccgtgaagcagctgaaagaggactacttcaagaaaatcgagtgcttcgactccgtggaaatctcc

ggcgtggaagatcggttcaacgcctccctgggcacataccacgatctgctgaaaattatcaaggacaaggacttcctggacaatgagga

aaacgaggacattctggaagatatcgtgctgaccctgacactgtttgaggacagagagatgatcgaggaacggctgaaaacctatgcc

cacctgttcgacgacaaagtgatgaagcagctgaagcggcggagatacaccggctggggcaggctgagccggaagctgatcaacg

gcatccgggacaagcagtccggcaagacaatcctggatttcctgaagtccgacggcttcgccaacagaaacttcatgcagctgatccac

gacgacagcctgacctttaaagaggacatccagaaagcccaggtgtccggccagggcgatagcctgcacgagcacattgccaatctg

gccggcagccccgccattaagaagggcatcctgcagacagtgaaggtggtggacgagctcgtgaaagtgatgggccggcacaagc

ccgagaacatcgtgatcgaaatggccagagagaaccagaccacccagaagggacagaagaacagccgcgagagaatgaagcgga
tcgaagagggcatcaaagagctgggcagccagatcctgaaagaacaccccgtggaaaacacccagctgcagaacgagaagctgtac
ctgtactacctgcagaatgggcgggatatgtacgtggaccaggaactggacatcaaccggctgtccgactacgatgtggaccatatcgt
gcctcagagctttctgaaggacgactccatcgacaacaaggtgctgaccagaagcgacaagaaccggggcaagagcgacaacgtgc
cctccgaagaggtcgtgaagaagatgaagaactactggcggcagctgctgaacgccaagctgattacccagagaaagttcgacaatct
gaccaaggccgagagaggcggcctgagcgaactggataaggccggcttcatcaagagacagctggtggaaacccggcagatcaca
aagcacgtggcacagatcctggactcccggatgaacactaagtacgacgagaatgacaagctgatccgggaagtgaaagtgatcacc
ctgaagtccaagctggtgtccgatttccggaaggatttccagttttacaaagtgcgcgagatcaacaactaccaccacgcccacgacgc
ctacctgaacgccgtcgtgggaaccgccctgatcaaaaagtaccctaagctggaaagcgagttcgtgtacggcgactacaaggtgtac
gacgtgcggaagatgatcgccaagagcgagcaggaaatcggcaaggctaccgccaagtacttcttctacagcaacatcatgaacttttt
caagaccgagattaccctggccaacggcgagatccggaagcggcctctgatcgagacaaacggcgaaaccggggagatcgtgtgg
gataagggccgggattttgccaccgtgcggaaagtgctgagcatgccccaagtgaatatcgtgaaaaagaccgaggtgcagacaggc
ggcttcagcaaagagtctatcctgcccaagaggaacagcgataagctgatcgccagaaagaaggactgggaccctaagaagtacggc
ggcttcgacagccccaccgtggcctattctgtgctggtggtggccaaagtggaaaagggcaagtccaagaaactgaagagtgtgaaag
agctgctggggatcaccatcatggaaagaagcagcttcgagaagaatcccatcgactttctggaagccaagggctacaaagaagtgaa
aaaggacctgatcatcaagctgcctaagtactccctgttcgagctggaaaacggccggaagagaatgctggcctctgccggcgaactg
cagaagggaaacgaactggccctgccctccaaatatgtgaacttcctgtacctggccagccactatgagaagctgaagggctcccccg
aggataatgagcagaaacagctgtttgtggaacagcacaagcactacctggacgagatcatcgagcagatcagcgagttctccaagag
agtgatcctggccgacgctaatctggacaaagtgctgtccgcctacaacaagcaccgggataagcccatcagagagcaggccgagaa
tatcatccacctgtttaccctgaccaatctgggagcccctgccgccttcaagtactttgacaccaccatcgaccggaagaggtacaccag
caccaaagaggtgctggacgccaccctgatccaccagagcatcaccggcctgtacgagacacggatcgacctgtctcagctgggagg
tgactctggcggctcaaaaagaaccgccgacggcagcgaattcgagcccaagaagaagaggaaagtc (SEQ ID NO: 162).

A nucleotide sequence of the 004V10-nCas9 is as follows:

atgaaacggacagccgacggaagcgagttcgagtcaccaaagaagaagcggaaagtcatgtacaatgcccctagattcttttgt

agatctgctgccgtgtctgatcatgagtttaacgatgagtactggatgagacacgccctgacactggccaagagggctcgggaggaag

gagaggtgccagtgggagctgtgctggtgctgaataatcaggtcattggagaaggatggaatagagccatcggcctgcacgacccca

ccgcccacgccgagatcatggccctgagacagggcggcctggtgctgcagaactaccggctgatcgacgccaccctgtacgtgacct

tcgagccctgcgtgatgtgcgccggcgccatggtgcatagcagattgaagaaactggtgttcggcgtgagaaactcaaagagaggcg

ccgccggcagcctgatcaacgtgctgaactaccccggcatgaaccacagagtggagatcaccgagggcatcctggccgagagctgc

agcgccatgctgtgcgacttctataggtggcctagggaggtgttcaatgccctgaagaaggccagacaggaggagggctccggagga

tctagcggaggctcctctggctctgagacacctggcacaagcgagagcgcaacacctgaaagcagcgggggcagcagcggggggt

cagacaagaagtacagcatcggcctggccatcggcaccaactctgtgggctgggccgtgatcaccgacgagtacaaggtgcccagc

aagaaattcaaggtgctgggcaacaccgaccggcacagcatcaagaagaacctgatcggagccctgctgttcgacagcggcgaaac

agccgaggccacccggctgaagagaaccgccagaagaagatacaccagacggaagaaccggatctgctatctgcaagagatcttca

gcaacgagatggccaaggtggacgacagcttcttccacagactggaagagtccttcctggtggaagaggataagaagcacgagcgg

caccccatcttcggcaacatcgtggacgaggtggcctaccacgagaagtaccccaccatctaccacctgagaaagaaactggtggaca

gcaccgacaaggccgacctgcggctgatctatctggccctggcccacatgatcaagttccggggccacttcctgatcgagggcgacct

gaaccccgacaacagcgacgtggacaagctgttcatccagctggtgcagacctacaaccagctgttcgaggaaaaccccatcaacgc

cagcggcgtggacgccaaggccatcctgtctgccagactgagcaagagcagacggctggaaaatctgatcgcccagctgcccggcg

agaagaagaatggcctgttcggaaacctgattgccctgagcctgggcctgacccccaacttcaagagcaacttcgacctggccgagga

tgccaaactgcagctgagcaaggacacctacgacgacgacctggacaacctgctggcccagatcggcgaccagtacgccgacctgtt

tctggccgccaagaacctgtccgacgccatcctgctgagcgacatcctgagagtgaacaccgagatcaccaaggcccccctgagcgc

ctctatgatcaagagatacgacgagcaccaccaggacctgaccctgctgaaagctctcgtgcggcagcagctgcctgagaagtacaaa

gagattttcttcgaccagagcaagaacggctacgccggctacattgacggcggagccagccaggaagagttctacaagttcatcaagc

ccatcctggaaaagatggacggcaccgaggaactgctcgtgaagctgaacagagaggacctgctgcggaagcagcggaccttcgac

aacggcagcatcccccaccagatccacctgggagagctgcacgccattctgcggcggcaggaagatttttacccattcctgaaggaca

accgggaaaagatcgagaagatcctgaccttccgcatcccctactacgtgggccctctggccaggggaaacagcagattcgcctggat

gaccagaaagagcgaggaaaccatcaccccctggaacttcgaggaagtggtggacaagggcgcttccgcccagagcttcatcgagc

ggatgaccaacttcgataagaacctgcccaacgagaaggtgctgcccaagcacagcctgctgtacgagtacttcaccgtgtataacga

gctgaccaaagtgaaatacgtgaccgagggaatgagaaagcccgccttcctgagcggcgagcagaaaaaggccatcgtggacctgc

tgttcaagaccaaccggaaagtgaccgtgaagcagctgaaagaggactacttcaagaaaatcgagtgcttcgactccgtggaaatctcc

ggcgtggaagatcggttcaacgcctccctgggcacataccacgatctgctgaaaattatcaaggacaaggacttcctggacaatgagga

aaacgaggacattctggaagatatcgtgctgaccctgacactgtttgaggacagagagatgatcgaggaacggctgaaaacctatgcc

cacctgttcgacgacaaagtgatgaagcagctgaagcggcggagatacaccggctggggcaggctgagccggaagctgatcaacg

gcatccgggacaagcagtccggcaagacaatcctggatttcctgaagtccgacggcttcgccaacagaaacttcatgcagctgatccac

gacgacagcctgacctttaaagaggacatccagaaagcccaggtgtccggccagggcgatagcctgcacgagcacattgccaatctg

gccggcagccccgccattaagaagggcatcctgcagacagtgaaggtggtggacgagctcgtgaaagtgatgggccggcacaagc

ccgagaacatcgtgatcgaaatggccagagagaaccagaccacccagaagggacagaagaacagccgcgagagaatgaagcgga

tcgaagagggcatcaaagagctgggcagccagatcctgaaagaacaccccgtggaaaacacccagctgcagaacgagaagctgtac

ctgtactacctgcagaatgggcgggatatgtacgtggaccaggaactggacatcaaccggctgtccgactacgatgtggaccatatcgt

gcctcagagctttctgaaggacgactccatcgacaacaaggtgctgaccagaagcgacaagaaccggggcaagagcgacaacgtgc

cctccgaagaggtcgtgaagaagatgaagaactactggcggcagctgctgaacgccaagctgattacccagagaaagttcgacaatct

gaccaaggccgagagaggcggcctgagcgaactggataaggccggcttcatcaagagacagctggtggaaacccggcagatcaca

aagcacgtggcacagatcctggactcccggatgaacactaagtacgacgagaatgacaagctgatccgggaagtgaaagtgatcacc

ctgaagtccaagctggtgtccgatttccggaaggatttccagttttacaaagtgcgcgagatcaacaactaccaccacgcccacgacgc

ctacctgaacgccgtcgtgggaaccgccctgatcaaaaagtaccctaagctggaaagcgagttcgtgtacggcgactacaaggtgtac

gacgtgcggaagatgatcgccaagagcgagcaggaaatcggcaaggctaccgccaagtacttcttctacagcaacatcatgaacttttt

caagaccgagattaccctggccaacggcgagatccggaagcggcctctgatcgagacaaacggcgaaaccggggagatcgtgtgg

gataagggccgggattttgccaccgtgcggaaagtgctgagcatgccccaagtgaatatcgtgaaaaagaccgaggtgcagacaggc

ggcttcagcaaagagtctatcctgcccaagaggaacagcgataagctgatcgccagaaagaaggactgggaccctaagaagtacggc

ggcttcgacagccccaccgtggcctattctgtgctggtggtggccaaagtggaaaagggcaagtccaagaaactgaagagtgtgaaag

agctgctggggatcaccatcatggaaagaagcagcttcgagaagaatcccatcgactttctggaagccaagggctacaaagaagtgaa

aaaggacctgatcatcaagctgcctaagtactccctgttcgagctggaaaacggccggaagagaatgctggcctctgccggcgaactg

cagaagggaaacgaactggccctgccctccaaatatgtgaacttcctgtacctggccagccactatgagaagctgaagggctcccccg

aggataatgagcagaaacagctgtttgtggaacagcacaagcactacctggacgagatcatcgagcagatcagcgagttctccaagag

agtgatcctggccgacgctaatctggacaaagtgctgtccgcctacaacaagcaccgggataagcccatcagagagcaggccgagaa

tatcatccacctgtttaccctgaccaatctgggagcccctgccgccttcaagtactttgacaccaccatcgaccggaagaggtacaccag

caccaaagaggtgctggacgccaccctgatccaccagagcatcaccggcctgtacgagacacggatcgacctgtctcagctgggagg

tgactctggcggctcaaaaagaaccgccgacggcagcgaattcgagcccaagaagaagaggaaagtc (SEQ ID NO: 163).

A nucleotide sequence of the 004V12-nCas9 is as follows:

atgaaacggacagccgacggaagcgagttcgagtcaccaaagaagaagcggaaagtcatgtacaatgcccctagattcttttgt
agatctgctgccgtgtctgatcatgagtttaacgatgagtactggatgaagacacgccctgacactggccaagagggctcgggaggaag
gagaggtgccagtgggagctgtgctggtgctgaataatcaggtcattggagaaggatggaatagagccatcggcctgcacgacccca
ccgcccacgccgagatcatggccctgagacagggcggcctggtgctgcagaactaccggctgatcgacgccaccctgtacgtgacct
tcgagccctgcgtgatgtgcgccggcgccatggtgcatagcagaatcagcagactggtgttcggcgtgagaaactcaaagagaggcg
ccgccggcagcctgatcaagatcttccactaccccggcatgaaccacagagtggagatcaccgagggcatcctggccgagagctgca
gcgccatgctgtgcgacttctataggtggcctagggaggtgttcaatgccctgaagaaggccagacaggaggagggctccggaggat
ctagcggaggctcctctggctctgagacacctggcacaagcgagagcgcaacacctgaaagcagcgggggcagcagcggggggtc
agacaagaagtacagcatcggcctggccatcggcaccaactctgtgggctgggccgtgatcaccgacgagtacaaggtgcccagca
agaaattcaaggtgctgggcaacaccgaccggcacagcatcaagaagaacctgatcggagccctgctgttcgacagcggcgaaaca
gccgaggccacccggctgaagagaaccgccagaagaagatacaccagacggaagaaccggatctgctatctgcaagagatcttcag
caacgagatggccaaggtggacgacagcttcttccacagactggaagagtccttcctggtggaagaggataagaagcacgagcggca
ccccatcttcggcaacatcgtggacgaggtggcctaccacgagaagtaccccaccatctaccacctgagaaagaaactggtggacag
caccgacaaggccgacctgcggctgatctatctggccctggcccacatgatcaagttccggggccacttcctgatcgagggcgacctg
aaccccgacaacagcgacgtggacaagctgttcatccagctggtgcagacctacaaccagctgttcgaggaaaaccccatcaacgcc
agcggcgtggacgccaaggccatcctgtctgccagactgagcaagagcagacggctggaaaatctgatcgcccagctgcccggcga

gaagaagaatggcctgttcggaaacctgattgccctgagcctgggcctgacccccaacttcaagagcaacttcgacctggccgaggat

gccaaactgcagctgagcaaggacacctacgacgacgacctggacaacctgctggcccagatcggcgaccagtacgccgacctgttt

ctggccgccaagaacctgtccgacgccatcctgctgagcgacatcctgagagtgaacaccgagatcaccaaggcccccctgagcgcc

tctatgatcaagagatacgacgagcaccaccaggacctgaccctgctgaaagctctcgtgcggcagcagctgcctgagaagtacaaag

agattttcttcgaccagagcaagaacggctacgccggctacattgacggcggagccagccaggaagagttctacaagttcatcaagcc

catcctggaaaagatggacggcaccgaggaactgctcgtgaagctgaacagagaggacctgctgcggaagcagcggaccttcgaca

acggcagcatcccccaccagatccacctgggagagctgcacgccattctgcggcggcaggaagatttttacccattcctgaaggacaa

ccgggaaaagatcgagaagatcctgaccttccgcatcccctactacgtgggccctctggccaggggaaacagcagattcgcctggatg

accagaaagagcgaggaaaccatcaccccctggaacttcgaggaagtggtggacaagggcgcttccgcccagagcttcatcgagcg

gatgaccaacttcgataagaacctgcccaacgagaaggtgctgcccaagcacagcctgctgtacgagtacttcaccgtgtataacgagc

tgaccaaagtgaaatacgtgaccgagggaatgagaaagcccgccttcctgagcggcgagcagaaaaaggccatcgtggacctgctgt

tcaagaccaaccggaaagtgaccgtgaagcagctgaaagaggactacttcaagaaaatcgagtgcttcgactccgtggaaatctccgg

cgtggaagatcggttcaacgcctccctgggcacataccacgatctgctgaaaattatcaaggacaaggacttcctggacaatgaggaaa

acgaggacattctggaagatatcgtgctgaccctgacactgtttgaggacagagagatgatcgaggaacggctgaaaacctatgccca

cctgttcgacgacaaagtgatgaagcagctgaagcggcggagatacaccggctggggcaggctgagccggaagctgatcaacggc

atccgggacaagcagtccggcaagacaatcctggatttcctgaagtccgacggcttcgccaacagaaacttcatgcagctgatccacga

cgacagcctgacctttaaagaggacatccagaaagcccaggtgtccggccagggcgatagcctgcacgagcacattgccaatctggc

cggcagccccgccattaagaagggcatcctgcagacagtgaaggtggtggacgagctcgtgaaagtgatgggccggcacaagccc

gagaacatcgtgatcgaaatggccagagagaaccagaccacccagaagggacagaagaacagccgcgagagaatgaagcggatc

gaagagggcatcaaagagctgggcagccagatcctgaaagaacaccccgtggaaaacacccagctgcagaacgagaagctgtacct

gtactacctgcagaatgggcgggatatgtacgtggaccaggaactggacatcaaccggctgtccgactacgatgtggaccatatcgtgc

ctcagagctttctgaaggacgactccatcgacaacaaggtgctgaccagaagcgacaagaaccggggcaagagcgacaacgtgccc

tccgaagaggtcgtgaagaagatgaagaactactggcggcagctgctgaacgccaagctgattacccagagaaagttcgacaatctga

ccaaggccgagagaggcggcctgagcgaactggataaggccggcttcatcaagagacagctggtggaaacccggcagatcacaaa

gcacgtggcacagatcctggactcccggatgaacactaagtacgacgagaatgacaagctgatccgggaagtgaaagtgatcaccct
gaagtccaagctggtgtccgatttccggaaggatttccagttttacaaagtgcgcgagatcaacaactaccaccacgcccacgacgcct
acctgaacgccgtcgtgggaaccgccctgatcaaaaagtaccctaagctggaaagcgagttcgtgtacggcgactacaaggtgtacga
cgtgcggaagatgatcgccaagagcgagcaggaaatcggcaaggctaccgccaagtacttcttctacagcaacatcatgaactttttca
agaccgagattaccctggccaacggcgagatccggaagcggcctctgatcgagacaaacggcgaaaccggggagatcgtgtgggat
aagggccgggattttgccaccgtgcggaaagtgctgagcatgccccaagtgaatatcgtgaaaaagaccgaggtgcagacaggcgg
cttcagcaaagagtctatcctgcccaagaggaacagcgataagctgatcgccagaaagaaggactgggaccctaagaagtacggcgg
cttcgacagccccaccgtggcctattctgtgctggtggtggccaaagtggaaaagggcaagtccaagaaactgaagagtgtgaaagag
ctgctggggatcaccatcatggaaagaagcagcttcgagaagaatcccatcgactttctggaagccaagggctacaaagaagtgaaaa
aggacctgatcatcaagctgcctaagtactccctgttcgagctggaaaacggccggaagagaatgctggcctctgccggcgaactgca
gaagggaaacgaactggccctgccctccaaatatgtgaacttcctgtacctggccagccactatgagaagctgaagggctcccccgag
gataatgagcagaaacagctgtttgtggaacagcacaagcactacctggacgagatcatcgagcagatcagcgagttctccaagagag
tgatcctggccgacgctaatctggacaaagtgctgtccgcctacaacaagcaccgggataagcccatcagagagcaggccgagaatat
catccacctgtttaccctgaccaatctgggagcccctgccgccttcaagtactttgacaccaccatcgaccggaagaggtacaccagca
ccaaagaggtgctggacgccaccctgatccaccagagcatcaccggcctgtacgagacacggatcgacctgtctcagctgggaggtg
actctggcggctcaaaaagaaccgccgacggcagcgaattcgagcccaagaagaagaggaaagtc (SEQ ID NO: 164).

A nucleotide sequence of the 004V13-nCas9 is as follows:

atgaaacggacagccgacggaagcgagttcgagtcaccaaagaagaagcggaaagtcatgtacaatgcccctagattcttttgt
agatctgctgccgtgtctgatcatgagtttaacgatgagtactggatgagacacgccctgacactggccaagagggctcgggaggaag
gagaggtgccagtgggagctgtgctggtgctgaataatcaggtcattggagaaggatggaatagagccatcggcctgcacgacccca
ccgcccacgccgagatcatggccctgagacagggcggcctggtgctgcagaactaccggctgatcgacgccaccctgtacgtgacct
tcgagccctgcgtgatgtgcgccggcgccatggtgcatagcagaatcagcagactggtgttcggcgtgagaaactcaaagagaggcg
ccgccggcagcctgatcaacgtgctgaactacttcgccctgaaccacagagtggagatcaccgagggcatcctggccgagagctgca
gcgccatgctgtgcgacttctataggtggcctagggaggtgttcaatgccctgaagaaggccagacaggaggagggctccggaggat

ctagcggaggctcctctggctctgagacacctggcacaagcgagagcgcaacacctgaaagcagcgggggcagcagcggggggtc

agacaagaagtacagcatcggcctggccatcggcaccaactctgtgggctgggccgtgatcaccgacgagtacaaggtgcccagca

agaaattcaaggtgctgggcaacaccgaccggcacagcatcaagaagaacctgatcggagccctgctgttcgacagcggcgaaaca

gccgaggccacccggctgaagagaaccgccagaagaagatacaccagacggaagaaccggatctgctatctgcaagagatcttcag

caacgagatggccaaggtggacgacagcttcttccacagactggaagagtccttcctggtggaagaggataagaagcacgagcggca

ccccatcttcggcaacatcgtggacgaggtggcctaccacgagaagtaccccaccatctaccacctgagaaagaaactggtggacag

caccgacaaggccgacctgcggctgatctatctggccctggcccacatgatcaagttccggggccacttcctgatcgagggcgacctg

aaccccgacaacagcgacgtggacaagctgttcatccagctggtgcagacctacaaccagctgttcgaggaaaaccccatcaacgcc

agcggcgtggacgccaaggccatcctgtctgccagactgagcaagagcagacggctggaaaatctgatcgcccagctgcccggcga

gaagaagaatggcctgttcggaaacctgattgccctgagcctgggcctgacccccaacttcaagagcaacttcgacctggccgaggat

gccaaactgcagctgagcaaggacacctacgacgacgacctggacaacctgctggcccagatcggcgaccagtacgccgacctgttt

ctggccgccaagaacctgtccgacgccatcctgctgagcgacatcctgagagtgaacaccgagatcaccaaggcccccctgagcgcc

tctatgatcaagagatacgacgagcaccaccaggacctgaccctgctgaaagctctcgtgcggcagcagctgcctgagaagtacaaag

agattttcttcgaccagagcaagaacggctacgccggctacattgacggcggagccagccaggaagagttctacaagttcatcaagcc

catcctggaaaagatggacggcaccgaggaactgctcgtgaagctgaacagagaggacctgctgcggaagcagcggaccttcgaca

acggcagcatcccccaccagatccacctgggagagctgcacgccattctgcggcggcaggaagatttttacccattcctgaaggacaa

ccgggaaaagatcgagaagatcctgaccttccgcatcccctactacgtgggccctctggccaggggaaacagcagattcgcctggatg

accagaaagagcgaggaaaccatcaccccctggaacttcgaggaagtggtggacaagggcgcttccgcccagagcttcatcgagcg

gatgaccaacttcgataagaacctgcccaacgagaaggtgctgcccaagcacagcctgctgtacgagtacttcaccgtgtataacgagc

tgaccaaagtgaaatacgtgaccgagggaatgagaaagcccgccttcctgagcggcgagcagaaaaaggccatcgtggacctgctgt

tcaagaccaaccggaaagtgaccgtgaagcagctgaaagaggactacttcaagaaaatcgagtgcttcgactccgtggaaatctccgg

cgtggaagatcggttcaacgcctccctgggcacataccacgatctgctgaaaattatcaaggacaaggacttcctggacaatgaggaaa

acgaggacattctggaagatatcgtgctgaccctgacactgtttgaggacagagagatgatcgaggaacggctgaaaacctatgccca

cctgttcgacgacaaagtgatgaagcagctgaagcggcggagatacaccggctggggcaggctgagccggaagctgatcaacggc

atccgggacaagcagtccggcaagacaatcctggatttcctgaagtccgacggcttcgccaacagaaacttcatgcagctgatccacga
cgacagcctgacctttaaagaggacatccagaaagcccaggtgtccggccagggcgatagcctgcacgagcacattgccaatctggc
cggcagccccgccattaagaagggcatcctgcagacagtgaaggtggtggacgagctcgtgaaagtgatgggccggcacaagccc
gagaacatcgtgatcgaaatggccagagagaaccagaccacccagaagggacagaagaacagccgcgagagaatgaagcggatc
gaagagggcatcaaagagctgggcagccagatcctgaaagaacaccccgtggaaaacacccagctgcagaacgagaagctgtacct
gtactacctgcagaatgggcgggatatgtacgtggaccaggaactggacatcaaccggctgtccgactacgatgtggaccatatcgtgc
ctcagagctttctgaaggacgactccatcgacaacaaggtgctgaccagaagcgacaagaaccggggcaagagcgacaacgtgccc
tccgaagaggtcgtgaagaagatgaagaactactggcggcagctgctgaacgccaagctgattacccagagaaagttcgacaatctga
ccaaggccgagagaggcggcctgagcgaactggataaggccggcttcatcaagagacagctggtggaaacccggcagatcacaaa
gcacgtggcacagatcctggactcccggatgaacactaagtacgacgagaatgacaagctgatccgggaagtgaaagtgatcaccct
gaagtccaagctggtgtccgatttccggaaggatttccagttttacaaagtgcgcgagatcaacaactaccaccacgcccacgacgcct
acctgaacgccgtcgtgggaaccgccctgatcaaaaagtaccctaagctggaaagcgagttcgtgtacggcgactacaaggtgtacga
cgtgcggaagatgatcgccaagagcgagcaggaaatcggcaaggctaccgccaagtacttcttctacagcaacatcatgaactttttca
agaccgagattaccctggccaacggcgagatccggaagcggcctctgatcgagacaaacggcgaaaccggggagatcgtgtgggat
aagggccgggattttgccaccgtgcggaaagtgctgagcatgccccaagtgaatatcgtgaaaaagaccgaggtgcagacaggcgg
cttcagcaaagagtctatcctgcccaagaggaacagcgataagctgatcgccagaaagaaggactgggaccctaagaagtacggcgg
cttcgacagccccaccgtggcctattctgtgctggtggtggccaaagtggaaaagggcaagtccaagaaactgaagagtgtgaaagag
ctgctggggatcaccatcatggaaagaagcagcttcgagaagaatcccatcgactttctggaagccaagggctacaaagaagtgaaaa
aggacctgatcatcaagctgcctaagtactccctgttcgagctggaaaacggccggaagagaatgctggcctctgccggcgaactgca
gaagggaaacgaactggccctgccctccaaatatgtgaacttcctgtacctggccagccactatgagaagctgaagggctcccccgag
gataatgagcagaaacagctgtttgtggaacagcacaagcactacctggacgagatcatcgagcagatcagcgagttctccaagagag
tgatcctggccgacgctaatctggacaaagtgctgtccgcctacaacaagcaccgggataagcccatcagagagcaggccgagaatat
catccacctgtttaccctgaccaatctgggagcccctgccgccttcaagtactttgacaccaccatcgaccggaagaggtacaccagca
ccaaagaggtgctggacgccaccctgatccaccagagcatcaccggcctgtacgagacacggatcgacctgtctcagctgggaggtg

actctggcggctcaaaaagaaccgccgacggcagcgaattcgagcccaagaagaagaggaaagtc (SEQ ID NO: 165).

### Example 6

Mutations were also performed on other amino acid sites of the adenosine deaminase 004V1 to obtain an adenosine deaminase 004V14, an adenosine deaminase 004V15, an adenosine deaminase 004V16, an adenosine deaminase 004V17, an adenosine deaminase 004V18, an adenosine deaminase 004V19, an adenosine deaminase 004V20, an adenosine deaminase 004V21, an adenosine deaminase 004V22, an adenosine deaminase 004V23, an adenosine deaminase 004V24, an adenosine deaminase 004V25, an adenosine deaminase 004V26, an adenosine deaminase 004V27, an adenosine deaminase 004V28, an adenosine deaminase 004V29, an adenosine deaminase 004V30, an adenosine deaminase 004V31, an adenosine deaminase 004V32, an adenosine deaminase 004V33, an adenosine deaminase 004V34, an adenosine deaminase 004V35, an adenosine deaminase 004V36, an adenosine deaminase 004V37, an adenosine deaminase 004V38, an adenosine deaminase 004V39, an adenosine deaminase 004V40, and an adenosine deaminase 004V41. Amino acid mutation patterns are shown in Table 12, and the amino acid mutation pattern involved in each deaminase mutant is shown in Table 13.

**Table 12 Amino acid mutation pattern involved in each deaminase variant**

| Mutation site | Mutation pattern | Codon before mutation | Codon after mutation |
|---|---|---|---|
| 15 | S15G | TCT | GGA |
| 16 | D16N | GAT | AAC |
| 17 | H17C | CAT | TGC |
| 18 | E18D | GAG | GAC |
| 18 | E18K | GAG | AAG |
| 19 | F19C | TTT | TGT |
| 19 | F19S | TTT | AGC |
| 20 | N20L | AAC | CTC |
| 21 | D21Q | GAT | CAG |
| 22 | E22R | GAG | CGG |
| 23 | Y23F | TAC | TTT |
| 24 | W24P | TGG | CCT |
| 25 | M25H | ATG | CAC |
| 25 | M25L | ATG | CTG |
| 25 | M25V | ATG | GTG |
| 26 | R26T | AGA | ACA |
| 27 | H27R | CAC | AGA |
| 28 | A28C | GCC | TGT |
| 30 | T30E | ACA | GAA |
| 33 | K33S | AAG | TCT |
| 36 | R36Q | CGG | CAG |
| 49 | L49F | CTG | TTC |
| 51 | N51G | AAT | GGT |
| 64 | L64M | CTG | ATG |
| 64 | L64Q | CTT | CAG |
| 64 | L64P | CTG | CCG |
| 82 | V82T | GTG | ACA |
| 83 | L83Q | CTG | CAA |
| 89 | I89E | ATC | GAG |
| 91 | A91C | GCC | TGT |
| 106 | A106C | GCC | TGT |
| 119 | V119L | GTG | TTG |
| 122 | S122N | TCA | AAT |
| 122 | S122P | TCA | CCA |
| 124 | R124H | AGA | CAT |
| 124 | R124T | AGA | ACA |
| 135 | N135S | AAC | TCT |
| 140 | N140K | AAC | AAG |
| 141 | H141A | CAC | GCT |
| 142 | R142L | AGA | TTG |
| 142 | R142S | AGA | TCC |
| 144 | E144H | GAG | CAC |
| 160 | D160E | GAC | GAA |
| 168 | V168A | GTG | GCG |
| 169 | F169C | TTC | TGC |
| 169 | F169V | TTC | GTC |
| 170 | N170D | AAT | GAT |

**Table 13 Mutation pattern of each deaminase mutant**

| Deaminase variants | Mutation pattern |
|---|---|
| Adenosine deaminase 004V14 | E18K + F19S + N20L |
| Adenosine deaminase 004V15 | M25L |
| Adenosine deaminase 004V16 | S15G + D16N + H17C |
| Adenosine deaminase 004V17 | L64Q + S122P + V168A |
| Adenosine deaminase 004V18 | R36Q + L64Q + S122P + F169V |
| Adenosine deaminase 004V19 | R36Q + V119L + V168A |
| Adenosine deaminase 004V20 | R36Q + V119L + N170D |
| Adenosine deaminase 004V21 | L64Q + V119L + V168A |
| Adenosine deaminase 004V22 | F169C |
| Adenosine deaminase 004V23 | L64Q + V119L + V168A |
| Adenosine deaminase 004V24 | L64P + R124T + F169V |
| Adenosine deaminase 004V25 | I89E + A91C |
| Adenosine deaminase 004V26 | L64Q + V119L + F169V |
| Adenosine deaminase 004V27 | L64M |
| Adenosine deaminase 004V28 | L64Q + S122P + N170D |
| Adenosine deaminase 004V29 | E18D + F19C + N20S |
| Adenosine deaminase 004V30 | L64P + V119L + N170D |
| Adenosine deaminase 004V31 | S122N + R124H + N135S + D160E |
| Adenosine deaminase 004V32 | R26T + H27R + A28C |
| Adenosine deaminase 004V33 | V82T + L83Q |
| Adenosine deaminase 004V34 | M25V |
| Adenosine deaminase 004V35 | D21Q + T30E + K33S |
| Adenosine deaminase 004V36 | N140K + H141A + R142S |
| Adenosine deaminase 004V37 | L49F + N51G |
| Adenosine deaminase 004V38 | A106C |
| Adenosine deaminase 004V39 | R142L + E144H |
| Adenosine deaminase 004V40 | E22R + Y23F + W24P + M25H |
| Adenosine deaminase 004V41 | L64Q + R124T + V168A |

The same method as in Examples 2 to 4 was used to construct an adenine base editor expression vector constituted by each of the adenosine deaminases 004V14 to 004V41 and a PCSK9-sgRNA expression vector, and cell culture and transfection were performed in the same manner as in the above examples. A gene editing effect test process is as follows.

For a calculation method of gene editing efficiency, see Kluesner MG, Nedveck DA, Lahr WS, Garbe JR, Abrahante JE, Webber BR, Moriarity BS. EditR: A Method to Quantify Base Editing from Sanger Sequencing. CRISPR J. 2018 Jun;1(3):239-250. doi: 10.1089/crispr.2018.0014. PMID: 31021262; PMCID: PMC6694769.

An editing efficiency of the base editor constituted by the above adenosine deaminase 004V1 mutant and nCas9 is compared with an editing efficiency of the deaminase 004V1-nCas9 as follows.

| Deaminase variant | Ratio of editing efficiency of base editor constituted by each mutant/editing efficiency of 004V1-nCas9 |
|---|---|
| Adenosine deaminase 004V14 | 2.57 |
| Adenosine deaminase 004V15 | 2.29 |
| Adenosine deaminase 004V16 | 2.14 |
| Adenosine deaminase 004V17 | 1.79 |
| Adenosine deaminase 004V18 | 1.79 |
| Adenosine deaminase 004V19 | 1.79 |
| Adenosine deaminase 004V20 | 1.71 |
| Adenosine deaminase 004V21 | 1.57 |
| Adenosine deaminase 004V22 | 1.55 |
| Adenosine deaminase 004V23 | 1.5 |
| Adenosine deaminase 004V24 | 1.43 |
| Adenosine deaminase 004V25 | 1.39 |
| Adenosine deaminase 004V26 | 1.36 |
| Adenosine deaminase 004V27 | 1.29 |
| Adenosine deaminase 004V28 | 1.29 |
| Adenosine deaminase 004V29 | 1.23 |
| Adenosine deaminase 004V30 | 1.07 |
| Adenosine deaminase 004V31 | 1.03 |
| Adenosine deaminase 004V32 | 1.03 |
| Adenosine deaminase 004V33 | 0.98 |
| Adenosine deaminase 004V34 | 0.97 |
| Adenosine deaminase 004V35 | 0.94 |
| Adenosine deaminase 004V36 | 0.92 |
| Adenosine deaminase 004V37 | 0.89 |
| Adenosine deaminase 004V38 | 0.86 |
| Adenosine deaminase 004V39 | 0.79 |
| Adenosine deaminase 004V40 | 0 |
| Adenosine deaminase 004V41 | 0 |

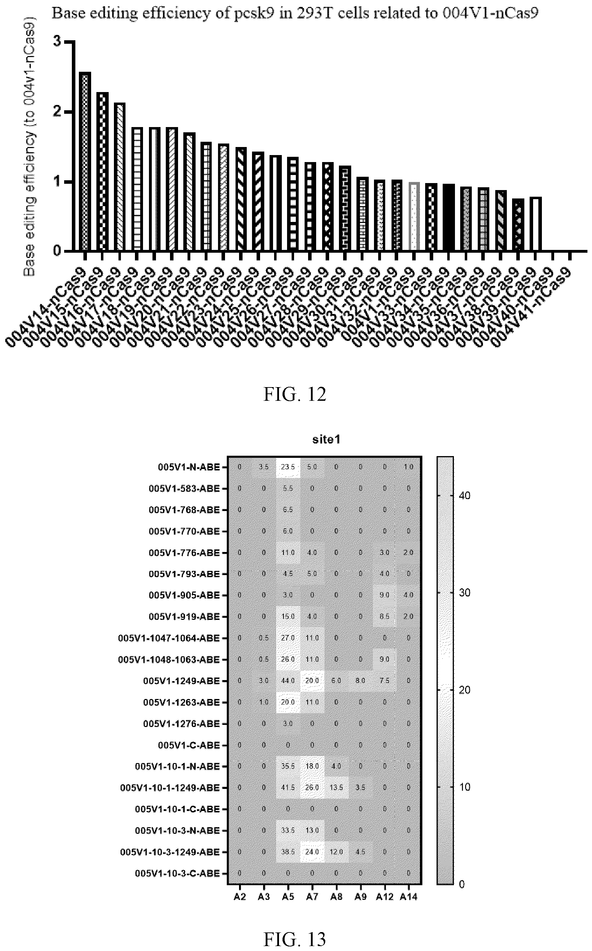

Relevant results of this example are shown in FIG. 12. Compared with the 004V1-nCas9, the editing efficiency of the base editor constituted by each of the deaminases 004V14 to 004V32 and nCas9 at a PCSK9 site is higher than that of the 004V1-nCas9. The editing efficiency of the base editor constituted by each of the adenosine deaminases 004V14 to 004V29 and nCas9 is greatly improved compared with the 004V1-nCas9, and the editing efficiencies of the 004V17-nCas9 to the 004V29-nCas9 are more than 20% higher than that of the 004V1-nCas9. In particular, the editing efficiencies of the 004V14-nCas9, the 004V15-nCas9, and the 004V16-nCas9 are each more than twice that of the 004V1-nCas9.

### Example 7

In order to further improve an editing efficiency of a base editor fusion protein, the applicant explored a structure of a base editor fusion protein. A suitable insertion site for an adenosine deaminase was found in the middle of a nCas9 protein. The adenosine deaminases used included adenosine deaminases 005V1, 005V1-10-1, and 005V1-10-3, and sequences of the above three adenosine deaminases are shown in Example 1.

Experimental steps are as follows.

### 1. Construction of nCas9 plasmid

### (1) Design of primers (primers synthesized by Shanghai Boshang Biotechnology Co., Ltd.):

**Table 14**

| Primer name | Sequence |
|---|---|
| nCas9-F | Gaagcggaaagtcgacaagaagtacagcatcggcc (SEQ ID NO: 202) |
| nCas9-R | ctgtacttcttgtcgactttccgcttcttctttggtgac (SEQ ID NO: 203) |

PCR amplification was performed on ABE8e (Addgene, #138489) using a high-fidelity enzyme kit (Vazyme, P501-d2) of Vazyme Biotech Co., Ltd., and an amplification system is shown in Table 15.

**Table 15**

| Component | 100 µl system |
|---|---|
| 2 × Phanta Flash Master Mix (Dye Plus) | 50 µl |
| 10 µM forward primer | 5 µl |
| 10 µM reverse primer | 5 µl |
| DNA | Plasmid (5 ng) |
| Nuclease-free water | To 100 µl |

A PCR amplification program is shown in the following table.

**Table 16**

| Step | TEMP | Time |
|---|---|---|
| Initial denaturation | 95°C | 30 s |
| 34 cycles | 95°C | 10 s |
| | 60°C | 30 s |
| | 72°C | 40 s |
| Final extension | 72°C | 2 min |
| Hold | 4°C | Forever |

An amplified PCR product was recovered according to the kit instructions (TIANGEN, Universal DNA Purification and Recovery Kit, DP214).

A purified PCR product was converted into E. coli DH5a competent cells (Weidi Biotechnology Co., Ltd., DL1001). A specific procedure is as follows: the DH5α competent cells were taken out from -80°C and were quickly inserted into ice. After 5 minutes, a bacterial block was melted, a connected product was added and gently mixed well by hand at a bottom of a centrifuge tube, and then allowed to stand for 25 minutes in ice. The heat shock was performed in a water bath at 42°C for 45 seconds, and the mixture was quickly placed back to ice and allowed to stand for 2 minutes. 700 µl of sterile LB medium without antibiotics was added to the centrifuge tube, mixed well, and recovered at 37°C, 200 rpm for 60 minutes. The cells were collected by centrifugation at 5000 rpm for one minute. About 100 µl of a supernatant was taken and gently blown to resuspend a bacterial block and spread on an LB medium containing Amp antibiotics. A plate was placed upside down in a 37°C incubator for overnight culture. Single colonies were picked, and after sequencing confirmation, positive clones were shaken and a nCas9 plasmid was extracted using an endotoxin-free plasmid extraction kit (TIANGEN: DP120-01), and a concentration was measured. The plasmid was stored in a -20°C refrigerator for later use.

### (2) Obtaining DNA Sequences Encoding Deaminase 005V1, 005V1-10-1, and 005V1-10-3

Primers were used to perform PCR amplification on 005V1-nCas9, 005V1-10-1-nCas9, and 005V1-10-3-nCas9 plasmids. An amplification system and a PCR program were the same as those in Tables 15 and 16. An amplified PCR product was recovered according to the kit instructions (TIANGEN, Universal DNA Purification and Recovery Kit, DP214).

The PCR primers used are shown in the following table.

**Table 17**

| Primer name | Corresponding sequence |
|---|---|
| Deaminase-F | TCTGGAGGATCTAGCGGCGGATCCTCTGGAAGCGAGACACCAGGC ACAAGCGAGTCCGCCACACCAGAGAGCTCCGGCGGCTCCTCCGG AGGATCCggaATGAGTGAGCTGAATGATGC (SEQ ID NO: 204) |
| Deaminase-R | tgaccccccgctgctgcccccgctgctttcaggtgttgcg (SEQ ID NO: 205) |

### (3) Design of Different Chimeric Base Editors and Primer Sequences of Corresponding nCas9 Plasmids

Insertion positions of the deaminases 005V1, 005V1-10-1, and 005V1-10-3 in the nCas9 were studied, and various chimeric base editors were designed. Primer sequences of the corresponding nCas9 were designed according to the different insertion positions of the above deaminases, as shown in Table 18.

**Table 18**

| Base editor name | Insertion position | Editor connection method |
|---|---|---|
| 005V1-N-ABE | Not involved | Deaminase 005V1 connected to N-terminal of nCas9 |
| 005V1-583-ABE | Between positions 583 and 584 of nCas9 | Deaminase 005V1 inserted between positions 583 and 584 of nCas9 |
| 005V1-768-ABE | Between positions 768 and 769 of nCas9 | Deaminase 005V1 inserted between positions 768 and 769 of nCas9 |
| 005V1-770-ABE | Between positions 770 and 771 of nCas9 | Deaminase 005V1 inserted between positions 770 and 771 of nCas9 |
| 005V1-776-ABE | Between positions 776 and 777 of nCas9 | Deaminase 005V1 inserted between positions 776 and 777 of nCas9 |
| 005V1-793-ABE | Between positions 793 and 794 of nCas9 | Deaminase 005V1 inserted between positions 793 and 794 of nCas9 |
| 005V1-905-ABE | Between positions 905 and 906 of nCas9 | Deaminase 005V1 inserted between positions 905 and 906 of nCas9 |
| 005V1-919-ABE | Between positions 919 and 920 of nCas9 | Deaminase 005V1 inserted between positions 919 and 920 of nCas9 |
| 005V1-1047-1064-ABE | Between positions 1048 to 1063 of nCas9 | Deaminase 005V1 inserted between positions 1048 to 1063 of nCas9 |
| 005V1-1048-1063-ABE | Between positions 1049 to 1062 of nCas9 | Replace positions 1048 to 1064 of nCas9 with coding sequence of deaminase 005V1 |
| 005V1-1249-ABE | Between positions 1249 and 1250 of nCas9 | Deaminase 005V1 inserted between positions 1249 and 1250 of nCas9 |
| 005V1-1263-ABE | Between positions 1263 and 1264 of nCas9 | Deaminase 005V1 inserted between positions 1263 and 1264 of nCas9 |
| 005V1-1276-ABE | Between positions 1276 and 1277 of nCas9 | Deaminase 005V1 inserted between positions 1276 and 1277 of nCas9 |
| 005V1-C-ABE | Not involved | Deaminase 005V1 connected to C-terminal of nCas9 |
| 005V1-10-1-N-ABE | Not involved | Deaminase 005V1-10-1 connected to N-terminal of nCas9 |
| 005V1-10-1-1249-ABE | Between positions 1249 and 1250 of nCas9 | Deaminase 005V1-10-1 inserted between positions 1249 and 1250 of nCas9 |
| 005V1-10-1-C-ABE | Not involved | Deaminase 005V1-10-1 connected to C-terminal of nCas9 |
| 005V1-10-3-N-ABE | Not involved | Deaminase 005V1-10-3 connected to N-terminal of nCas9 |
| 005V1-10-3-1249-ABE | Between positions 1249 and 1250 of nCas9 | Deaminase 005V1-10-3 inserted between positions 1249 and 1250 of nCas9 |
| 005V1-10-3-C-ABE | Not involved | Deaminase 005V1-10-3 connected to C-terminal of nCas9 |

The primer sequences of the nCas9 plasmids corresponding to different chimeric base editors described in Table 18 are designed as follows.

**Table 19**

| Deaminase insertion position in nCas9 | PCR primer sequence |
|---|---|
| Positions 583 and 584 | 583-F: ggggcagcagcggggggtcagtggaagatcggttcaacgcctcc (SEQ ID NO: 206) |
| | 583-R: CCGCCGCTAGATCCTCCAGAgccggagatttccacggagtcgaag (SEQ ID NO: 207) |
| Positions 768 and 769 | 768-F: ggggcagcagcggggggtcacagaccacccagaagggacagaagaacag (SEQ ID NO: 208) |
| | 768-R: CCGCCGCTAGATCCTCCAGAgttctctctggccatttcgatcacgatgttctcg (SEQ ID NO: 209) |
| Positions 770 and 771 | 770-F (ggggcagcagcggggggtcaacccagaagggacagaagaacagccg (SEQ ID NO: 210) |
| | 770-R: CCGCCGCTAGATCCTCCAGAggtctggttctctctggccatttcgatcacg (SEQ ID NO: 211) |
| Positions 776 and 777 | 776-F: ggggcagcagcggggggtcaaacagccgcgagagaatgaagcg (SEQ ID NO: 212) |
| | 776-R: CCGCCGCTAGATCCTCCAGActtctgtcccttctgggtggtctgg (SEQ ID NO: 213) |
| Positions 793 and 794 | 793-F: ggggcagcagcggggggtcaagccagatcctgaaagaacacc (SEQ ID NO: 214) |
| | 793-R: CCGCCGCTAGATCCTCCAGAgcccagctctttgatgccctcttcgatcc (SEQ ID NO: 215) |
| Positions 905 and 906 | 905-F: ggggcagcagcggggggtcaagaggcggcctgagcgaactggata (SEQ ID NO: 216) |
| | 905-R: CCGCCGCTAGATCCTCCAGActcggccttggtcagattgtcgaactttctctgg (SEQ ID NO: 217) |
| Positions 919 and 920 | 919-F: ggggcagcagcggggggtcaagacagctggtggaaacccggcagatca (SEQ ID NO: 218) |
| | 919-R: CCGCCGCTAGATCCTCCAGActtgatgaagccggccttatccagttcg (SEQ ID NO: 219) |
| Positions 1047 to 1064 | 1047-1064-F: ggggcagcagcggggggtcagagacaaacggcgaaaccgg (SEQ ID NO: 220) |
| | 1047-1064-R: CCGCCGCTAGATCCTCCAGActtgaaaaagttcatgatgttgctgtagaagaagtacttggcg (SEQ ID NO: 221) |
| Positions 1048 to 1063 | 1048-1063-F: ggggcagcagcggggggtcaatcgagacaaacggcgaaaccgg (SEQ ID NO: 222) |
| | 1048-1063-R: |
| | CCGCCGCTAGATCCTCCAGAggtcttgaaaaagttcatgatgttgctgtagaagaagtacttgg c (SEQ ID NO: 223) |
| Positions 1249 and 1250 | 1249-F: ggggcagcagcggggggtcacccgaggataatgagcagaaacagctgt (SEQ ID NO: 224) 1249-R: CCGCCGCTAGATCCTCCAGAggagcccttcagcttctcatagtggct (SEQ ID NO: 225) |
| Positions 1263 and 1264 | 1263-F: ggggcagcagcggggggtcaaagcactacctggacgagatcatcgagc (SEQ ID NO: 226) |
| | 1263-R: CCGCCGCTAGATCCTCCAGAgtgctgttccacaaacagctgtttctgc (SEQ ID NO: 227) |
| Positions 1276 and 1277 | 1276-F: ggggcagcagcggggggtcattctccaagagagtgatcctggccgac (SEQ ID NO: 228) |
| | 1276-R: CCGCCGCTAGATCCTCCAGActcgctgatctgctcgatgatctcgtccag (SEQ ID NO: 229) |

The nCas9 plasmids obtained in step (1) were amplified using the primer sequences in Table 19, respectively. An amplification system and a PCR program were the same as those in Tables 15 and 16. An amplified PCR product was recovered according to the kit instructions (TIANGEN, Universal DNA Purification and Recovery Kit, DP214). The primers used for the amplification of the nCas9 plasmids corresponding to different insertion positions are shown in Table 19. For example, an amino acid sequence of the 005V1-1249-ABE is as follows:
*PKKKRKV***MDKKYSIGLAIGTNSVGWAVITDEYKVPSKKFKVLGNTDRHSIKK NLIGALLFDSGETAEATRLKRTARRRYTRRKNRICYLQEIFSNEMAKVDDSFFHR LEESFLVEEDKKHERHPIFGNIVDEVAYHEKYPTIYHLRKKLVDSTDKADLRLIYL ALAHMIKFRGHFLIEGDLNPDNSDVDKLFIQLVQTYNQLFEENPINASGVDAKAIL SARLSKSRRLENLIAQLPGEKKNGLFGNLIALSLGLTPNFKSNFDLAEDAKLQLSK DTYDDDLDNLLAQIGDQYADLFLAAKNLSDAILLSDILRVNTEITKAPLSASMIKR YDEHHQDLTLLKALVRQQLPEKYKEIFFDQSKNGYAGYIDGGASQEEFYKFIKPI LEKMDGTEELLVKLNREDLLRKQRTFDNGSIPHQIHLGELHAILRRQEDFYPFLK DNREKIEKILTFRIPYYVGPLARGNSRFAWMTRKSEETITPWNFEEVVDKGASAQ SFIERMTNFDKNLPNEKVLPKHSLLYEYFTVYNELTKVKYVTEGMRKPAFLSGEQ KKAIVDLLFKTNRKVTVKQLKEDYFKKIECFDSVEISGVEDRFNASLGTYHDLLK IIKDKDFLDNEENEDILEDIVLTLTLFEDREMIEERLKTYAHLFDDKVMKQLKRRR YTGWGRLSRKLINGIRDKQSGKTILDFLKSDGFANRNFMQLIHDDSLTFKEDIQK AQVSGQGDSLHEHIANLAGSPAIKKGILQTVKVVDELVKVMGRHKPENIVIEMAR ENQTTQKGQKNSRERMKRIEEGIKELGSQILKEHPVENTQLQNEKLYLYYLQNG RDMYVDQELDINRLSDYDVDHIVPQSFLKDDSIDNKVLTRSDKNRGKSDNVPSEE VVKKMKNYWRQLLNAKLITQRKFDNLTKAERGGLSELDKAGFIKRQLVETRQIT KHVAQILDSRMNTKYDENDKLIREVKVITLKSKLVSDFRKDFQFYKVREINNYHH AHDAYLNAVVGTALIKKYPKLESEFVYGDYKVYDVRKMIAKSEQEIGKATAKYFF YSNIMNFFKTEITLANGEIRKRPLIETNGETGEIVWDKGRDFATVRKVLSMPQVNI VKKTEVQTGGFSKESILPKRNSDKLIARKKDWDPKKYGGFDSPTVAYSVLVVAKV EKGKSKKLKSVKELLGITIMERSSFEKNPIDFLEAKGYKEVKKDLIIKLPKYSLFE LENGRKRMLASAGELQKGNELALPSKYVNFLYLASHYEKLKGSSGGSSGGSSGSE** TPGTSESATPESSGGSSGGSSELNDAYWMKQALALAQKAREQGEVPVGAILVLDDEVI GQGWNRAITLHDPTAHAEIMALQQGGQIVQNYRLLNATLYVTFEPCVMCAGAMVHS RIKRLVYGVSNSKRGAAGSLLNVLNYPGMNHOIEITAGVMANECSEMLCOFYOOPRE VFNAEREARRLNOPDRADSGGSSGGSSGSETPGTSESATPESSGGSSGGSPEDNEQKQ **LFVEQHKHYLDEIIEQISEFSKRVILADANLDKVLSAYNKHRDKPIREQAENIIHLF TLTNLGAPAAFKYFDTTIDRKRYTSTKEVLDATLIHQSITGLYETRIDLSQLGGDSG** GSKRTADGSEFE*PKKKRKV** (SEQ ID NO: 230), wherein, the italic sequence indicates NLS, the bold sequence indicates a nCas9 fragment, the underline sequence indicates a linker, the double underline sequence indicates a deaminase, and the asterisk at a C-terminal indicates a stop codon position. The deaminase sequence is the deaminase 005V1, and the deaminase 005V1 is embedded in nCas9 at positions 1249 and 1250.

(4) The PCR products of the adenosine deaminase 005V1, 10-1, and 10-3 obtained in step (2) were homologously recombined with the linearized PCR products at different insertion positions in step (3) using a Gibson Assembly Master Mix recombination kit (NEB, E2611S) to obtain different chimeric recombinant plasmids. A reaction system is shown in the following table.

**Table 19**

| Reaction object | Volume (ul) |
|---|---|
| Deaminase fragment | 100 ng |
| Vector fragment | 50 ng |
| Gibson Assembly Master Mix (2X) | 10 ul |
| ddH₂O | Made up to 20 ul |
| Total volume | 20 ul |

A homologous recombination product was converted into E. coli DH5a competent cells (Weidi Biotechnology Co., Ltd., DL1001). A specific procedure is as follows: the DH5α competent cells were taken out from -80°C and were quickly inserted into ice. After 5 minutes, a bacterial block was melted, a connected product was added and gently mixed well by hand at a bottom of a centrifuge tube, and then allowed to stand for 25 minutes in ice. The heat shock was performed in a water bath at 42°C for 45 seconds, and the mixture was quickly placed back to ice and allowed to stand for 2 minutes. 700 µl of sterile LB medium without antibiotics was added to the centrifuge tube, mixed well, and recovered at 37°C, 200 rpm for 60 minutes. The cells were collected by centrifugation at 5000 rpm for one minute. About 100 µl of a supernatant was taken and gently blown to resuspend a bacterial block and spread on an LB medium containing Amp antibiotics. A plate was placed upside down in a 37°C incubator for overnight culture. Single colonies were picked, and after sequencing confirmation, positive clones were shaken and a chimeric recombinant plasmid was extracted using an endotoxin-free plasmid extraction kit (TIANGEN: DP120-01), and a concentration was measured. The plasmid was stored in a -20°C refrigerator for later use.

### (5) Cell Culture and Transfection

HEK293T cells (purchased from ATCC) were inoculated in a DMEM medium (Gibco, 11965092) supplemented with 10% FBS (v/v), containing 1% Penicillin Streptomycin (v/v) (Gibco, 15140122), and cultured in a 37°C cell culture incubator containing 5% CO₂. The cells used for transfection were inoculated in a 24-well cell culture plate the day before and cultured. The cells were observed the next day and transfected when the cells grew to a cell density of about 80%. The amount of plasmids transfected in each well of the 24-well plate was 0.4 µg of chimeric recombinant plasmid, and 0.4 µg of sgRNA plasmid. The sgRNA used is shown in the following table.

**Table 20**

| sgRNA name | Sequence |
|---|---|
| PCSK9-sgRNA | CCCGCACCTTGGCGCAGCGG (SEQ ID NO: 141) |
| site 1-sgRNA | GAACACAAAGCATAGACTGC (SEQ ID NO: 142) |
| FANCF-sgRNA | GGAACACGGATAAAGACGCT (SEQ ID NO: 231) |

The PCSK9-sgRNA and site 1-sgRNA sequences used in this example are the same as the corresponding sequences in other examples, and the FANCF-sgRNA is a sgRNA targeting a FANCF gene.

The chimeric recombinant plasmid and the sgRNA plasmid were mixed, and then diluted with 25 µl of reduced serum medium (Basal Media, L530KJ), 2 µl of p3000 reagent was added, the mixture was blown and mixed well as a reagent A, and allowed to stand for 5 minutes. At the same time, 2 µl of Lipofectamine 3000 transfection reagent (Thermo, 11668019) was diluted with 25 µl of reduced serum medium and mixed well as a reagent B, and allowed to stand for 5 minutes. The above reagent A and reagent B were mixed and blown evenly, and allowed to stand for 20 minutes. After the standing was finished, the mixed reagent was added dropwise to the cells of the 24-well plate to be transfected and placed back to the 37°C incubator for culture. After 6 hours of transfection, the culture medium was replaced with a DMEM medium containing 10% FBS. After 48 hours of transfection, the cells were collected for editing efficiency detection.

### (6) Editing Efficiency Detection

A genomic DNA extraction kit (TIANGEN, DP304-03) was used to perform genomic extraction on the HEK293T cells. Primers were designed according to experimental requirements, and identification primer sequences used are shown in Table 21.

**Table 21**

| Primer name | Sequence |
|---|---|
| PCSK9-forward primer | gctagccttgcgttccg (SEQ ID NO. 146) |
| PCSK9-reverse primer | gtccccaagatcgtgccaa (SEQ ID NO. 147) |
| site 1-forward primer | cctcagcattcagccactaa (SEQ ID NO. 148) |
| site 1-reverse primer | agaggcccattaacgtttgg (SEQ ID NO. 149) |
| FANCF-forward primer | ATGGATGTGGCGCAGGTAG (SEQ ID NO. 232) |
| FANCF-reverse primer | AAGGAAGCGCGGAGACGTT (SEQ ID NO. 233) |

A genome was used as a template, PCR amplification was performed on a sequence near a sgRNA target by using an identification primer, and an amplified PCR product was used for Sanger sequencing (Boshang Biotechnology (Shanghai) Co., Ltd.) to identify the editing efficiency.

The PCR amplification system used for target site sequence is as follows: 25 µL of 2 × Taq Master Mix (Vazyme, P112-03), 1 µL of Primer-F (10 pmol/µL), 1 µL of Primer-R (10 pmol/µL), 1 µL of template, and ddH₂O made up to 50 µL.

For a calculation method of gene editing efficiency, see Kluesner MG, Nedveck DA, Lahr WS, Garbe JR, Abrahante JE, Webber BR, Moriarity BS. EditR: A Method to Quantify Base Editing from Sanger Sequencing. CRISPR J. 2018 Jun;1(3):239-250. doi: 10.1089/crispr.2018.0014. PMID: 31021262; PMCID: PMC6694769.

Relevant results of this example are shown in FIGS. 13 to 15. At a site 1, base editing efficiencies of each chimeric base editor and the base editors with the deaminase connected to the N-terminal or the C-terminal of the nCas9 are shown in FIG. 13. It can be seen that at the site 1, the base editors 005V1-C-ABE, 005V1-10-1-C-ABE, and 005V1-10-3-C-ABE have no editing activity. For other base editors, the editing efficiency of each of the 005V1-1047-1064-ABE, 005V1-1048-1063-ABE, and 005V1-1249-ABE at an A5 position of the site 1 is significantly higher than that of the 005V1-N-ABE, especially the editing efficiency of the 005V1-1249-ABE is nearly two times that of the 005V1-N-ABE. The base editing efficiency of each of the 005V1-776-ABE, 005V1-793-ABE, 005V1-905-ABE, and 005V1-919-ABE at a specific position of the site 1 is higher than that of the 005V1-N-ABE, which can be used to edit a specific gene position to achieve disease treatment. The editing efficiencies of the 005V1-10-1-1249-ABE at all positions of the site 1 are higher than that of the 005V1-10-1-N-ABE. The editing efficiencies of the 005V1-10-3-1249-ABE at all positions of the site 1 are higher than that of the 005V1-10-3-N-ABE. It can be seen that for the 005V1 and its mutants 005V1-10-1 and 005V1-10-3, when the nCas9 insertion position is selected between 1249 and 1250, a better editing efficiency can be obtained.

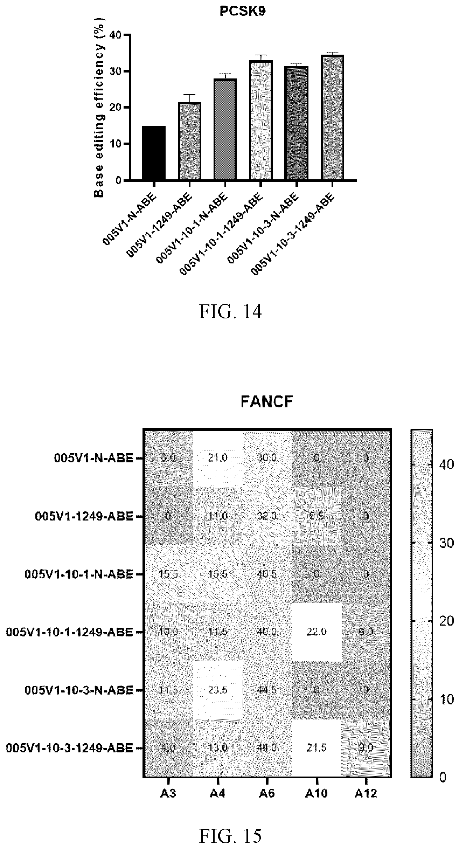

At the PCSK9 site, base editing efficiencies of each chimeric base editor and the base editors with the deaminase connected to the N-terminal or the C-terminal of the nCas9 are shown in FIG. 14. It can be seen that at the PCSK9 site, the editing efficiency of the 005V1-1249-ABE is higher than that of the 005V1-N-ABE, the editing efficiency of the 005V1-10-1-1249-ABE at this site is higher than that of the 005V1-10-1-N-ABE, and the editing efficiency of the 005V1-10-3-1249-ABE at this site is higher than that of the 005V1-10-3-N-ABE.

At the FANCF site, base editing efficiencies of each chimeric base editor and the base editors with the deaminase connected to the N-terminal or the C-terminal of the nCas9 are shown in FIG. 15. It can be seen that at the FANCF site, the 005V1-1249-ABE shows a higher editing efficiency than the 005V1-N-ABE at a specific position, and editing characteristics thereof at some positions are different from those of the 005V1-N-ABE. The 005V1-10-1-1249-ABE shows an editing efficiency at positions A10 and A12 of the site, while the 005V1-10-1-N-ABE does not show an editing activity at these positions, and an editing activity at other positions is not much different from that of the 005V1-10-1-N-ABE. The 005V1-10-3-1249-ABE shows similar characteristics to the 005V1-10-1-1249-ABE at the site and shows an editing efficiency at the positions A10 and A12, while the 005V1-10-3-N-ABE does not show an editing activity at these positions, and an editing activity at other positions is not much different from that of the 005V1-10-3-N-ABE.

## Claims

1. An adenosine deaminase having one or more of the following sequences:
(i) an amino acid sequence set forth in SEQ ID NO: 1 or SEQ ID NO: 170;
(ii) an amino acid sequence that has at least 80%, 82%, 85%, 87%, 90%, 92%, 95%, 96%, 97%, 98%, or 99% identity to the amino acid sequence set forth in SEQ ID NO: 1 or SEQ ID NO: 170, and retains a deamination activity of the amino acid sequence set forth in SEQ ID NO: 1 or SEQ ID NO: 170;
(iii) an amino acid sequence, in which one or more amino acid residues are added, substituted, deleted, or inserted into the amino acid sequence set forth in SEQ ID NO: 1 or SEQ ID NO: 170, and which retains the deamination activity of the amino acid sequence set forth in SEQ ID NO: 1 or SEQ ID NO: 170; or
(iv) an amino acid sequence encoded by a nucleotide sequence, the nucleotide sequence hybridizes, under a stringency condition, with a polynucleotide sequence encoding the amino acid sequence set forth in SEQ ID NO: 1 or SEQ ID NO: 170, the amino acid sequence retains the deamination activity of the amino acid sequence set forth in SEQ ID NO: 1 or SEQ ID NO: 170, and the stringency condition is a medium stringency condition, a medium-high stringency condition, a high stringency condition, or a very high stringency condition.

2. The adenosine deaminase according to claim 1, wherein the adenosine deaminase has a change at any of the following amino acid positions relative to the amino acid sequence set forth in SEQ ID NO: 1: 33, 35, 36, 46, 47, 48, 49, 104, 105, 107, 148, 149, 150, 151, 152, 153, 154, and 155.

3. The adenosine deaminase according to claim 1, wherein the substitution is a substitution that occurs at one or more of the following sites of the amino acid sequence set forth in SEQ ID NO: 170:
S15, D16, H17, E18, F19, N20, D21, E22, Y23, W24, M25, R26, H27, A28, L29, T30, K33, R34, A35, R36, V41, V43, L47, L49, N51, N59, A61, I62, L64, A69, E72, G80, L81, V82, L83, Q84, N85, Y86, I89, D90, A91, T92, V95, F97, A106, R111, I112, S113, R114, L115, F117, V119, R120, N121, S122, K123, R124, N132, V133, L134, N135, P137, G138, M139, N140, H141, R142, E144, D160, V168, F169, and N170.

4. The adenosine deaminase according to claim 2, wherein the deaminase has a change(s) selected from the following amino acid positions relative to the amino acid sequence set forth in SEQ ID NO: 1:
V33I, D35G, D35R, D36G, D36N, A46C, I47Y, I47R, I47V, T48G, T48R, L49T, L49K, L49H, V104A, V104M, S105C, S105G, S107R, S107K, S107A, Q148P, Q148C, Q148A, Q148G, Q149M, Q149G, Q149L, P150R, P150L, P150C, R151K, E152R, E152T, E152G, E152W, V153P, V153F, V153I, V153T, F154H, F154K, F154L, N155T, N155R, and N155H.

5. The adenosine deaminase according to claim 4, wherein the adenosine deaminase has a change of any one selected from the following groups relative to the amino acid sequence set forth in SEQ ID NO: 1:
(1) Q148G + Q149M + P150R, (2) S107A, (3) E152R + V153P + F154H + N155T, (4) S107K, (5) Q148P + Q149G + P150L, (6) A46C + I47Y + T48G + L49H, (7) E152T + V153F + N155T, (8) S107R, (9) V104M, (10) E152G + V153I + F154K + N155R, (11) S105C, (12) Q148C + Q149L + P150R + R151K, (13) Q148A + Q149G + P150C, (14) E152W + V153T + F154L + N155H, (15) S105G, (16) I47R + T48G + L49T, (17) D35G + D36N, (18) V33I + D35R + D36G, (19) V104A, and (20) I47V + T48R + L49K.

6. The adenosine deaminase according to claim 3, wherein the substitution is a substitution that occurs at one or more of the following sites of the amino acid sequence set forth in SEQ ID NO: 170: S15T, S15G, D16E, D16N, H17C, H17K, E18D, E18K, F19C, F19Y, F19S, N20Q, N20L, N20S, D21Q, E22D, Y23F, W24F, M25L, M25V, R26K, R26T, H27R, A28C, L29I, T30E, K33R, K33S, R34K, A35S, R36Q, L49F, N51G, L64M, L64Q, L64P, G80A, L81N, V82A, V82T, L83I, L83Q, Q84N, N85S, Y86W, I89E, I89L, D90G, A91C, A91T, T92D, A106C, I112L, S113K, R114K, V119L, S122N, S122P, R124H, R124T, N132K, V133I, L134F, N135H, N135S, P137F, G138A, M139L, N140K, H141A, R142L, R142S, E144H, D160E, V168A, F169C, F169V, and N170D, and
preferably, the substitution is a substitution that occurs at a combination of the following sites of the amino acid sequence set forth in SEQ ID NO: 170:
(1) S15T + D16E + H17K + F19Y + N20Q, (2) E22D + Y23F + W24F + R26K + H27R + L29I, (3) K33R + R34K + A35S, (4) G80A + L81N + V82A + L83I + Q84N + N85S + Y86W, (5) I89L + D90G + A91T + T92D, (6) I112L + S113K + R114K, (7) N132K + V133I + L134F + N135H, (8) P137F + G138A + M139L, (9) E18K + F19S + N20L, (10) M25L, (11) S15G + D16N + H17C, (12) L64Q + S122P + V168A, (13) R36Q + L64Q + S122P + F169V, (14) R36Q + V119L + V168A, (15) R36Q + V119L + N170D, (16) L64Q + V119L + V168A, (17) F169C, (18) L64Q + V119L + V168A, (19) L64P + R124T + F169V, (20) I89E + A91C, (21) L64Q + V119L + F169V, (22) L64M, (23) L64Q + S122P + N170D, (24) E18D + F19C + N20S, (25) L64P + V119L + N170D, (26) S122N + R124H + N135S + D160E, (27) R26T + H27R + A28C, (28) V82T + L83Q, (29) M25V, (30) D21Q + T30E + K33S, (31) N140K + H141A + R142S, (32) L49F + N51G, (33) A106C, and (34) R142L + E144H.

7. A base editor fusion protein comprising: the adenosine deaminase according to any one of claims 1 to 6; and a nucleic acid programmable nucleotide binding domain.

8. The base editor fusion protein according to claim 7, wherein the nucleic acid programmable nucleotide binding domain is a Cas protein or an AGO protein.

9. The base editor fusion protein according to claim 7 or 8, further comprising: at least one nuclear localization signal sequence, wherein
optionally, the base editor fusion protein further comprises a linker,
optionally, the linker includes one or more of sequences set forth in SEQ ID NO: 171 to SEQ ID NO: 180, and
optionally, the nuclear localization signal sequence includes one or more of sequences set forth in SEQ ID NO: 181 to SEQ ID NO: 189.

10. The fusion protein according to claim 8, wherein the Cas protein includes a Cas9, Cas12, or Cas13 family.

11. The fusion protein according to claim 10, wherein the Cas protein includes SpCas9, SaCas9, Cas12a, Cas12b, Cas12c, Cas12d, Cas12e, Cas12g, Cas12h, Cas12i, Cas12j, or Cas13.

12. The fusion protein according to claim 10 or 11, wherein the Cas protein is an inactivated nuclease or a nicking nuclease.

13. The base editor fusion protein according to any one of claims 7 to 12, comprising one or more of the following sequences:
(i) an amino acid sequence set forth in SEQ ID NO: 190 or SEQ ID NO: 3;
(ii) an amino acid sequence that has at least 80%, 82%, 85%, 87%, 90%, 92%, 95%, 96%, 97%, 98%, or 99% identity to the amino acid sequence set forth in SEQ ID NO: 190 or SEQ ID NO: 3, and retains a polynucleotide binding and base editing activity of the amino acid sequence set forth in SEQ ID NO: 190 or SEQ ID NO: 3;
(iii) an amino acid sequence, in which one or more amino acid residues are added, substituted, deleted, or inserted into the amino acid sequence set forth in SEQ ID NO: 190 or SEQ ID NO: 3, and which retains the polynucleotide binding and base editing activity of the amino acid sequence set forth in SEQ ID NO: 190 or SEQ ID NO: 3; or
(iv) an amino acid sequence encoded by a nucleotide sequence, the nucleotide sequence hybridizes, under a stringency condition, with a polynucleotide sequence encoding the amino acid sequence set forth in SEQ ID NO: 190 or SEQ ID NO: 3, the amino acid sequence retains the polynucleotide binding and base editing activity of the amino acid sequence set forth in SEQ ID NO: 190 or SEQ ID NO: 3, and the stringency condition is a medium stringency condition, a medium-high stringency condition, a high stringency condition, or a very high stringency condition.

14. The base editor fusion protein according to claim 13, comprising any one of sequences set forth in SEQ ID NO: 191 to SEQ ID NO: 198.

15. A base editor fusion protein comprising: the adenosine deaminase according to any one of claims 1 to 6; and the nucleic acid programmable nucleotide binding domain according to any one of claims 7 to 9, wherein the fusion protein has a structure of NH2-[N-terminal fragment of nucleic acid programmable nucleotide binding domain]-[deaminase]-[C-terminal fragment of nucleic acid programmable nucleotide binding domain]-COOH.

16. The base editor fusion protein according to claim 15, wherein a nCas9 domain is selected as the nucleic acid programmable nucleotide binding domain, and the adenosine deaminase is inserted at a position between amino acid positions 583 to 584, 768 to 769, 770 to 771, 776 to 777, 793 to 794, 905 to 906, 919 to 920, 1048 to 1063, 1049 to 1062, 1249 to 1250, 1263 to 1264, or 1276 to 1277 of SEQ ID NO: 61.

17. A polynucleotide encoding the adenosine deaminase according to any one of claims 1 to 6 or encoding the base editor fusion protein according to any one of claims 7 to 16, comprising, but not limited to: nucleotide sequences set forth in SEQ ID NO. 2, SEQ ID NO. 4, and SEQ ID NO. 156 to SEQ ID NO. 165.

18. A vector comprising: the polynucleotide according to claim 17.

19. A cell comprising: the adenosine deaminase according to any one of claims 1 to 6; the base editor fusion protein according to any one of claims 7 to 16; the polynucleotide according to claim 17; or the vector according to claim 18.

20. A base editor system comprising: the adenosine deaminase according to any one of claims 1 to 6; a nucleic acid programmable nucleotide binding domain; and a guide polynucleotide, or comprising:
the base editor fusion protein according to any one of claims 7 to 16; and a guide polynucleotide.

21. A pharmaceutical composition comprising: the adenosine deaminase according to any one of claims 1 to 6, the base editor fusion protein according to any one of claims 7 to 16, the polynucleotide according to claim 17, the vector according to claim 18, the cell according to claim 19, or the base editor system according to claim 20; and a pharmaceutically acceptable carri er.

22. A kit comprising: the adenosine deaminase according to any one of claims 1 to 6; the base editor fusion protein according to any one of claims 7 to 16; the polynucleotide according to claim 17; the vector according to claim 18; the cell according to claim 19; or the base editor system according to claim 20.

23. A delivery system comprising: the adenosine deaminase according to any one of claims 1 to 6, the base editor fusion protein according to any one of claims 7 to 16, the polynucleotide according to claim 17, the vector according to claim 18, the cell according to claim 19, or the base editor system according to claim 20; and a delivery medium.

24. A base editing method of a nucleic acid, the method comprising: a step of contacting a nucleic acid to be base edited with the base editor system according to claim 20.

25. A use of the adenosine deaminase according to any one of claims 1 to 6, the base editor fusion protein according to any one of claims 7 to 16, the polynucleotide according to claim 17, the vector according to claim 18, the cell according to claim 19, the base editor system according to claim 20, the pharmaceutical composition according to claim 21, or the delivery system according to claim 23 in treating a disease associated with or caused by a point mutation or in preparing a medicine for treating a disease associated with or caused by a point mutation.

26. The use according to claim 25, mainly comprising: a use in treating a disease associated with a PCSK9 target, and/or a use in preparing a medicine for treating hypercholesterolemia, transthyretin amyloidosis, and β-hemoglobinopathy.
